(19) Europäisches Patentamt
European Patent Office
Office européen des brevets

(11) EP 4 177 258 B1

(12) **EUROPEAN PATENT SPECIFICATION**

(45) Date of publication and mention of the grant of the patent:
**03.06.2026 Bulletin 2026/23**

(21) Application number: **21831694.1**

(22) Date of filing: **02.07.2021**

(51) International Patent Classification (IPC):
***C07F 9/6558*** *(2006.01)* ***C07F 9/6512*** *(2006.01)*
***A61P 35/02*** *(2006.01)* ***A61P 35/00*** *(2006.01)*
***A61K 31/675*** *(2006.01)*

(52) Cooperative Patent Classification (CPC):
**C07F 9/65583; A61P 35/00; A61P 35/02; C07F 9/65586;** A61K 31/675

(86) International application number:
**PCT/CN2021/104219**

(87) International publication number:
**WO 2022/002241 (06.01.2022 Gazette 2022/01)**

(54) **ARYLPHOSPHINE OXIDE COMPOUNDS AND USE THEREOF**

ARYLPHOSPHINOXIDVERBINDUNGEN UND IHRE VERWENDUNG

COMPOSÉS D'OXYDE D'ARYLPHOSPHINE ET LEUR UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.07.2020 CN 202010638649**
**11.12.2020 CN 202011464023**

(43) Date of publication of application:
**10.05.2023 Bulletin 2023/19**

(73) Proprietor: **Chengdu Di'Ao Jiuhong Pharmaceutical Factory**
**Chengdu, Sichuan 610041 (CN)**

(72) Inventors:
- **HE, Peng**
**Chengdu, Sichuan 610041 (CN)**
- **DONG, Guangxin**
**Chengdu, Sichuan 610041 (CN)**
- **ZHAO, Hai**
**Chengdu, Sichuan 610041 (CN)**
- **WANG, Xuechao**
**Chengdu, Sichuan 610041 (CN)**
- **CHEN, Shuang**
**Chengdu, Sichuan 610041 (CN)**
- **HUANG, Pei**
**Chengdu, Sichuan 610041 (CN)**
- **DENG, Ta**
**Chengdu, Sichuan 610041 (CN)**
- **ZHANG, Rui**
**Chengdu, Sichuan 610041 (CN)**
- **LI, Haiyan**
**Chengdu, Sichuan 610041 (CN)**

(74) Representative: **Kador & Partner Part mbB**
**Corneliusstraße 15**
**80469 München (DE)**

(56) References cited:
**WO-A1-2012/151561**
**WO-A1-2013/169401**
**WO-A1-2021/057882**
**CN-A- 102 105 150**
**CN-A- 103 501 612**
**CN-A- 108 689 994**
**CN-A- 110 467 638**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 177 258 B1

## Description

TECHNICAL FIELD

The present invention relates to the field of drugs, in particular to an aryl phosphorus oxide compound used as a protein kinase inhibitor.

BACKGROUNG

Protein kinases represent a large family of proteins that play an important role in the regulation of a plurality of cellular processes and in the maintenance and control of cell functions, which include proliferation, apoptosis, cytoskeletal rearrangement, differentiation, development, immunoreaction, nervous system function and conduction. In addition, many diseases and/or functional disorders are associated with aberrant, abnormal or deregulated activity of one or more kinases.

Lung cancer is one of the most common malignant tumors, generally divided into Small Cell Lung Cancer (SCLC) and Non-Small Cell Lung Cancer (NSCLC), and the lung cancer ranks first whether in China or the world. The Non-Small Cell Lung Cancer (NSCLC) accounts for more than 80% of all lung cancers, which seriously threatens human health (Chinese Journal of Lung Cancer [J], 2012 Feb 20; 15(2): 106-111).

EGFR with a full name of epidermal growth factor receptor is a transmembrane glycoprotein with tyrosine kinase activity widely distributed on cell membranes of various human tissues. Mutation and abnormal activation of EGFR are closely related to the occurrence and development, grade malignancy, metastasis with various tumors such as non-small cell lung cancer, breast cancer, esophageal cancer. Most patients with Non-Small Cell Lung Cancer (NSCLC) have EGFR overexpression, and about 40-50% of Non-Small Cell Lung Cancer patients in Asia (especially in China) belong to EGFR mutations, so EGFR inhibition can significantly improve the survival time of the NSCLC patients. Common mutations of EGFR may be divided into two categories, one category refers to drug-sensitive mutations, i.e., anti-tumor targeted drugs may be used after mutation, such as deletions at exon 19, and L858R mutation at exon 21; while the other category refers to drug-resistant mutations, i.e., resistant to a certain anti-tumor targeted drug after mutation, such as T790M mutation, and C797S mutation. The first-generation EGFR small molecule inhibitor drugs Gefitinib, Erlotinib and Icotinib obtain remarkable clinical therapeutic effects in patients with EGFR-sensitive mutations, and prolong survival time. However, most patients who benefit from these drugs develop drug resistance after taking the drugs for several months. More than 50% of the drug-resistant patients develop drug resistance due to the T790M mutation in EGFR. The second-generation EGFR irreversible inhibitor drugs Afatinib and Neratinib obtain better results in preclinical research, but lack selectivity on wild-type EGFR (EGFRWT), and have large side effects such as skin toxicity. The third-generation irreversible inhibitor Osimertinib (AZD9291) overcomes the drug resistance of EGFR T790M, and can effectively treat advanced non-small cell lung cancer patients with epidermal growth factor receptor T790M mutation or drug resistance to other EGFR inhibitors in clinic. Although the Osimertinib has great success in clinically treating the non-small cell lung cancer with EGFR T790M mutation, part of the patients who benefit from the Osimertinib have drug resistance after 9-14 months of treatment (Nature Medicine 2015, 21(6), 560-562). It was found that in up to 40% of the drug-resistant patients, Osimertinib resistance was caused due to point mutation at (EGFR) C797S. Further mechanistic studies have shown that the point mutations at (EGFR) C797S convert cysteine at position 797 to serine, resulting in the inability of the Osimertinib to form covalent bonds with target proteins, ultimately leading to drug resistance. At present, there is no clinically available EGFR inhibitor that is effective against the new mutation (C797S). Therefore, a novel EGFR inhibitor with high selectivity is urgently needed to solve the problems of drug resistance caused by the point mutation at (EGFR) C797S.

Anaplastic lymphoma kinase (ALK), also known as ALK tyrosine kinase receptor or CD246, is an active enzyme encoded by ALK gene in human body. A fusion gene formed by ALK is closely related to the occurrence and development of various tumors such as non-small cell lung cancer. In the non-small cell lung cancer patients, fusion oncogenes such as EML4-ALK (fusion gene of microtubule-associated protein 4 and anaplastic lymphoma kinase in echinoderms) account for about 3-7%. Therefore, it is of great clinical value to develop protein kinase inhibitors to positive ALK fusion genes. On the other hand, in recent years, with the increase of non-small cell lung cancer patients and the popularization of the second-generation sequencing technology (deep sequencing), researchers have found that EGFR mutant and ALK gene fusion can occur simultaneously in some non-small cell lung cancer patients. Therefore, a novel protein kinase inhibitor with high selectivity is urgently needed to solve the problems of drug resistance caused by point mutation of (EGFR) C797S, and solve the fusion and mutation of the ALK genes at the same time.

Patent application having publication number WO 2012/151561 discloses pharmaceutical compositions and methods for treating patients who have an EGFR-driven cancer of formula (I),

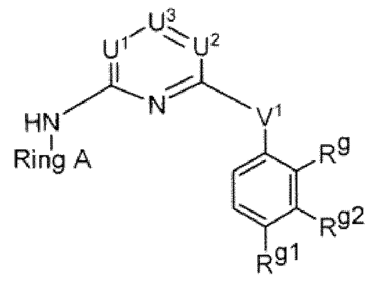

wherein the variables are as defined therein.

SUMMARY

One or more embodiments of the present application provide aryl phosphorus oxide compounds represented by following formula (I) used as a protein kinase inhibitor or pharmaceutically acceptable salts thereof. The aryl phosphorus oxide compounds can effectively inhibit activity of various EGFR drug-resistant mutants (e.g., $EGFR^{del19}$, $EGFR^{del19/T790M}$, $EGFR^{del19/C797S}$, $EGFR^{T790M/L858R}$, $EGFR^{L858R/C797S}$, $EGFR^{del19/T790M/C797S}$, and $EGFR^{L858R/T790M/C797S}$), and also have significant inhibitory effects on ALK fusion genes and mutants (e.g., L1196M), and can be used for the treatment, combined treatment or prevention of various cancers.

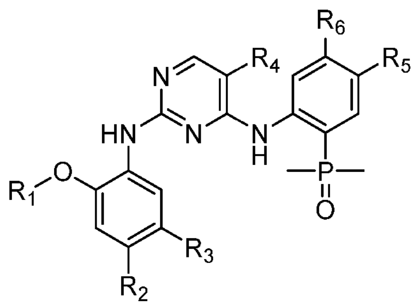

formula (I)

wherein $R_1$ is selected from $C_1$-$C_6$ alkyl and $C_3$-$C_6$ cycloalkyl, and the $C_1$-$C_6$ alkyl and the $C_3$-$C_6$ cycloalkyl are optionally substituted by zero to six R';

$R_2$ is selected from hydrogen, amino, $C_1$-$C_6$ alkyl, $C_3$-$C_6$ cycloalkyl, $C_3$-$C_6$ cycloalkenyl, phenyl, 5-6 membered heteroaryl,

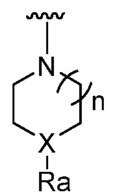 or 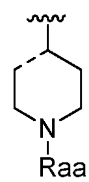;

and the amino, the $C_1$-$C_6$ alkyl, the $C_3$-$C_6$ cycloalkyl, the phenyl and the 5-6 membered heteroaryl are optionally substituted by zero to three $R_a$ groups;

X is selected from CH, S, N or O;

n is 0, 1 or 2;

when X is O, $R_a$ is not contained;

the bond - - - - represents C-C saturated bond or C=C olefinic bond;

$R_3$ is selected from $C_1$-$C_6$ alkyl, $C_3$-$C_6$ cycloalkyl, - and halogen; and the $C_1$-$C_6$ alkyl, the $C_3$-$C_6$ cycloalkyl are optionally substituted by zero to three R';

$R_4$ and $R_6$ are each independently selected from hydrogen, $C_1$-$C_6$ alkyl, $C_3$-$C_6$ cycloalkyl and halogen, and the $C_1$-$C_6$ alkyl and the $C_3$-$C_6$ cycloalkyl are optionally substituted by zero to three R';

$R_5$ is selected from $C_1$-$C_6$ alkyl and halogen, and the $C_1$-$C_6$ alkyl is optionally substituted by zero to three R';

$R_a$ and $R_{aa}$ are each independently selected from hydrogen, $C_1$-$C_6$ alkyl, $C_3$-$C_6$ cycloalkyl, phenyl, 5-6 membered heteroaryl, halogen, amino, hydroxyl, cyano, nitro, $-NR_bC(O)R_c$, $-NR_bR_c$ and

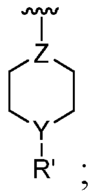

wherein the $C_1$-$C_6$ alkyl, the $C_3$-$C_6$ cycloalkyl, the phenyl, the 5-6 membered heteroaryl, the amino and the hydroxyl are optionally substituted by zero to three R';
Y is selected from N or O, and Z is selected from CH or N; and when Y is O, R' does not exist;
$R_b$ and $R_c$ are each independently selected from hydrogen, $C_1$-$C_6$ alkyl, $C_3$-$C_6$ cycloalkyl, $C_2$-$C_6$ alkenyl, $C_2$-$C_6$ alkynyl, hydroxyl and amino; and the $C_1$-$C_6$ alkyl, the $C_3$-$C_6$ cycloalkyl, the $C_2$-$C_6$ alkenyl and the $C_2$-$C_6$ alkynyl are optionally substituted by zero to three R';
R' is selected from hydrogen, F, Cl, Br, I, hydroxyl, acyl, carboxyl, amino, nitro, cyano, $C_1$-$C_6$ alkyl, $C_3$-$C_6$ cycloalkyl, $C_2$-$C_6$ alkenyl and $C_2$-$C_6$ alkynyl; and the $C_1$-$C_6$ alkyl, the $C_3$-$C_6$ cycloalkyl, the $C_2$-$C_6$ alkenyl, the $C_2$-$C_6$ alkynyl, the amino and the hydroxyl are optionally substituted by zero to three hydrogen, hydroxyl, carboxyl, carbonyl, F, Cl, Br, I, amino, nitro, cyano, methyl, trifluoroethyl, difluoromethyl and monofluoromethyl.

In one or more embodiments of the present application,

$R_1$ is selected from $C_1$-$C_6$ alkyl; and the $C_1$-$C_6$ alkyl is optionally substituted by zero to three R';
$R_2$ is selected from hydrogen, $C_1$-$C_6$ alkyl, amino,

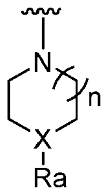

or

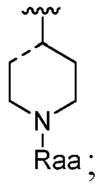

wherein, the amino and the $C_1$-$C_6$ alkyl are substituted by one to three $R_a$;
$R_a$ and $R_{aa}$ are each independently selected from hydrogen, $NR_bR_c$, $C_1$-$C_6$ alkyl or

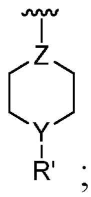

$R_3$ is selected from $C_1$-$C_6$ alkyl, $C_3$-$C_6$ cycloalkyl and halogen ; and the $C_1$-$C_6$ alkyl and the $C_3$-$C_6$ cycloalkyl are optionally substituted by zero to three R';
$R_4$ is selected from halogen, $C_1$-$C_6$ alkyl and $C_3$-$C_6$ cycloalkyl; and the $C_1$-$C_6$ alkyl and the $C_3$-$C_6$ cycloalkyl are optionally substituted by zero to three R';
$R_5$ is selected from $C_1$-$C_6$ alkyl and halogen; and the $C_1$-$C_6$ alkyl is optionally substituted by zero to three R';
$R_6$ is selected from hydrogen or methyl;
$R_b$ and $R_c$ are each independently selected from hydrogen, $C_1$-$C_6$ alkyl and $C_2$-$C_6$ alkenyl; and the $C_1$-$C_6$ alkyl and the $C_2$-$C_6$ alkenyl are optionally substituted by zero to three R'.

The compound or the pharmaceutically acceptable salt thereof in one or more embodiments of the present application, wherein:

$R_3$ is selected from $C_1$-$C_6$ alkyl and $C_3$-$C_6$ cycloalkyl; and the $C_1$-$C_6$ alkyl and the $C_3$-$C_6$ cycloalkyl are optionally substituted by zero to three R';
$R_4$ is selected from halogen and $C_1$-$C_6$ alkyl; wherein, the $C_1$-$C_6$ alkyl is optionally substituted by zero to three R';
$R_5$ is selected from $C_1$-$C_6$ alkyl; wherein, the $C_1$-$C_6$ alkyl is optionally substituted by zero to three R'; and
$R_6$ is hydrogen.

The compound or the pharmaceutically acceptable salt thereof in one or more embodiments of the present application, wherein:

$R_2$ is selected from amino and

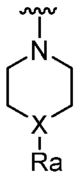

;

and the amino is optionally substituted by zero to three $R_a$; wherein, X is selected from CH, N or O;
$R_a$ is selected from

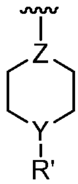

,

$C_1$-$C_3$ alkyl or $NR_bR_c$; wherein, Y is selected from N or O, and Z is selected from CH or N;
$R_b$ and $R_c$ are each independently selected from hydrogen and $C_1$-$C_3$ alkyl;
$R_3$ is selected from $C_1$-$C_6$ alkyl; and the $C_1$-$C_6$ alkyl is optionally substituted by zero to three R';
$R_4$ is selected from Cl, Br and $C_1$-$C_6$ alkyl, and the $C_1$-$C_6$ alkyl is optionally substituted by zero to three R';
$R_5$ is selected from $C_1$-$C_6$ alkyl, and the $C_1$-$C_6$ alkyl is optionally substituted by zero to three R'; and
R' is selected from hydrogen, $C_1$-$C_6$ alkyl, F, Cl, Br, I, hydroxyl and amino.

In one or more embodiments of the present application, $R_2$ is selected from amino and

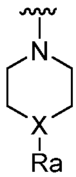

;

and the amino is optionally substituted by zero to three $R_a$; wherein, X is selected from CH, N or O;

$R_a$ is selected from

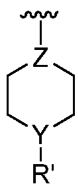

,

$C_1$-$C_3$ alkyl or $NR_dR_c$;
wherein, Y is selected from N or O, and Z is selected from CH or N;
$R_b$ and $R_c$ are each independently selected from hydrogen and $C_1$-$C_3$ alkyl;
$R_3$ is selected from $C_1$-$C_6$ alkyl; and the $C_1$-$C_6$ alkyl is optionally substituted by zero to three R';
$R_4$ is selected from Cl, Br and $C_1$-$C_6$ alkyl, and the $C_1$-$C_6$ alkyl is optionally substituted by zero to three R';
$R_5$ is selected from $C_1$-$C_6$ alkyl, and the $C_1$-$C_6$ alkyl is optionally substituted by zero to three R'; and
R' is selected from F, Cl, Br, I, hydroxyl and amino.

In one or more embodiments of the present application, $R_1$ is selected from methyl, ethyl, isopropyl, $CF_2H$ and $CH_2CF_3$;

$R_2$ is selected from

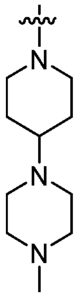, 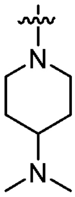, 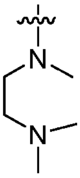, 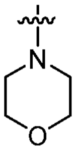, 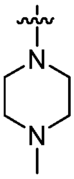 and 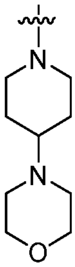;

$R_3$ is selected from methyl, ethyl and isopropyl;
$R_4$ is selected from Cl, Br, $CH_3$, $CF_3$ and $CH_2CF_3$; and
$R_5$ is selected from methyl, ethyl and isopropyl.

In one or more embodiments of the present application, $R_1$ is selected from methyl, ethyl, isopropyl, $CF_2H$ and $CH_2CF_3$;

$R_2$ is selected from

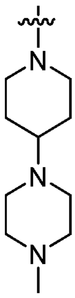, 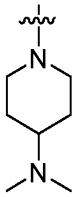, 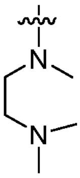, 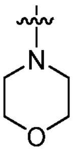, 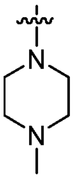 and 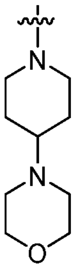;

$R_3$ is selected from methyl and ethyl;
$R_4$ is selected from Cl, Br, $CF_3$ and $CH_2CF_3$; and
$R_5$ is selected from methyl and ethyl.

The compound or pharmaceutically acceptable salt thereof in one or more embodiments of the present application, wherein:

$R_3$ is halogen;
$R_4$ is selected from halogen and $C_1$-$C_6$ alkyl; wherein, the $C_1$-$C_6$ alkyl is optionally substituted by zero to three R';
$R_5$ is selected from $C_1$-$C_6$ alkyl; and the $C_1$-$C_6$ alkyl is optionally substituted by zero to three R';
$R_6$ is hydrogen; and
R' is selected from hydrogen, $C_1$-$C_6$ alkyl, F, Cl, Br, I, hydroxyl and amino.

In one or more embodiments of the present application, $R_3$ is halogen;

$R_4$ is selected from halogen and $C_1$-$C_6$ alkyl; wherein the $C_1$-$C_6$ alkyl is optionally substituted by zero to three R';
$R_5$ is selected from $C_1$-$C_6$ alkyl; and the $C_1$-$C_6$ alkyl is optionally substituted by zero to three R';
$R_6$ is hydrogen; and
R' is selected from F, Cl, Br, I, hydroxyl and amino.

In one or more embodiments of the present application,

$R_1$ is selected from methyl, ethyl and isopropyl;
$R_2$ is selected from

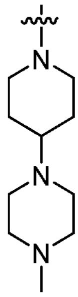, 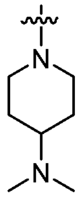, 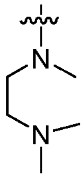, 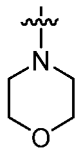, 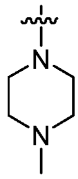 and 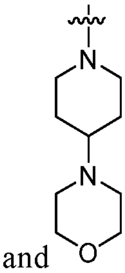;

$R_3$ is selected from Cl and Br;
$R_4$ is selected from Cl, Br, $CF_3$ and $CH_2CF_3$; and
$R_5$ is selected from methyl, ethyl and isopropyl.

In one or more embodiments of the present application, $R_1$ is selected from methyl, ethyl and isopropyl;

$R_2$ is selected from

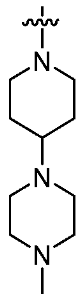, 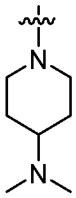, 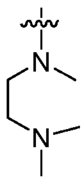, 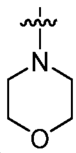, 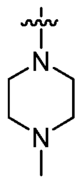 and 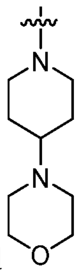;

$R_3$ is selected from Cl and Br;
$R_4$ is selected from Cl, Br, $CF_3$ and $CH_2CF_3$; and
$R_5$ is selected from methyl and ethyl.

In one or more embodiments of the present application,

$R_3$ is selected from $C_1$-$C_6$ alkyl; and the $C_1$-$C_6$ alkyl is optionally substituted by zero to three R';
$R_4$ is selected from halogen and $C_1$-$C_6$ alkyl; wherein, the $C_1$-$C_6$ alkyl is optionally substituted by zero to three R';
$R_5$ is selected from halogen;
$R_6$ is hydrogen; and
R' is selected from hydrogen, $C_1$-$C_6$ alkyl, F, Cl, Br, I, hydroxyl and amino.

In one or more embodiments of the present application, $R_3$ is selected from $C_1$-$C_6$ alkyl; and the $C_1$-$C_6$ alkyl is optionally substituted by zero to three R';

$R_4$ is selected from halogen and $C_1$-$C_6$ alkyl; wherein, the $C_1$-$C_6$ alkyl is optionally substituted by zero to three R';
$R_5$ is selected from halogen;
$R_6$ is hydrogen; and
R' is selected from F, Cl, Br, I, hydroxyl and amino.

In one or more embodiments of the present application,

$R_1$ is selected from methyl, ethyl and isopropyl;
$R_2$ is selected from

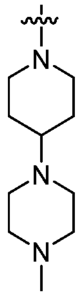, 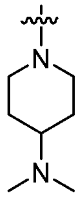, 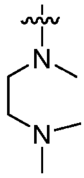, 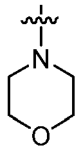, 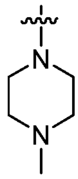 and 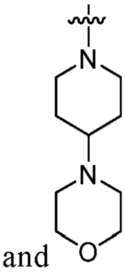;

$R_3$ is selected from methyl, ethyl and isopropyl;
$R_4$ is selected from Cl, Br, $CF_3$ and $CH_2CF_3$; and
$R_5$ is selected from F and Cl.

In one or more embodiments of the present application, $R_1$ is selected from methyl, ethyl and isopropyl;

$R_2$ is selected from

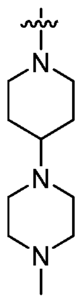, 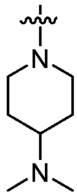, 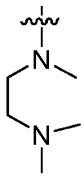, 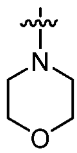, 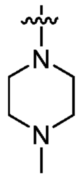 and 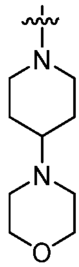;

$R_3$ is selected from methyl and ethyl;
$R_4$ is selected from Cl, Br, $CF_3$ and $CH_2CF_3$; and
$R_5$ is selected from F and Cl.

In one or more embodiments of the present application,

$R_1$ is selected from methyl, ethyl, isopropyl and $-CF_2H$;
$R_2$ is selected from

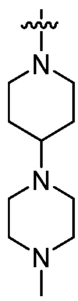, 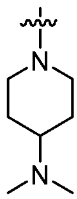, 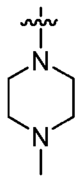 and 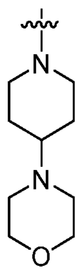;

$R_3$ is selected from methyl, ethyl and isopropyl;
$R_4$ is selected from Br; and
$R_5$ is selected from methyl, ethyl and isopropyl.

In one or more embodiments of the present application,

$R_1$ is selected from methyl, ethyl and isopropyl;
$R_2$ is selected from

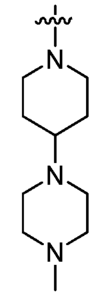, 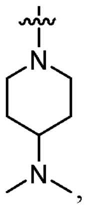, 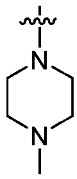 and 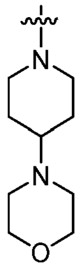;

$R_3$ is selected from methyl, ethyl and isopropyl;
$R_4$ is selected from Br; and
$R_5$ is selected from F and Cl.

The present invention provides the following compounds or pharmaceutically acceptable salts thereof:

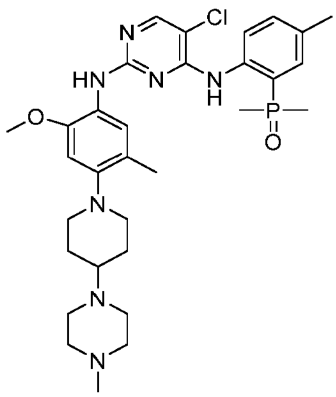, 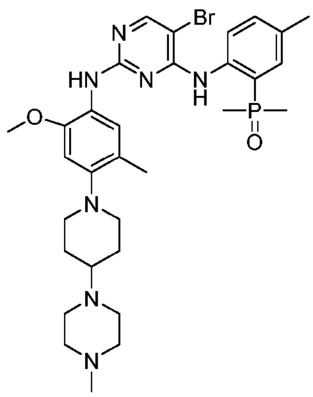, 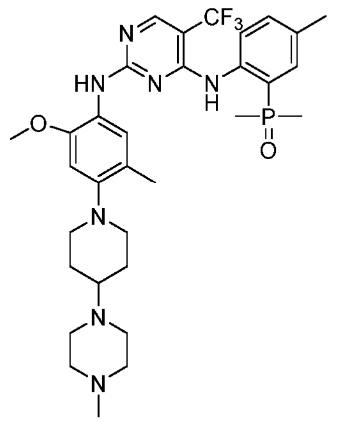,

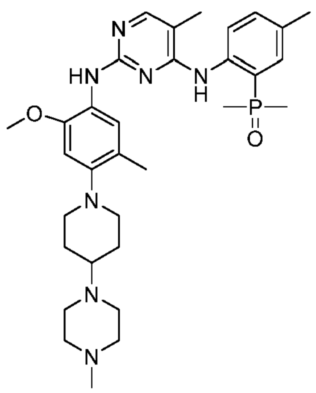, 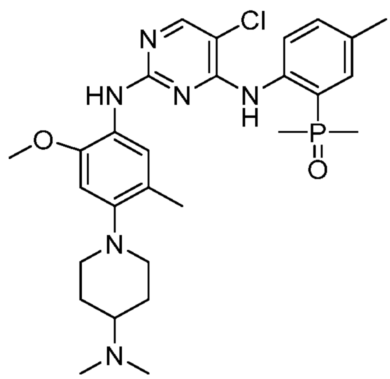, 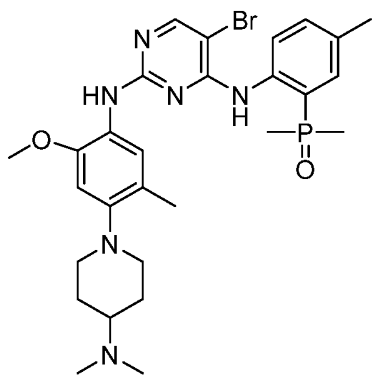,

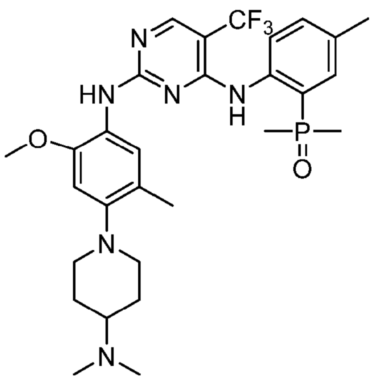, 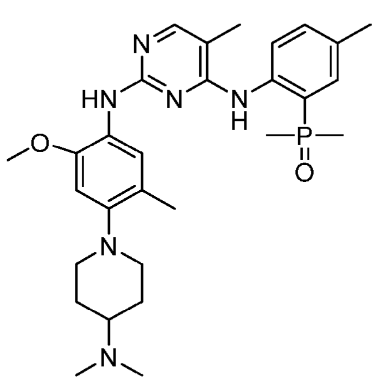, 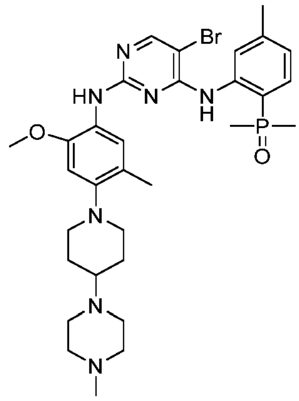,

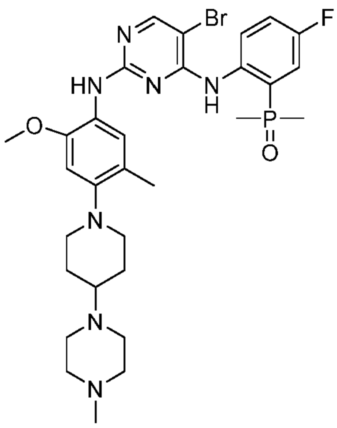, 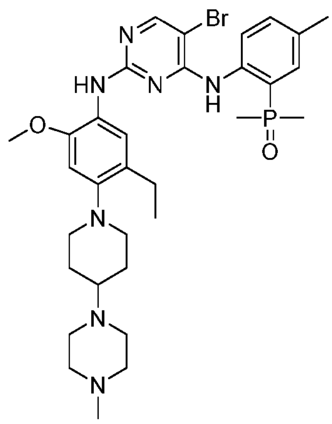, 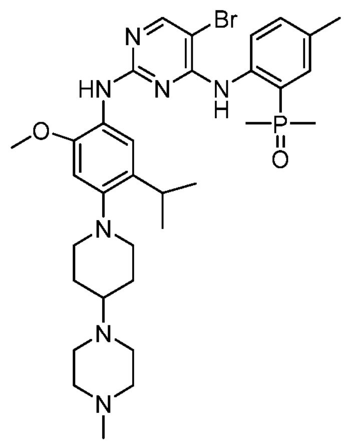,

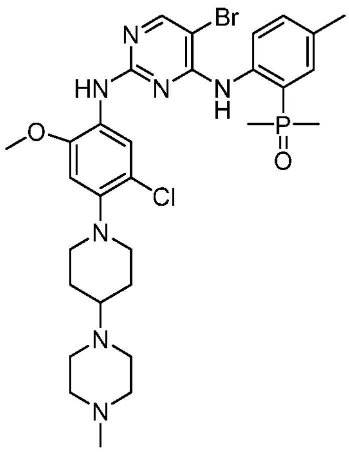, 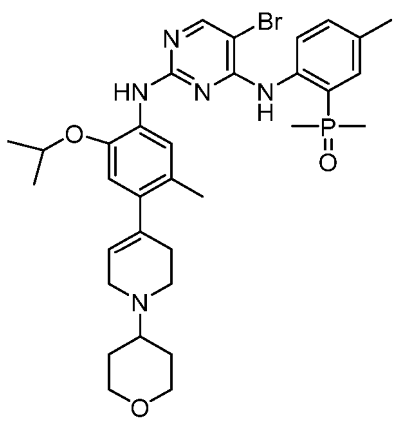,

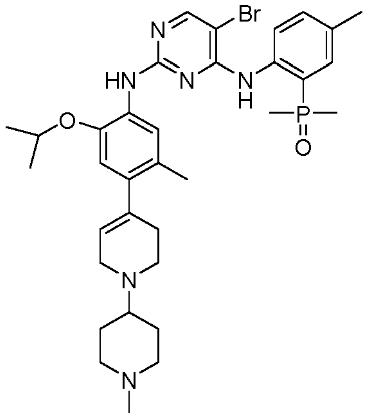 , 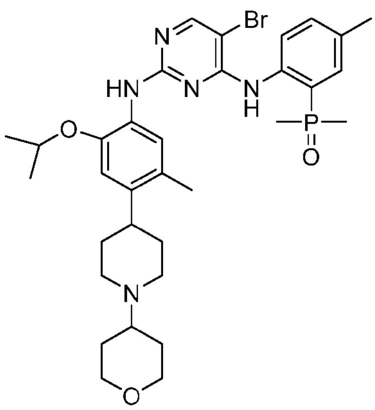 ,

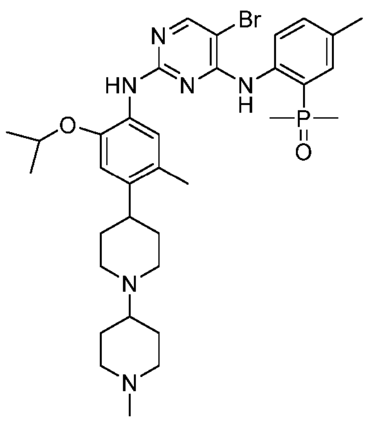 , 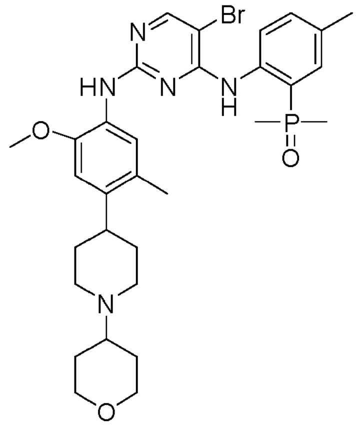 ,

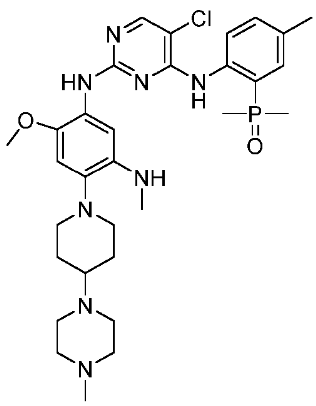 , 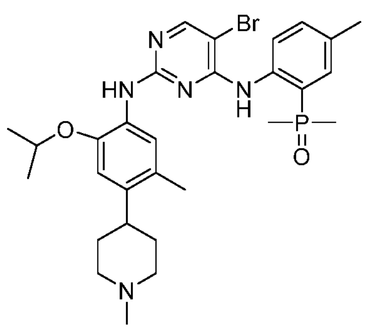 , 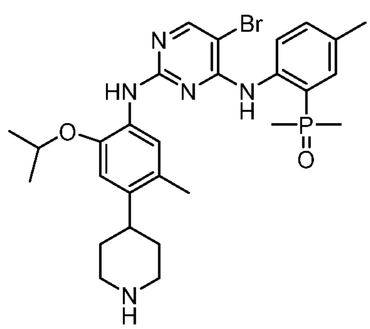 ,

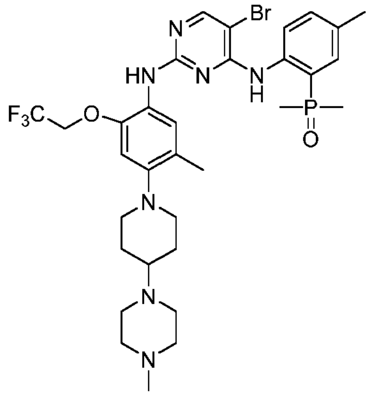 , 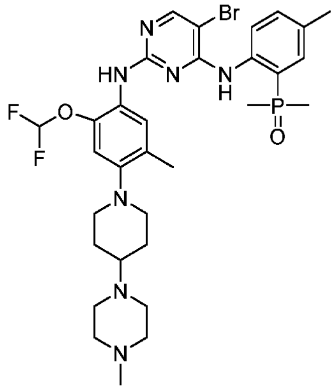 ,

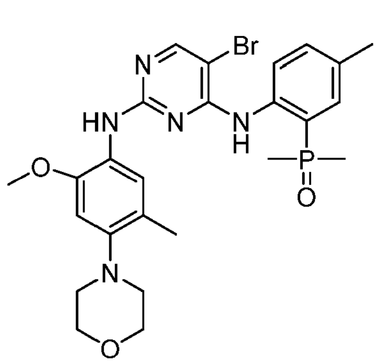,
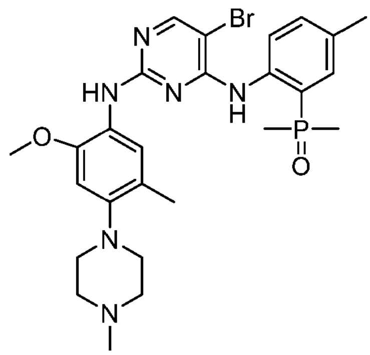,
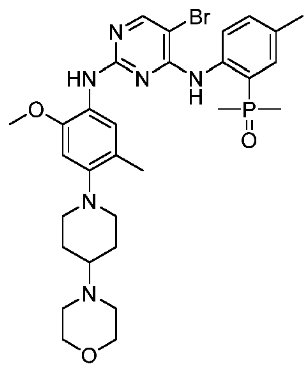,
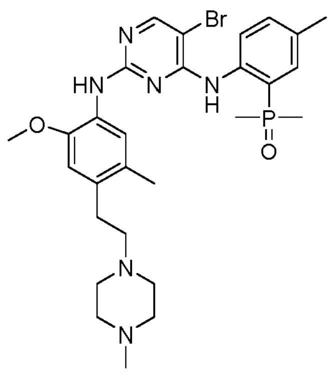,
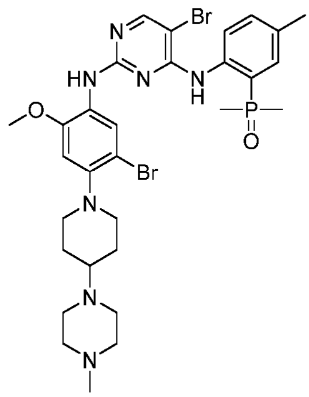,
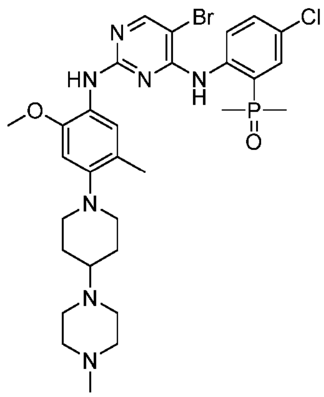,
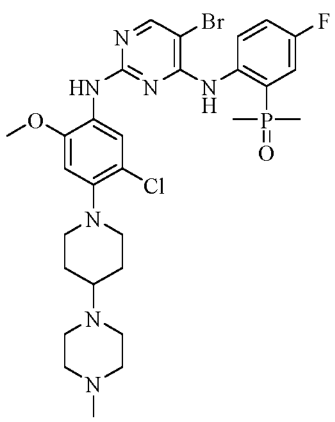,
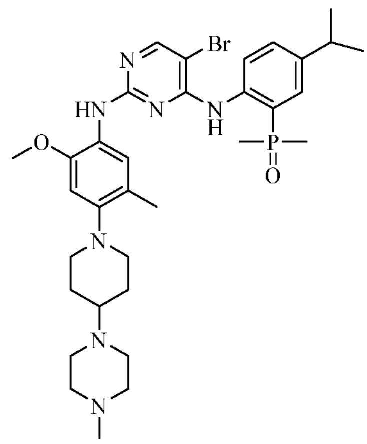,
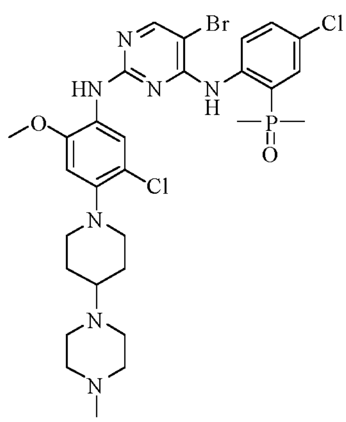

and

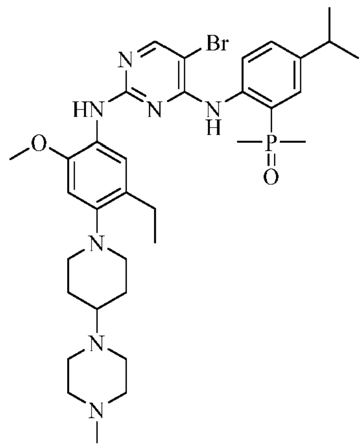

.

One or more embodiments of the present application provide a pharmaceutical composition comprising the compound according to the present application or the pharmaceutically acceptable salt thereof, and a pharmaceutically acceptable carrier.

One or more embodiments of the present application provide a pharmaceutical preparation comprising the compound according to the present application or the pharmaceutically acceptable salt thereof, and a pharmaceutically acceptable adjuvant.

One or more embodiments of the present application provide use of the compound according to the present application or the pharmaceutically acceptable salt thereof, or the pharmaceutical preparation above or the pharmaceutical composition above in preparing a medicament for preventing and/or treating a disease related to EGFR or ALK or EGFR and ALK.

In one or more embodiments, the disease related to EGFR or ALK or EGFR and ALK is cancer.

One or more embodiments of the present application provide use of the compound according to the present application or the pharmaceutically acceptable salt thereof, or the pharmaceutical preparation, or the pharmaceutical composition according to the present application in preparing a medicament for preventing and/or treating cancer.

One or more embodiments of the present application provide the compound according to the present application or the pharmaceutically acceptable salt thereof, or the pharmaceutical preparation above, or the pharmaceutical composition above for use as a medicament.

One or more embodiments of the present application provide a method of using the compound according to the present application or the pharmaceutically acceptable salt thereof, or the pharmaceutical preparation above, or the pharmaceutical composition above for preventing and/or treating cancer.

One or more embodiments of the present application provide use of the compound or the pharmaceutically acceptable salt thereof according to the present application, or the pharmaceutical preparation above, or the pharmaceutical composition above for preventing and/or treating a disease related to EGFR or ALK or EGFR and ALK.

One or more embodiments of the present application provide use of the compound according to the present application or pharmaceutically acceptable salt thereof, or the pharmaceutical preparation above, or the pharmaceutical composition above as an EGFR inhibitor, or an ALK inhibitor, or an EGFR and ALK inhibitor, or a protein kinase inhibitor. One or more embodiments of the present application provide use of the compound according to the present application or the pharmaceutically acceptable salt thereof, or the pharmaceutical preparation above, or the pharmaceutical composition above in preparing an EGFR inhibitor.

One or more embodiments of the present application provide use of the compound according to the present application or the pharmaceutically acceptable salt thereof, or the pharmaceutical preparation above, or the pharmaceutical composition above in preparing an ALK inhibitor.

One or more embodiments of the present application provide use of the compound according to the present application or the pharmaceutically acceptable salt thereof, or the pharmaceutical preparation above, or the pharmaceutical composition above in preparing an EGFR and ALK inhibitor.

In one or more embodiments of the present application, the medicament for treating cancer is an aryl phosphorus oxide compound with protein kinase inhibitor activity, comprising a medicament for preventing and/or treating a lung cancer, such as a medicament for preventing and/or treating multiple myeloma; and a medicament for preventing and/or treating lymphoma, such as a medicament for preventing and/or treating non-Hodgkin lymphoma, mantle cell lymphoma and follicular lymphoma; a medicament for preventing and/or treating leukemia; and a medicament for preventing and treating mantle cell tumor, breast cancer, liver cancer, colon cancer, cervical cancer, lung cancer, plasmoma, lymphoma, ovarian cancer, kidney cancer, gastric cancer, nasopharyngeal cancer, leukemia, melanoma, thyroid cancer, pancreatic cancer, adenocarcinoma or squamous cell carcinoma.

In one or more embodiments of the present application, the compound according to the present application can be used for treating lung cancer, plasmoma, mantle cell tumor, multiple myeloma, melanoma, breast cancer, liver cancer, cervical cancer, lymphoma, leukemia, ovarian cancer, kidney cancer, gastric cancer, nasopharyngeal cancer, thyroid cancer, pancreatic cancer, prostate cancer, adenocarcinoma, oral cancer, esophagus cancer, squamous cell carcinoma or colon cancer.

One or more embodiments of the present application further provide a method for treating and/or preventing a disease related to EGFR or ALK or EGFR and ALK. The method comprises administering the compound according to the present application or the pharmaceutically acceptable salt thereof, or the pharmaceutical preparation or the pharmaceutical composition according to the present application to a subject in need thereof.

One or more embodiments of the present application further provide a method for treating and/or preventing cancer or tumor. The method comprises administering the compound according to the present application or the pharmaceutically acceptable salt thereof, or the pharmaceutical preparation or the pharmaceutical composition according to the present application to a subject in need thereof.

One or more embodiments of the present application further provide use of the compound according to the present application or the pharmaceutically acceptable salt thereof, or the pharmaceutical preparation above, or the pharmaceutical composition above in preparing a protein kinase inhibitor.

One or more embodiments of the present application further provide a method for inhibiting an EGFR inhibitor, and/or an ALK inhibitor, or a protein kinase in vivo or in vitro. The method comprises administering the compound according to the present application or the pharmaceutically acceptable salt thereof, or the pharmaceutical preparation or the pharmaceutical composition according to the present application to a subject or a subject in need thereof.

Terms used in the technical solutions of the present application are explained below. As used in the specification and the appended claims, unless specified to the contrary, the terms of the present application have the meanings indicated below:

The term "compound" comprises all stereoisomers, geometric isomers and tautomers. The "compound" described herein may be asymmetric, e.g., having one or more stereoisomers. Unless otherwise indicated, all stereoisomers are included, for example, individual enantiomers and diastereomers or other stereoisomeric forms or mixtures thereof. The compounds containing asymmetric carbon atoms herein may be isolated in optically pure form or as racemates. The optically active pure form may be resolved from a racemic mixture or synthesized by using chiral materials or chiral reagents. The term "compound" as used herein also comprises a geometrical isomeric form, which refers to compounds having different cis-trans isomerisms and no chirality at double bonds or at ring substituents. The term "compound" as used herein also comprises a tautomer form. The tautomer forms may result from the exchange of one single bond with an adjacent double bond and the concomitant migration of one proton.

The compounds herein, whether intermediates or the compounds of formula (I), may also be isotopically labeled by substituting one or more atoms therein with atoms having different atomic masses or mass numbers. Such isotopically labeled (i.e., radiolabeled) compounds are considered to be within the scope herein. Examples of isotopes in the compounds herein comprise isotopes of carbon, nitrogen, oxygen, sulfur, fluorine, chlorine, and iodine, each having the same proton number but different mass numbers.

The term "halogen" refers to fluorine, chlorine, bromine or iodine.

The term "amino" refers to $-NH_2$.

The term "cyano" refers to -CN.

The term "nitro" refers to $-NO_2$.

The term "hydroxyl" refers to -OH.

The term "carboxyl" refers to -COOH.

The term "alkyl" refers to a saturated aliphatic hydrocarbyl group, and the term comprises straight-chain and branched-chain hydrocarbyl, for example, $C_1$-$C_{20}$ alkyl, and preferably $C_1$-$C_6$ alkyl. $C_1$-$C_{20}$ alkyl refers to alkyls having1 to 20 carbon atoms, such as alkyls having 1 carbon atom, 2 carbon atoms, 3 carbon atoms, 4 carbon atoms, 5 carbon atoms, 6 carbon atoms, 7 carbon atoms, 8 carbon atoms, 9 carbon atoms, 10 carbon atoms, 11 carbon atoms, 12 carbon atoms, 13 carbon atoms, 14 carbon atoms, 15 carbon atoms, 16 carbon atoms, 17 carbon atoms, 18 carbon atoms, 19 carbon atoms, or 20 carbon atoms. Non-limiting examples of alkyl include methyl, ethyl, n-propyl, isopropyl, n-butyl, isobutyl, sec-butyl, tert-butyl, n-pentyl, neopentyl, n-hexyl, and the like. The alkyl may be unsubstituted or substituted by one or more substituents comprising, but not limited to, alkyl, alkoxy, cyano, hydroxy, carbonyl, carboxyl, aryl, heteroaryl, amido, halogen, sulfonyl, sulfinyl, phosphonyl, and the like.

The term "cycloalkyl" refers to a cyclic alkyl having a single ring or multiple rings (comprising fused, bridged, and spiro ring systems), comprising cyclic alkyl composed of 3 to 8 carbon atoms (e.g., 3, 4, 5, 6, 7, or 8 carbon atoms) and hydrogen atoms. Non-limiting examples of cycloalkyl comprise cyclopropyl, cyclobutyl, cyclopentyl, cyclohexyl, spiro[3.4]octyl, bicyclo[1.1.1]pentyl, bicyclo[3.1.0]hexyl, and the like.

The term "cycloalkenyl" refers to cyclic or polycyclic hydrocarbyl with 3 to 13 carbon atoms, and preferably 5 to 8 carbon atoms, containing one or more double bonds. Cycloalkenyl groups may be substituted or unsubstituted.

Cycloalkenyl groups comprise, but are not limited to, cyclopentenyl, cyclohexenyl, and cyclooctenyl.

The term "alkenyl" refers to a hydrocarbyl that contains one or more double bonds in a straight or branched hydrocarbon chain. Alkenyl may be unsubstituted or substituted. Alkenyl may have 1 to 20 carbon atoms, and a numerical range such as "1 to 20" refers to each integer in the given range. For example, "1 to 20 carbon atoms" refers to alkenyls which may include 1 carbon atom, 2 carbon atoms, 3 carbon atoms, 4 carbon atoms, 5 carbon atoms, 6 carbon atoms, 7 carbon atoms, 8 carbon atoms, 9 carbon atoms, 10 carbon atoms, 11 carbon atoms, 12 carbon atoms, 13 carbon atoms, 14 carbon atoms, 15 carbon atoms, 16 carbon atoms, 17 carbon atoms, 18 carbon atoms, 19 carbon atoms, or 20 carbon atoms.

The term "alkynyl" refers to a hydrocarbyl that contains one or more triple bonds in a straight or branched hydrocarbon chain. Alkynyl may be unsubstituted or substituted. Alkynyl may have 1 to 20 carbon atoms, and a numerical range such as "1 to 20" refers to each integer in the given range. For example, "1 to 20 carbon atoms" refers to alkynyls which may include 1 carbon atom, 2 carbon atoms, 3 carbon atoms, 4 carbon atoms, 5 carbon atoms, 6 carbon atoms, 7 carbon atoms, 8 carbon atoms, 9 carbon atoms, 10 carbon atoms, 11 carbon atoms, 12 carbon atoms, 13 carbon atoms, 14 carbon atoms, 15 carbon atoms, 16 carbon atoms, 17 carbon atoms, 18 carbon atoms, 19 carbon atoms, or 20 carbon atoms.

The term "heteroaryl" refers to a monocyclic or fused ring having 5 to 12 ring atoms (e.g., 5, 6, 10, 12, 14 ring atoms) containing 1 to 4 (e.g., 1, 2, 3, or 4) heteroatoms selected from N, O and S, and the remaining ring atoms being C, and having a fully conjugated $\pi$-electron system comprising, but is not limited to, pyrrolyl, furanyl, thienyl, imidazolyl, oxazolyl, isoxazolyl, pyrazolyl, pyridinyl, pyrimidinyl, pyrazinyl, pyridazinyl, quinolinyl, isoquinolinyl, triazolyl, benzimidazole, benzotriazole, and the like. The heteroaryl may be unsubstituted or substituted, the substituents comprise, but are not limited to, alkyl, alkoxy, cyano, hydroxy, carbonyl, carboxyl, aryl, aralkyl, amido, halogen, sulfonyl, sulfinyl, phosphonyl, and the like. The term "substituted" as used herein means that any group is monosubstituted or polysubstituted by a specified substituent to the extent that such monosubstitution or polysubstitution (including multiple substitutions on the same moiety) is chemically permissible, and that each substituent may be at any available position on the group and may be attached through any available atom on the substituent. "Any available position" refers to any position on the group that is chemically available by methods known in the art or taught herein, and that does not result in an overly labile molecule. When there are two or more substituents on any group, each substituent is defined independently of any other substituent and thus may be the same or different.

The term "acyl" refers to hydrogen, alkyl, alkenyl, alkynyl, or aryl as a substituent attached through a carbonyl. Examples include formyl, acetyl, propionyl, benzoyl and acryloyl. The acyl may be substituted or unsubstituted.

The term "substituted" as used herein means that any group is monosubstituted or polysubstituted by a specified substituent to the extent that such monosubstitution or polysubstitution (comprising multiple substitutions on the same moiety) is chemically permissible, and that each substituent may be at any available position on the group and may be attached through any available atom on the substituent. "Any available position" refers to any position on the group that is chemically available by methods known in the art or taught herein, and that does not result in an overly labile molecule. When there are two or more substituents on any group, each substituent is defined independently of any other substituent and thus may be the same or different.

When a group is described as "optionally substituted by zero to six R'", it means that the group may be optionally substituted by one, two, three, four, five or six R' or not substituted by R', i.e., corresponding to zero R'. When a group is described as "optionally substituted by zero to three R'", it means that the group may be optionally substituted by one, two or three R' or not substituted by R', i.e., corresponding to zero R'. When a group is described as "optionally substituted by zero to three $R_a$", it means that the group may be optionally substituted by one, two or three $R_a$ or not substituted by Ra, i.e., corresponding to zero $R_a$.

When a group is described as "optionally substituted", then the group may be unsubstituted or substituted by one or more of the following substituents: hydrogen, fluorine, chlorine, bromine, iodine, nitro, trifluoromethyl, difluoromethyl, trifluoromethoxy, difluoromethoxy, methoxy, acyl, alkoxy, heterocycloalkyl, alkyl, alkenyl, alkynyl, cycloalkyl, cycloalkenyl, cycloalkynyl, aryl, heteroaryl, heterocyclic group, aralkyl, heteroaralkyl, (heterocyclic)alkyl, hydroxyl, aryloxy, sulfydryl, alkylthio, arylthio, cyano, halogen, thiocarbonyl, O-carbamoyl, N-carbamoyl, O-thiocarbamoyl, N-thiocarbamoyl, C-acylamino, N-acylamino, S-thioformamyl, N-thioformamyl, C-acyamino, N-acyamino, S-sulfonylamino, N-sulfonylamino, carboxyl, isocyanate, thiocyanate, isothiocyanate, silyl, alkylthio, sulfinyl, sulfonyl, haloalkyl, haloalkoxy and amino.

The term "pharmaceutically acceptable" means that a substance or composition must be compatible chemically and/or toxicologically, with other ingredients constituting a formulation and/or a mammal being treated therewith.

The "pharmaceutical preparation" mentioned in the present application may be a pharmaceutical composition directly or in combination with other active ingredients, together with pharmaceutically acceptable excipients or carriers. The preparation comprises tablets, pills, capsules, granules, suspensions, emulsions, and the like. The pharmaceutically acceptable adjuvant or carrier comprises a binder such as microcrystalline cellulose, tragacanth gum or gelatin; an excipient such as starch or lactose; a dispersing agent such as alginic acid, Primogel or corn starch; a lubricant such as magnesium stearate; a glidant such as colloidal silicon dioxide; a sweetening agent such as sucrose or saccharin; or a

flavoring agent such as peppermint oil, methyl salicylate or orange flavoring; a non-aqueous solvent such as dimethyl sulfoxide, alcohol, propylene glycol, polyethylene glycol, a vegetable oil such as olive oil, and an injectable organic ester such as ethyl oleate; an aqueous carrier such as a mixture of alcohol and water, buffered media and saline; and preservatives, antibacterial agents, antioxidants, chelating agents, dyes, pigments or perfumes, and the like.

The cancer in one or more embodiments of the present application is in particular any one or more of plasmoma, mantle cell tumor, multiple myeloma, melanoma, breast cancer, liver cancer, cervical cancer, lung cancer, lymphoma, leukemia, ovarian cancer, kidney cancer, gastric cancer, nasopharyngeal cancer, thyroid cancer, pancreatic cancer, prostate cancer, adenocarcinoma, oral cancer, esophageal cancer, squamous cell carcinoma or colon cancer.

Other anti-tumor drugs which may be combined with the compounds of the present application to form the pharmaceutical compositions include: a cytotoxic drug, a hormone drug, an antimetabolite, a tumor-targeted drug, an adjuvant therapy drug, and the like. The cytotoxic drug comprises, for example, carboplatin, cisplatin, irinotecan, paclitaxel, fluorouracil, cytarabine, lenalidomide, and retinoic acid; the hormone drug comprises, for example, dexamethasone, fulvestrant, tamoxifen, and the like; the antimetabolite comprises fluorouracil, methotrexate, furan fluorouracil and cytarabine; molecular targeted drug comprises, for example, imatinib, erlotinib, lapatinib and the like, which are tinib drugs; a PARP inhibitor comprises, for example, Olaparib, Rubraca, Zejula and the like; and the adjuvant therapy drug comprises, for example, recombinant human granulocyte colony stimulating factor, erythropoietin, pamidronate disodium, zoledronic acid, and the like. In addition, anti-tumor biological drugs such as Keytruda, Opdiv, Teentriq, Imfinzi, Bavencio, and the like, are also included.

Technical Effects

The compounds in one or more embodiments of the present invention can effectively inhibit the activity of various EGFR drug-resistant mutations (such as $EGFR^{del19}$, $EGFR^{del19/T790M}$, $EGFR^{del19/C797S}$, $EGFR^{T790M/L858R}$, $EGFR^{L858R/C797S}$, $EGFR^{del19/T790M/C797S}$, and $EGFR^{L858R/T790M/C797S}$), and the compounds in one or more embodiments of the present invention have good inhibitory effects on ALK fusion genes and mutations thereof. The compounds in one or more embodiments of the present invention have inhibitory activity on both EGFR drug-resistant mutations and ALK fusion genes and mutations thereof.

In one or more embodiments, for a plurality of EGFR drug-resistant mutations characterized by T790M mutation, the compounds of the present invention exhibit significantly lower $IC_{50}$ values and/or $GI_{50}$ values than the compounds disclosed in the prior art, and exhibit better inhibitory activity; and the plurality of EGFR drug-resistant mutations characterized by T790M mutation comprise EGFR-T790M/Del19, EGFR-T790M/L858R, EGFR-C797S/T790M/L858R, EGFR-C797S/T790M/Del19, and the like.

In one or more embodiments, for a plurality of EGFR drug-resistant mutations characterized by C797S mutation, the compounds of the present invention exhibit significantly lower $IC_{50}$ values and/or $GI_{50}$ values than the compounds disclosed in the prior art, and exhibit better inhibitory activity; and the plurality of EGFR drug-resistant mutations characterized by C797S mutation include EGFR-C797S/Del19, EGFR-C797S/L858R, EGFR-C797S/T790M/L858R, EGFR-C797S/T790M/Del19, and the like.

In one or more embodiments, for a plurality of EGFR drug-resistant mutations characterized by L858R mutation, the compounds of the present invention exhibit significantly lower $IC_{50}$ values and/or $GI_{50}$ values than the compounds disclosed in the prior art, and exhibit better inhibitory activity. For a plurality of EGFR drug-resistant mutations characterized by Del19 mutation, the compounds of the present invention exhibit significantly lower $IC_{50}$ values and/or $GI_{50}$ values than the compounds disclosed in the prior art, and exhibit better inhibitory activity.

In one or more embodiments, the compounds in one or more embodiments of the present invention also exhibit greater selectivity for mutant EGFR and wild-type EGFR than the compounds disclosed in the prior art.

In one or more embodiments, the compounds in one or more embodiments of the present invention exhibit significantly lower $IC_{50}$ values and/or $GI_{50}$ values for ALK mutant genes such as EML4-ALK, EML4-ALK-L1196M, and the like, and exhibit better inhibitory activity than the compounds disclosed in the prior art.

In one or more embodiments, the compounds of the present application exhibit unexpected activity in the EGFR drug-resistant mutation inhibition activity assay relative to the compounds of the comparative examples, while also exhibiting significant and unexpected inhibitory activity on ALK fusion gene and mutants.

In one or more embodiments, the compounds of the present application exhibit greater safety and have significantly improved tolerated doses relative to the compounds of the comparative examples.

The compounds of one or more embodiments of the present application have good physicochemical properties and high stability.

EXAMPLES

The present application is further described below in conjunction with the examples below, which are set forth to

provide further illustration of the practice of the present application, but it should be noted that the examples described below are exemplarily, which are only used to illustrate the present application, but are not to be construed as limiting the present application. Modifications and substitutions thereof by those skilled in the art are also within the scope of the present application as determined by the appended claims. The reagents used in the examples of the present application are all commercially available.

**Example 1**

## Compound 1A:

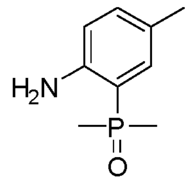

2-iodo-4-methylaniline (10 g, 42.9 mmol), $K_3PO_4$ (10.9 g, 51.5 mmol), Xantphos (2.48 g, 4.3 mmol), $Pd(OAc)_2$ (0.96 g, 4.3 mmol), dimethylphosphine oxide (5 g, 64.4 mmol) and 100 mL of DMF were added into a 500mL three-necked flask, then the reaction solution was heated to 120°C and reacted for about 3 hours until the reaction was completed by TLC test. The reaction solution was cooled to room temperature, subjected to suction filtration, and added with 600 mL of $H_2O$ to precipitate a large amount of yellow solids which were subjected to suction filtration. The filtrate was extracted with 500 mL of ethyl acetate thrice, the organic phases were combined, washed with 250 mL of saturated aqueous solution of sodium chloride twice, dried with anhydrous sodium sulfate and filtered, and then the filtrate was concentrated to obtain compound 1A which was continuously put into the next reaction without purification. $^1$H NMR (400MHz, DMSO-$d_6$) δ=9.21(brs, 2H), 7.57 (d, *J*=13.6, 1H), 7.43 (d, *J*=8.4, 1H), 7.26-7.23 (m, 1H), 2.33 (s, 3H), 1.85 (s, 3H), 1.82 (s, 3H).

## Compound 1B:

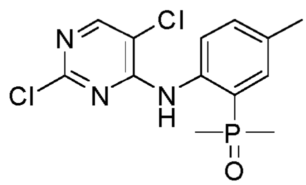

The compound 1A obtained in the above step, 2,4, 5-trichloropyrimidine (11.80 g, 64.3 mmol), $K_2CO_3$ (7.76 g, 128.73 mmol), $nBu_4NHSO_4$(1.45 g, 4.29 mmol) and 100 mL of DMF were added into a 500mL three-necked flask, then the reaction solution was heated to 65°C and reacted for about 4.5 hours until TLC test showed that the reaction was completed. The reaction solution was cooled to room temperature and added with 200 mL of $H_2O$ to precipitate a large amount of yellow solids which were continuously stirred for about 0.5 hour and then subjected to suction filtration. The filter cake was washed with 100 mL of $H_2O$, and dried to obtain compound 1B (8.57 g, wherein the total yield of the two steps was 60.5%). $^1$H NMR (400 MHz, DMSO) δ=11.62 (s, 1H), 8.40 (s, 1H), 8.27 (dd, *J*=8.4, 4.4 Hz, 1H), 7.44 (m, 2H), 2.33 (s, 3H), 1.81 (s, 3H), 1.77 (s, 3H).

## Compound 1C:

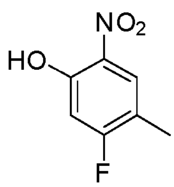

3-fluoro-4-methylphenol (10 g, 79.28 mmol) and benzyltriethylammonium chloride (1.77 g, 7.93 mmol) were weighed and added into a 250mL three-necked flask, and then dissolved by adding 80 mL of dichloromethane with stirring. Nitric acid (14.7 g, 158.57 mmol) was slowly added dropwise into the above reaction solution at 0-10°C. After the dropwise

addition was completed, the reaction mixture was continuously stirred for about 0.5 hour until TLC test showed that the reaction was completed. 120 mL of saturated aqueous solution of sodium bicarbonate was then added slowly, then the reaction solution was separated, and the organic phases were then washed with 40 mL of $H_2O$, and 40 mL of saturated sodium chloride in turn.

The aqueous solution was washed, and then the organic phases were concentrated, separated and purified through column chromatography isolation (PE/EA=8/1) to obtain compound 1C (8 g, yield 59.2%). $^1$H NMR (400MHz, $CDCl_3$) δ=10.66 (d, *J*=0.8 Hz, 1H), 8.02 (d, *J*=7.6 Hz, 1H), 6.83 (d, *J=10.0* Hz, 1H), 2.28 (d, *J*=0.8 Hz, 3H).

**Compound 1D:**

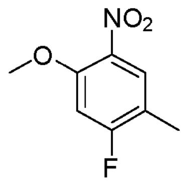

Compound 1C (2 g, 11.68 mmol), $K_2CO_3$ (2.42 g, 17.54 mmol), methyl iodide (1.08 mL, 17.54 mmol) and 30 mL of DMF were weighed and added into a 100mL three-necked flask, and stirred overnight at room temperature until TLC test showed that the reaction was completed. Then, 60 mL of water was added to the reaction solution to precipitate a large amount of off-white solids which were continuously stirred for about 0.5 hour and then subjected to suction filtration. The filter cake was filtered, washed with water, and dried to obtain compound 1D (1.59 g, yield 73.6%). $^1$H NMR(400MHz, $CDCl_3$) δ = 7.85(d, *J*=7.6Hz, 1H), 6.79(d, *J=11.2Hz,* 1H), 3.05(s, 3H), 2.27(d, *J=1.6Hz,* 3H).

**Compound 1E:**

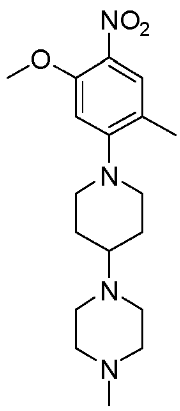

Compound 1D (4.5 g, 24.3 mmol), $K_2CO_3$ (6.71 g, 48.6 mmol), 1-methyl-4-(4-piperidinyl)piperazine (6.67 g, 36.4 mmol) and 67.5 mL of DMF were weighed and added into a 250mL three-necked flask, then the reaction solution was heated to 120°C and reacted for 4 hours with stirring until TLC test showed that the reaction was completed. Then, the reaction solution was cooled to room temperature and added with 130 mL of $H_2O$ to precipitate a large amount of solids which were continuously stirred for about 0.5 hour and then subjected to suction filtration. The filter cake was filtered, washed with water, and dried to obtain compound 1E (4.82 g, yield 56.8%). MS -ESI (m/z): 349.2248 $(M+H)^+$.

**Compound 1F:**

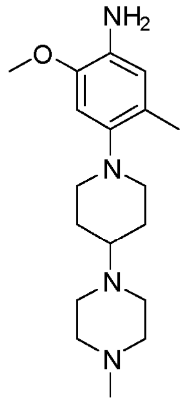

Compound 1E (4.5 g, 12.91 mmol), 0.45 g of 5% Pd/C and 90 mL of methanol were weighed and added into a 250mL single-necked flask, replaced with hydrogen for 2-3 times, and stirred overnight at room temperature in a hydrogen atmosphere (normal pressure) until TLC test showed that the reaction was completed. Then, after diatomite filtration, the filtrate was concentrated in vacuum, separated and purified through column chromatography (DCM/MeOH=8/1) to obtain compound 1F (4.02 g, yield 97.8%). MS -ESI (m/z): 319.2509 $(M+H)^+$. $^1H$ NMR (400 MHz, $CDCl_3$) δ=6.57 (d, *J*=4.2 Hz, 2H), 3.83 (s, 3H), 3.08 (d, *J*=8.4 Hz, 2H), 2.81 - 2.45 (m, 10H), 2.32 (s, 4H), 2.17 (s, 3H), 2.01 - 1.87 (m, 2H), 1.70 - 1.68 (m, 2H). MS -ESI (m/z): 319.2509 $(M+H)^+$.

**Compound 1:**

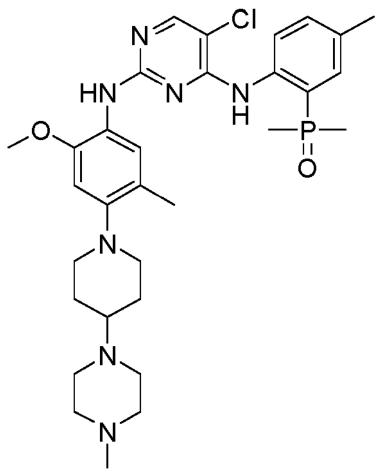

Compound 1B (500 mg, 1.51 mmol), compound 1F (667 mg, 2.12 mmol), 15% hydrogen chloride ethanol solution (960 mg, 3.64 mmol), and 7.5 mL of ethylene glycol monomethyl ether were added into a reaction flask, sealed and reacted at 120°C for 5-6 hours. After TLC test showed that the reaction was completed, the reaction solution was cooled to room temperature, and then 15 mL of saturated $NaHCO_3$ aqueous solution was added to precipitate a large amount of solids which were continuously stirred for 0.5 hour and then subjected to suction filtration. The filter cake was washed with water, and the filter cake was pulped with 9 mL of mixed solvent ($EtOH/H_2O$=1/2) and purified to obtain compound 1 (707 mg, yield 76.3%). $^1H$ NMR (400 MHz, $CDCl_3$) δ=10.57 (s, 1H), 8.45 (dd, *J*=8.4, 4.8 Hz, 1H), 8.05 (m, 2H), 7.33 (m, 2H), 7.08 (dd, *J=14.4,* 1.2 Hz, 1H), 6.62 (s, 1H), 3.86 (s, 3H), 3.15 (d, *J=11.6* Hz, 2H), 2.81 - 2.59 (m, 6H), 2.51 (m, 2H), 2.37 (s, 3H), 2.31 (s, 3H), 2.18 (s, 3H), 2.12 (s, 3H), 1.96 (m, 2H), 1.85 (s, 3H), 1.82 (s, 3H),1.72 (m, 2H). MS-ESI (m/z): 612.3098 $(M+H)^+$.

**Example 2**

Compound 2B:

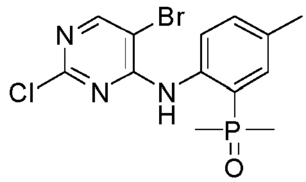

Compound 1A (3.00 g,16.38 mmol), 5-bromo-2,4-dichloropyrimidine (5.6 g, 24.57 mmol), $K_2CO_3$ (6.79 g, 49.14 mmol), $nBu_4NHSO_4$ (0.56 g, 1.638 mmol) and 60 mL of DMF were added into a 250mL three-necked flask, and heated to 65°C and reacted for about 4.5 hours until TLC test showed that the reaction was completed. Then, the reaction solution was cooled to room temperature and added with 200 mL of $H_2O$ to precipitate a large amount of yellow solids which were continuously stirred for about 0.5 hour and then subjected to suction filtration. The filter cake was washed with100 mL of $H_2O$, and dried to obtain compound 2B (4.5 g, yield 73.4%). $^1H$ NMR (400 MHz, $CDCl_3$) δ=11.19 (s, 1H), 8.43 (dd, *J*=8.4, 4.8 Hz, 1H), 8.32 (s, 1H), 7.41 (d, *J*=8.8 Hz, 1H), 7.08 (m, 1H), 2.38 (s, 3H), 1.86 (s, 3H), 1.83 (s, 3H). MS-ESI (m/z): 395.9642 $(M+Na)^+$.

Compound 2:

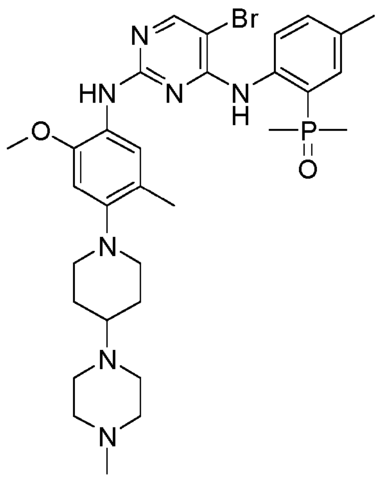

Compound 2B (375 mg, 1.00 mmol), compound 1F (414 mg, 1.30 mmol), 15% hydrogen chloride ethanol solution (730 mg, 3.00 mmol) and 4.0 mL of ethylene glycol monomethyl ether were added into a reaction flask, sealed and reacted at 120°C for 5-6 hours. After TLC test showed that the reaction was completed, the reaction solution was cooled to room temperature, and then 50 mL of 3% $K_2CO_3$ aqueous solution and 50 mL of dichloromethane were added, stirred for 10 minutes, and then system was allowed to settle for liquid separation. The upper aqueous phase was continuously washed once with 50 mL of dichloromethane, and the two dichloromethane phases were combined. Then, the dichloromethane phase was washed with 50 mL of saturated salt solution, dried with anhydrous sodium sulfate and filtered, the filtrate was concentrated and purified through column chromatography to obtain compound 2 (360 mg, yield 54.83%). $^1H$ NMR (400 MHz, $CDCl_3$) δ=10.29 (s, 1H), 8.32 (dd, *J*=8.8, 4.8 Hz, 1H), 8.18 (s, 1H), 8.01 (s, 1H), 7.44 - 7.30 (m, 2H), 7.11 (d, *J*=14.0Hz, 1H), 6.61 (s, 1H), 3.85 (s, 3H), 3.15 (d, *J*=11.6 Hz, 2H), 2.80-2.52 (m, 11H), 2.38 (s, 3H), 2.35 (s, 3H), 2.14 (s, 3H), 1.96 (d, *J=11.0* Hz, 2H), 1.85 (s, 3H), 1.82 (s, 3H), 1.78 - 1.68 (m, 2H). MS-ESI (m/z): 656.2459 $(M+H)^+$.

**Example 3**

Compound 3B:

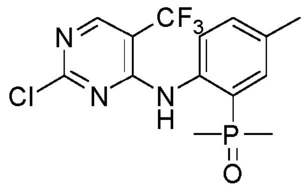

Compound 1A (420 mg, 2.29 mmol), 5-trifluoromethyl-2,4-dichloropyrimidine (745 mg, 3.43 mmol), $K_2CO_3$ (949 mg, 6.87 mmol), $nBu_4NHSO_4$ (78 mg, 0.23 mmol) and 8.5 mL of DMF were added into a 50 mL three-necked flask, then the reaction solution was heated to 65°C and reacted for about 4.5 hours until TLC test showed that the reaction was completed. The reaction solution was cooled to room temperature, and added with 18.5 mL of $H_2O$ to precipitate a large amount of yellow solids which were continuously stirred for about 0.5 hour and then subjected to suction filtration. The filter cake was washed with 20 mL of $H_2O$, and dried to obtain compound 3B (466 mg, yield 56.0%). $^1$H NMR (400 MHz, $CDCl_3$) δ=11.49 (s, 1H), 8.58 (s, 1H), 8.50-8.47 (dd, *J*=8.4Hz, 4.4Hz, 1H), 7.39 (d, *J*=8.4Hz, 1H), 7.08-7.04 (dd, *J*=14.4Hz, 1.2Hz, 1H), 2.37 (s, 3H), 1.87 (s, 3H), 1.84 (s, 3H). MS-ESI (m/z):364.0593$(M+H)^+$; 386.0413$(M+Na)^+$.

**Compound 3:**

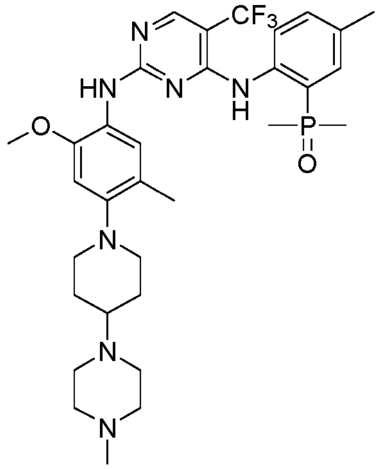

Compound 3B (181 mg, 0.50 mmol), compound 1F (207 mg, 0.65 mmol), 15% hydrogen chloride ethanol solution (365 mg, 1.50mmol) and 3.0 mL of ethylene glycol monomethyl ether were added into a reaction flask, sealed and reacted at 120°C for 5-6 hours. After TLC test showed that the reaction was completed, the reaction solution was cooled to room temperature, and then 50 mL of 3% $K_2CO_3$ aqueous solution and 50 mL of dichloromethane were added, stirred for 10 minutes, and then the system was allowed to settle for liquid separation. The upper aqueous phase was continuously washed once with 50 mL of dichloromethane, and the two dichloromethane phases were combined. Then, the dichloromethane phases were washed with 50 mL of saturated salt solution, dried with anhydrous sodium sulfate and filtered. The filtrate was concentrated and purified through column chromatography to obtain compound 3 (70 mg, yield 21.68%). $^1$H NMR (400 MHz, $CDCl_3$) δ=10.51 (s, 1H), 8.30 (s, 2H), 8.10 (s, 1H), 7.50 (s, 1H), 7.22 (d, *J*=8.4 Hz, 1H), 7.07 (d, *J=14.0* Hz, 1H), 6.64 (s, 1H), 3.87 (s, 3H), 3.19 (d, *J=10.4* Hz, 2H), 3.08 - 2.12 (m, 20H), 1.99 (d, *J*=10.8 Hz, 2H), 1.79 (m, 8H). MS-ESI (m/z): 646.3213 $(M+H)^+$.

**Example 4**

**Compound 4B:**

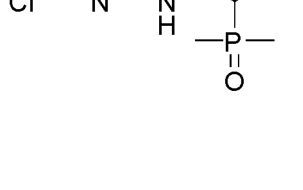

A mixed solvent of DMF/DMSO (15 mL/1.5 mL) was added into a 50mL single-necked flask, and cooled in an ice bath, then NaH (0.66 g, 16.38 mmol) was added into the mixed solvent above in batches, stirred for 5-10 minutes, then dropwise added with a DMF/DMSO (9 mL/1 mL) mixed solution of the compound 1A (1.0 g, 5.46 mmol) in Example 1, kept stirring at a low temperature for 30 minutes after the dropwise addition was completed, then dropwise added with a mixed solution of DMF/DMSO (9 mL/1 mL) of 5-methyl-2,4-dichloropyrimidine (1.33 g, 8.16 mmol), slowly heated to room temperature after the addition was completed, and reacted overnight at room temperature. After TLC test showed that the reaction was completed, 90 mL of $H_2O$ was added and then 25 mL of ethyl acetate was added for extraction, then the aqueous phases were extracted with 25 mL of ethyl acetate twice, the organic phases were combined and then washed with 50 mL of water twice, washed with 50 mL of saturated salt solution twice, the organic phases were dried with anhydrous sodium sulfate, and then filtered. The filtrate was concentrated, and purified through column chromatography to obtain compound 4B (1.2 g, yield 70.59%). $^1$H NMR (400 MHz, $CDCl_3$) $\delta$ = 10.85 (s, 1H), 8.62 (dd, *J*= 8.8, 4.8 Hz, 1H), 7.99 (s, 1H), 7.39 (d, *J* = 8.8 Hz, 1H), 7.03 (m, 1H), 2.36 (s, 3H), 2.24 (s, 3H), 1.85 (s, 3H), 1.82 (s, 3H). MS-ESI (m/z): 310.0874 $(M+H)^+$.

## Compound 4:

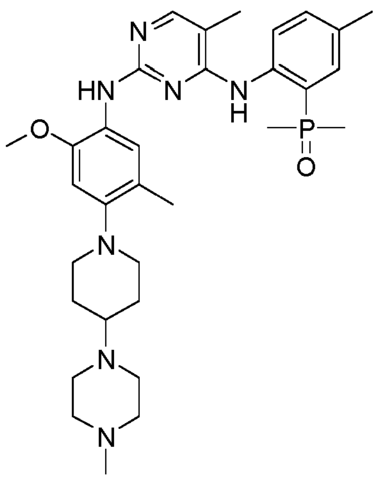

Compound 4B (500 mg, 1.61 mmol), compound 1F (461 mg, 1.45 mmol), 15% hydrogen chloride ethanol solution (1.18 g, 4.83 mmol) and 7.5 mL of ethylene glycol monomethyl ether were added into a reaction flask, sealed and reacted at 100°C for 5 hours. After TLC test showed that the reaction was completed, the reaction solution was cooled to room temperature, and then 15 mL of saturated $NaHCO_3$ aqueous solution and 10 mL of water were added, then 10 mL of dichloromethane was added to stir and extract. Then the aqueous phases were extracted with dichloromethane twice, the organic phases were combined, washed with 15 mL of water twice, and then washed with 15 mL of saturated sodium chloride twice, dried with anhydrous sodium sulfate, filtered, and concentrated under reduced pressure to obtain oily substance, and then pulped and purified by a mixed solvent (EA/PE=3/2) to obtain compound 4 (120 mg, yield 14.0%). $^1$H NMR (400 MHz, $CDCl_3$) $\delta$=10.17 (s, 1H), 8.59 (dd, *J*=8.4, 4.8 Hz, 1H), 8.14 (s, 1H), 7.91 (s, 1H), 7.32 (d, *J*=8.8 Hz, 1H), 7.26 (s, 1H), 7.04 (d, *J*=13.4 Hz, 1H), 6.63 (s, 1H), 3.86 (s, 3H), 3.16 (d, *J*=12.0 Hz, 2H), 2.67 (m, 9H), 2.36 (m, 8H), 2.20 (d, *J*=6.4 Hz, 6H), 1.97 (m, 2H), 1.84 (s, 3H), 1.81 (s, 3H), 1.79 - 1.69 (m, 2H). MS-ESI (m/z): 592.3526 $(M+H)^+$.

**Example 5**

**Compound 5E:**

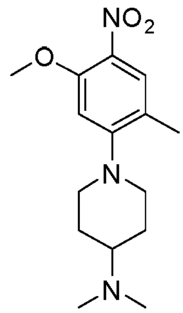

Compound 1D (1.5 g, 8.10 mmol), $K_2CO_3$ (2.24 g, 16.20 mmol), 4-dimethylaminopiperidine (1.56 g, 12.15 mmol) and 30 mL of DMF are weighed and added into a 100mL single-necked flask, then the reaction solution was heated to 120°C and reacted for about 4 hours with stirring until TLC test showed that the reaction was completed. Then, the reaction solution was cooled to room temperature, added with 60 mL of $H_2O$ to precipitate a large amount of solids which were continuously stirred for about 0.5 hour and then subjected to suction filtration. The filter cake was filtered, washed with water, and dried to obtain compound 5E (1.46 g, yield 61.3%). MS-ESI (m/z): 294.1825 $(M+H)^+$.

**Compound 5F:**

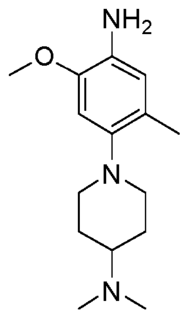

Compound 5E (1.4 g, 4.77 mmol), 0.52 g of 5% Pd/C and 28 mL of methanol were weighed and added into a 250mL single-necked flask, replaced with hydrogen for 2-3 times, and stirred overnight at room temperature in a hydrogen atmosphere (normal pressure) until TLC test showed that the reaction was completed. Then, after diatomite filtration, the filtrate was concentrated in vacuum, separated and purified through column chromatography (DCM/MeOH=8/1) and to obtain compound 5F (918 mg, yield 73.0%). MS-ESI (m/z): 264.2065 $(M+H)^+$.

**Compound 5:**

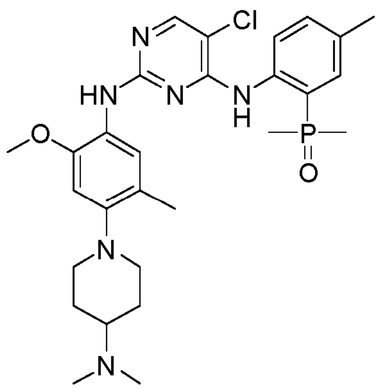

Compound 1B (400 mg, 1.21 mmol), compound 5F (383 mg, 1.45 mmol), 15% hydrogen chloride ethanol solution (884 mg, 3.63 mmol) and 6.0 mL of ethylene glycol monomethyl ether were added into a reaction flask, sealed and reacted at 120°C for 5-6 hours until TLC test showed that the reaction was completed. Then, the reaction solution was cooled to room temperature, and added with 15mL of saturated $NaHCO_3$ aqueous solution and 10 mL of $H_2O$ to precipitate a large

amount of solids which were continuously stirred for about 0.5 hour and then subjected to suction filtration. The filter cake was washed with water, and then pulped with 6 mL of mixed solvent (PE/EA=5/1) and purified to obtain compound 5 (410 mg, yield 61.5%). $^1$H NMR (400 MHz, $CDCl_3$) δ=10.58 (s, 1H), 8.45 (dd, *J*=8.4, 4.8 Hz, 1H), 8.06 (m, 2H), 7.33 (m, 2H), 7.09 (d, *J=14.4* Hz, 1H), 6.64 (s, 1H), 3.86 (s, 3H), 3.15 (d, *J*=12.0 Hz, 2H), 2.65 (m, 2H), 2.37 (m, 9H), 2.27 (m, 1H), 2.19 (s, 3H), 1.93 (m, 2H), 1.85 (s, 3H), 1.82 (s, 3H),1.70 (m, 2H). MS-ESI (m/z): 557.2571 $(M+H)^+$.

**Example 6**

**Compound 6**

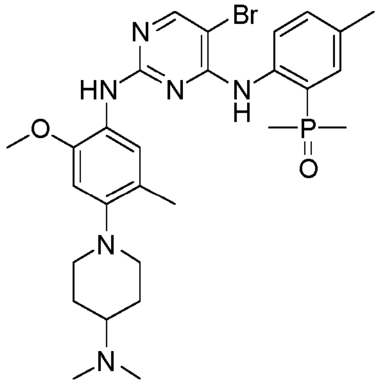

Compound 2B (400 mg, 1.07 mmol), compound 5F (422 mg, 1.60 mmol), 15% hydrogen chloride ethanol solution (779 mg, 3.20 mmol) and 6.0 mL of ethylene glycol monomethyl ether were added into a reaction flask, sealed and reacted at 120°C for 5-6 hours until TLC test showed that the reaction was completed. Then, the reaction solution was cooled to room temperature and added with 15 mL of saturated $NaHCO_3$ aqueous solution and 10 mL of $H_2O$ to precipitate a large amount of solids which were continuously stirred for about 0.5 hour and then subjected to suction filtration. The filter cake was washed with water, dried, and then separated and purified through column chromatography (DCM/MeOH=8/1), and then pulped with 5 mL of mixed solvent (PE/EA=4/1) to obtain compound 6 (276 mg, yield 43.0%). $^1$H NMR (400 MHz, $CDCl_3$) δ=10.30 (s, 1H), 8.33 (dd, *J*=8.4, 4.4 Hz, 1H), 8.18 (s, 1H), 8.03 (s, 1H), 7.43 - 7.30 (m, 2H), 7.11 (d, *J=14.0* Hz, 1H), 6.62 (s, 1H), 3.86 (s, 3H), 3.16 (d, *J*=11.8 Hz, 2H), 2.65 (m, 2H), 2.40 (m, 10H), 2.14 (s, 3H), 2.03 - 1.94 (m, 2H), 1.85 (s, 3H), 1.82 (s, 3H), 1.73 (m, 2H). MS-ESI (m/z): 601.2015 $(M+H)^+$.

**Example 7**

**Compound 7:**

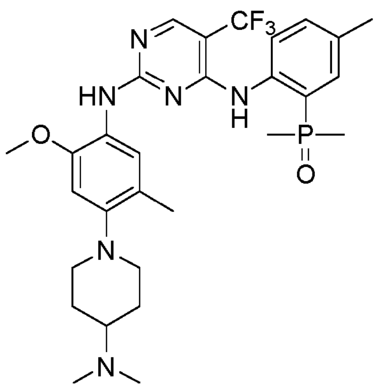

Referring to the synthesis method of compound 5, compound 1B therein was simply replaced with compound 3B

to obtain compound 7. MS-ESI (m/z): 591.2856 $(M+H)^+$. $^1H$ NMR (400 MHz, $CDCl_3$) δ=10.55 (s, 1H), 8.36 (s, 2H), 8.12 (s, 1H), 7.52 (s, 1H), 7.25 (d, *J*=8.4 Hz, 1H), 7.10 (d, *J=14.0* Hz, 1H), 6.65 (s, 1H), 3.82 (s, 3H), 3.13 (d, *J*=12.0 Hz, 2H), 2.65 (m, 2H), 2.38 (m,10H), 2.17 (s, 3H), 1.95 (m, 2H), 1.86 (s, 3H), 1.83 (s, 3H),1.71 (m, 2H).

**Example 8**

**Compound 8:**

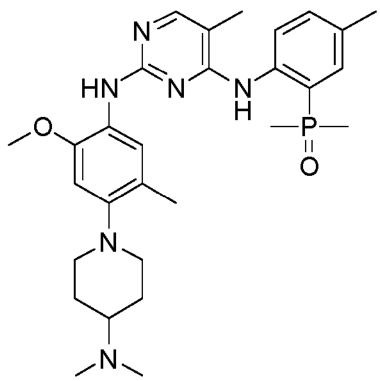

Referring to the synthesis method of compound 5, compound 1B therein was simply replaced with compound 4B to obtain compound 8. MS-ESI (m/z): 537.3169 $(M+H)^+$. $^1H$ NMR (400 MHz, $CDCl_3$) δ=10.25 (s, 1H), 8.45 (dd, *J*=8.4, 4.8 Hz, 1H), 8.12 (s, 1H), 7.89 (s, 1H), 7.35 (m, 2H), 7.04 (d, *J=14.0* Hz, 1H), 6.60 (s, 1H), 3.83 (s, 3H), 3.15 (d, *J*=12.0 Hz, 2H), 2.68 (m, 2H), 2.40 (m, 10H), 2.18 (m, 6H), 1.93 (m, 2H), 1.85 (s, 3H), 1.82 (s, 3H),1.74 (m, 2H).

**Example 9**

**Compound 9A:**

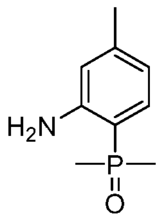

2-iodo-5-methylaniline (2.0 g, 8.58 mmol), $K_3PO_4$ (2.91 g, 13.73 mmol), Xantphos (0.546 g,0.944 mmol), $Pd(OAc)_2$ (0.212 g, 0.944 mmol), dimethylphosphine oxide (1.34 g, 17.16 mmol), 36 mL of DMF and 4.8 mL of water were added into a 100mL single-necked flask, subjected to nitrogen replacement for 3-4 times, reacted at 120°C for about 4-5 hours until TLC test showed that the reaction was completed. Then, the reaction solution was cooled to room temperature and filtered. The filtrate was added with 72 mL of $H_2O$ to precipitate a large amount of solids which were filtered. The filtrate was extracted with 50 mL of dichloromethane thrice, the organic phases were combined, washed with 50 mL of saturated aqueous solution of sodium chloride twice, dried with anhydrous sodium sulfate and filtered, and then the filtrate was concentrated to obtain compound 9A (1.0 g, yield 64.0%). $^1H$ NMR (400 MHz, $CDCl_3$) δ = 6.96 (dd, *J*= 13.6, 8.0 Hz, 1H), 6.52 (d, *J* = 8.0Hz, 1H), 6.47 (d, *J* = 4.0 Hz, 1H), 5.29 (s, 2H), 2.26 (s, 3H), 1.76 (s, 3H), 1.73 (s, 3H).

**Compound 9B:**

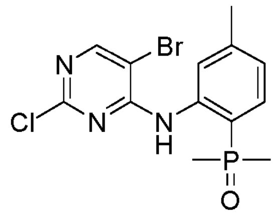

Compound 9A (500 mg, 2.73 mmol), 5-bromo-2,4-dichloropyrimidine (808 mg, 3.55 mmol), $K_2CO_3$ (1.5 g, 10.92 mmol), $nBu_4NHSO_4$ (92.69 mg, 0.273 mmol) and 10mL of DMF were added into a 50mL three-necked flask, heated to 65°C and reacted for about 4.5 hours until TLC test showed that the reaction was completed. Then, the reaction solution was cooled to room temperature, added with 50 mL of $H_2O$, and extracted with 20 mL of ethyl acetate. The aqueous phases were extracted with 10 mL of ethyl acetate twice, and the organic phases were combined. The organic phases were washed with 20 mL of water twice and washed with 20 mL of saturated sodium chloride solution twice in turn, dried with anhydrous sodium sulfate, and filtered. The filtrate was concentrated under reduced pressure to obtain compound 9B (466 mg, yield 45.7%).

**Compound 9:**

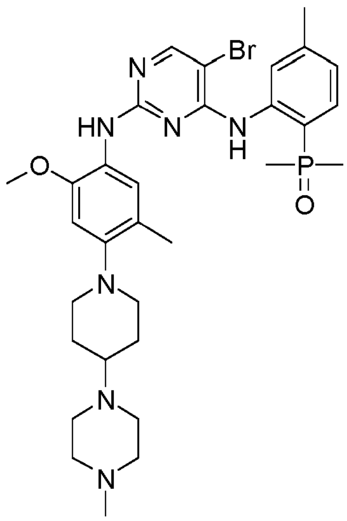

Compound 9B (500 mg, 1.33 mmol), compound 1F (383 mg, 1.2 mmol), 15% hydrogen chloride ethanol solution (974 mg, 4.0 mmol) and 7.5 mL of ethylene glycol monomethyl ether were added into a reaction flask, sealed and reacted at 100°C for 5 hours until TLC test showed that the reaction was completed. Then, the reaction solution was cooled to room temperature, and added with 15mL of saturated $NaHCO_3$ aqueous solution and 10 mL of $H_2O$ to precipitate a large amount of solids which were continuously stirred for about 0.5 hour and then subjected to suction filtration. The filter cake was washed with water, and then pulped with 5 mL of mixed solvent (PE/EA=2/3) and purified to obtain compound 9 (340 mg, yield 43.15%). $^1H$ NMR (400 MHz, $CDCl_3$) $\delta$=10.38 (s, 1H), 8.25 (d, *J=4.0* Hz, 1H), 8.20 (s, 1H), 7.99 (s, 1H), 7.32 (s, 1H), 7.21 (dd, *J=14.0,* 8.0 Hz, 1H), 6.95 (d, *J*=7.8 Hz, 1H), 6.62 (s, 1H), 3.86 (s, 3H), 3.13 (d, *J*=12.0 Hz, 2H), 2.81 - 2.42 (m, 9H), 2.34 (s, 3H), 2.30 (s, 3H), 2.07 (s, 3H), 1.95 (d, *J*=12.0 Hz, 2H), 1.86 (m, 2H), 1.84 (s, 3H), 1.81 (s, 3H), 1.77-1.66 (m, 2H). MS- ESI (m/z): 656.2456 $(M+H)^+$.

**Example 10**

## Compound 10A:

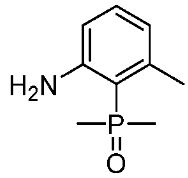

2-iodo-3-methylaniline (1.0 g, 4.29 mmol), $K_3PO_4$ (1.09 g, 5.15 mmol), Xantphos (0.25 g, 0.429 mmol), $Pd(OAc)_2$ (0.096 g, 0.429 mmol), dimethylphosphine oxide (0.5 g, 6.44 mmol) and 10 mL of DMF were added into a 250mL three-necked flask, heated to120°C and reacted until TLC test showed that the reaction was completed. Then, the reaction solution was cooled to room temperature, and subjected to suction filtration. The filtrate was added with 30 mL of $H_2O$, extracted with 30 mL of dichloromethane thrice, and the organic phases were combined. The organic phases were washed with 50 mL of saturated aqueous solution of sodium chloride once, dried with anhydrous sodium sulfate, and filtered. The filtrate was concentrated to obtain compound 10A (440.0 mg, yield 56.0%). MS-ESI (m/z): 184.0921 $(M+H)^+$.

## Compound 10B:

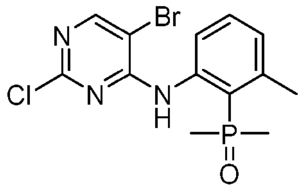

Compound 10A (3.93 g, 21.45 mmol), 5-bromo-2,4-dichloropyrimidine (7.33 g, 32.2 mmol), $K_2CO_3$ (8.89 g, 64.35 mmol), $nBu_4NHSO_4$ (0.73 g, 2.15 mmol) and 20 mL of DMF were added into a 250mL three-necked flask, heated to 65°C and reacted until TLC test showed that the reaction was completed. The reaction solution was cooled to room temperature and added with 50 mL of $H_2O$ to precipitate yellow solids which were continuously stirred for about 0.5 hour and then subjected to suction filtration. The filtrate cake was washed 100 mL of $H_2O$, and then dried to obtain compound 10B (3.88 g, wherein the total yield of the two steps was 48.5%). MS-ESI (m/z): 373.9770 $(M+H)^+$.

## Compound 10:

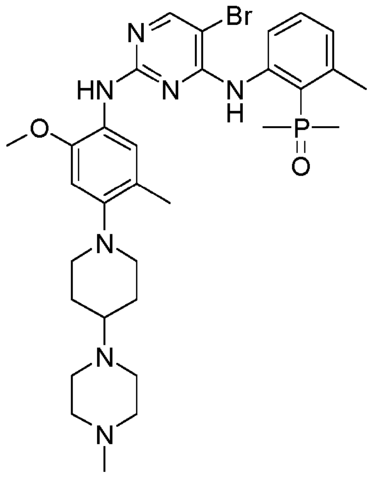

Compound 1F (500 mg, 1.57 mmol), compound 10B (588.12 mg, 1.57 mmol), 15% hydrogen chloride ethanol solution (917 mg, 3.77 mmol) and 7.5 mL of ethylene glycol monomethyl ether were added into a reaction flask, and sealed and reacted at 120°C until TLC test showed that the reaction was completed. Then, the reaction solution was cooled to room temperature, added with 50 mL of 3% $K_2CO_3$ aqueous solution and 30 mL of dichloromethane, and stirred for 10 minutes. The organic phases were combined, dried with anhydrous sodium sulfate, and filtered. The filtrate was concentrated, then separated and purified through column chromatography to obtain compound 10 (551 mg, yield 53.5%). MS-ESI (m/z): 656.2486$(M+H)^+$.

**Example 11**

## Compound 11A:

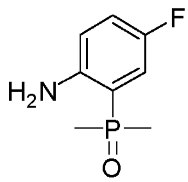

2-iodo-4-fluoroaniline (5.0 g, 21.1 mmol), $K_3PO_4$ (5.37 g, 25.32 mmol), Xantphos (1.22 g, 2.11 mmol), $P(OAc)_2$ (0.474 g, 2.11 mmol), dimethylphosphine oxide (2.47 g, 31.65 mmol) and 50 mL of DMF were added into a 100mL three-necked flask, heated to 120°C and reacted for about 4 hours until TLC test showed that the reaction was completed. Then, the reaction solution was cooled to room temperature and subjected to suction filtration. The filtrate was added with 100 mL of $H_2O$ to precipitate a large amount of yellow solids which were then subjected to suction filtration. The filtrate was extracted with 50 mL of dichloromethane for four times, and the organic phases were combined. The organic phases were washed with 100 mL of saturated aqueous solution of sodium chloride twice, dried with anhydrous sodium sulfate, and filtered. The filtrate was concentrated to obtain oily compound 11A, then added with 4 mL of ethyl acetate and 8 mL of petroleum ether to precipitate solid 11A (1.2 g, yield 30%). $^1H$ NMR (400 MHz, $CDCl_3$) $\delta$ = 6.98 (m, 1H), 6.78 (m, 1H), 6.61 (m, 1H), 5.23 (s, 2H), 1.79 (s, 3H), 1.75 (s, 3H). MS-ESI (m/z): 188.0639$(M+H)^+$.

## Compound 11B:

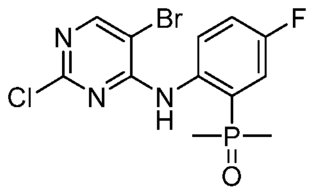

Compound 11A (500 mg, 2.67 mmol), 5-bromo-2,4-dichloropyrimidine (790.94 mg, 3.47 mmol), $K_2CO_3$ (1.11 g, 8.01 mmol), $nBu_4NHSO_4$ (0.09 g, 0.267 mmol) and 10 mL of DMF were added into a 50 mL three-necked flask, heated to 65°C and reacted until TLC test showed that the reaction was completed. Then, the reaction solution was cooled to room temperature, added with 50 mL of $H_2O$, and extracted with 20 mL of ethyl acetate. The aqueous phases were extracted with 10 mL of ethyl acetate twice, and the organic phases were combined. The organic phases were washed with water twice and washed with saturated sodium chloride solution twice in turn, dried with anhydrous sodium sulfate, and filtered. The filtrate was concentrated under reduced pressure to obtain compound 11B (700 mg, yield 69.3%).

**Compound 11:**

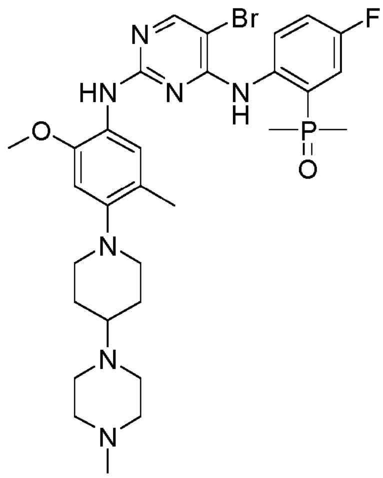

Compound 11B (300 mg, 0.79 mmol), compound 1F (303 mg, 0.95 mmol), 15% hydrogen chloride ethanol solution (578 mg, 2.38 mmol), and 4.5 mL of ethylene glycol monomethyl ether were added into a reaction flask, sealed and reacted at 120°C for 5-6 hours until TLC test showed that the reaction was completed. Then, the reaction solution was cooled to room temperature and added with 4.5 mL of saturated $NaHCO_3$ aqueous solution and 13.5 mL of $H_2O$ to precipitate a large amount of solids which were continuously stirred for about 0.5 hour and then subjected to suction filtration. The filter cake was washed with water, dried and then separated and purified through column chromatography (DCM/MeOH=15/1 to 10/1), and then pulped with 6 mL of mixed solvent (PE/EA=5/1) to obtain compound 11 (90 mg, yield 17.2%). $^1$H NMR (400 MHz, $CDCl_3$) δ=10.25 (s, 1H), 8.46 (m, 1H), 8.20 (s, 1H), 7.95 (s, 1H), 7.34 (s, 1H), 7.21 (m, 1H), 7.10-6.92 (m, 1H), 6.62 (s, 1H), 3.86 (s, 3H), 3.16 (d, *J*=11.2 Hz, 2H), 2.84 - 2.47 (m, 9H), 2.37 (m, 5H), 2.16 (s, 3H), 1.97 (d, *J=11.4* Hz, 2H), 1.87 (s, 3H), 1.84 (s, 3H), 1.79 - 1.67 (m, 2H). MS-ESI (m/z): 660.2227$(M+H)^+$.

**Example 12**

**Compound 12B:**

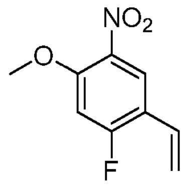

1-bromo-2-fluoro-4-methoxy-5-nitrobenzene (2.0 g, 8.0 mmol), Pd(dppf)$Cl_2$ (0.59 g, 0.8 mmol), triphenylphosphine (0.63 g, 2.4 mmol), cuprous bromide (0.34 g, 2.4 mmol), triethyl(vinyl)tin (3.8 g, 12.0 mmol) and 80 mL of toluene were added into a 250 mL three-necked flask, heated to 110°C under the protection of nitrogen and reacted for about 4 hours until TLC test showed that the reaction was completed. Then, the reaction solution was cooled to room temperature, and added with 80 mL of saturated potassium fluoride aqueous solution to quench the reaction, and then extracted with 80 mL of ethyl acetate thrice. The organic phases were combined, washed with 80 mL of saturated aqueous solution of sodium chloride, and then dried with anhydrous sodium sulfate, filtered, concentrated, separated and then purified by silica gel column chromatography (petroleum ether/ethyl acetate=10/1) to obtain compound 12B (1.41 g, yield 89.2%). $^1$H NMR (400 MHz, DMSO-$d_6$) δ=8.24 (d, *J*=7.9 Hz, 1H), 7.33 (d, *J*=12.6 Hz, 1H), 6.75 (dd, *J*=17.7, 11.3 Hz, 1H), 5.95 (d, *J*=17.7 Hz, 1H), 5.44 (d, *J*=11.3 Hz, 1H), 3.94 (s, 3H).

## Compound 12C:

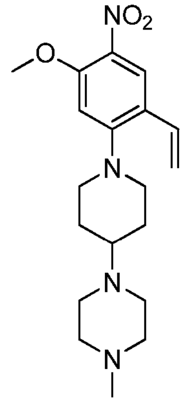

12B (1.20 g, 6.09 mmol), 1-methyl-4-(4-piperidinyl)piperazine (1.34 g, 7.30 mmol), potassium carbonate (1.69 g, 12.17 mmol) and 6 mL of DMF were added into a 50mL round-bottom flask, heated to 120°C and reacted for 4 hours. After TLC test showed that the reaction was completed, the reaction solution was cooled to room temperature, and added with 20 mL of water to precipitate a large amount of yellow solids which were continuously stirred for 1 hour and then subjected to suction filtration. The filter cake was washed with water, and dried to obtain compound 12C (1.87 g, yield 85.3%).

## Compound 12A:

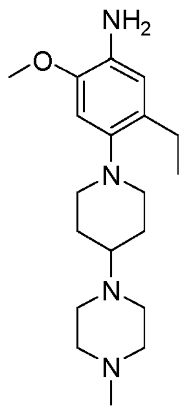

Compound 12C (1.8 g, 5.0 mmol), 0.36 g of 5% Pd/C and 36 mL of methanol were weighed and added into a 150mL single-necked flask, replaced with hydrogen for 2-3 times, and stirred overnight at room temperature in a hydrogen atmosphere (normal pressure) until TLC test showed that the reaction was completed. Then, after diatomite filtration, the filtrate was concentrated in vacuum, separated and purified through column chromatography (DCM/MeOH=20/1) to obtain compound 12A (780 mg, yield 47.0%). $^1H$ NMR (400 MHz, $CDCl_3$) $\delta$=6.62 (s, 1H), 6.58 (s, 1H), 3.82 (s, 3H), 3.04 - 2.99 (m, 2H), 2.78 - 2.42 (m, 12H), 2.31 (s, 4H), 1.97 - 1.85 (m, 2H), 1.69 - 1.64 (m 2H), 1.16 (t, *J*=7.5 Hz, 3H).

**Compound 12:**

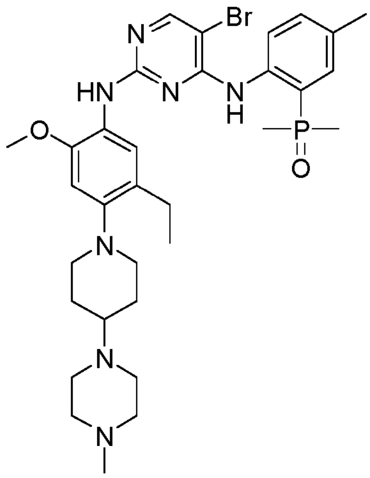

Compound 2B (565 mg, 1.51 mmol), compound 12A (705 mg, 2.12 mmol), 15% hydrogen chloride ethanol solution (960 mg, 3.64 mmol), and 7.5 mL of ethylene glycol monomethyl ether were added into a reaction flask, sealed and reacted at 120°C for 5-6 hours. After TLC test showed that the reaction was completed, the reaction solution was cooled to room temperature, and then 15 mL of saturated $NaHCO_3$ aqueous solution was added to precipitate a large amount of solids which were continuously stirred for 0.5 hour and then subjected to suction filtration. The filter cake was washed with water, and then pulped and purified to obtain compound 12 (673 mg, yield 66.5%). MS-ESI (m/z): 670.2642(M+H)$^+$. $^1$H NMR (400 MHz, $CDCl_3$) δ=10.23 (s, 1H), 8.25 (dd, *J*=8.4, 4.8 Hz, 1H), 8.19 (s, 1H), 8.06 (s, 1H), 7.44 - 7.29 (m, 2H), 7.11 (d, *J=14.0* Hz, 1H), 6.66 (s, 1H), 3.85 (s, 3H), 3.08 (d, *J*=11.6 Hz, 2H), 2.93 - 2.21 (m, 19H), 1.95 (d, *J*=12.0 Hz, 2H), 1.82 (d, *J*=13.2 Hz, 6H), 1.78 - 1.66 (m, 2H), 1.00 (t, *J*=7.6 Hz, 3H).

**Example 13**

**Compound 13A:**

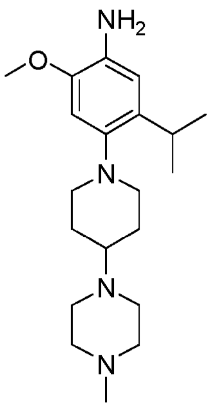

Referring to the synthesis of compound 12A, compound triethyl(vinyl)tin therein was simply replaced with compound 2-(tributylstannyl)propene to obtain compound 13A.

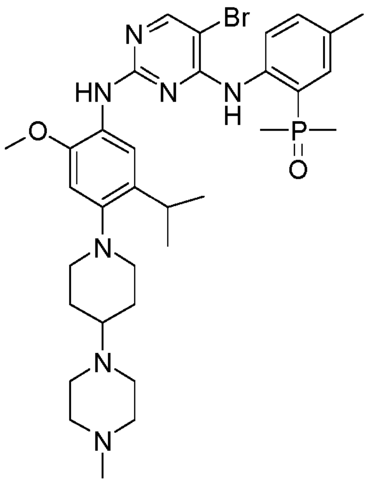

Compound 2B (565 mg, 1.51 mmol), compound 13A (735 mg, 2.12 mmol), 15% hydrogen chloride ethanol solution (960 mg, 3.64 mmol), and 7.5 mL of ethylene glycol monomethyl ether were added into a reaction flask, sealed and reacted at 120°C for 5-6 hours. After TLC test showed that the reaction was completed, the reaction solution was cooled to room temperature, and then 15 mL of saturated $NaHCO_3$ aqueous solution was added to precipitate a large amount of solids which were continuously stirred for 0.5 hour and then subjected to suction filtration. The filter cake was washed with water, and then pulped and purified to obtain compound 13 (622 mg, yield 60.2%). MS-ESI (m/z): 684.2756$(M+H)^+$.

**Example 14**

**Compound 14A:**

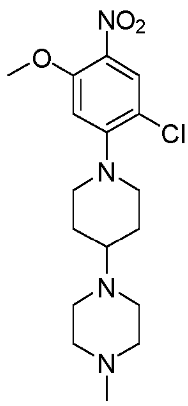

1-Chloro-2-fluoro-4-methoxy-5-nitrobenzene (500 mg, 2.43 mmol), 1-methyl-4-(4-piperidinyl)piperazine (490 mg, 2.68 mmol), potassium carbonate (507 mg, 3.65 mmol) and 10 mL of DMF were added into a 100mL round-bottom flask, heated to 80°C and reacted for 2.5 hours. After TLC test showed that the reaction was completed, the reaction solution was cooled to room temperature and added with 30 mL of water to precipitate a large amount of yellow solids which were continuously stirred for 1 hour and then subjected to suction filtration. The filter cake was washed with water, and dried to obtain compound 14A (740 mg, yield 82.5%).

**Compound 14B:**

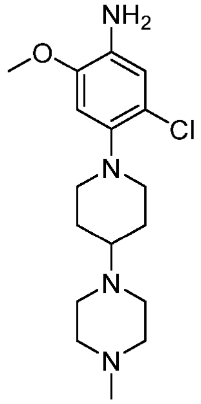

Compound 14A (740 mg, 2.01 mmol), reduced iron powder (672 mg, 12.04 mmol), ammonium chloride (75 mg, 1.40 mmol) and 67 mL of mixed solution (ethanol/water=3/1) were added into a 250 mL round-bottom flask, and heated to reflux for about 6.5 hours until TLC test showed that the reaction was completed. The reaction solution was cooled to room temperature, and a pH of the reaction solution was adjusted to about 8 with 2 mol/L aqueous solution of sodium hydroxide. After the reaction solution was concentrated to dryness, 110 mL of mixed solution (dichloromethane/methanol=10/1) was added and stirred at room temperature for about 0.5 hour, and then subjected to suction filtration. The filtrate was concentrated and separated by column chromatography (dichloromethane/methanol=8/1) to obtain compound 14B (647 mg, yield 95.2%). MS-ESI (m/z): 339.1928 $(M+H)^+$.

**Compound 14:**

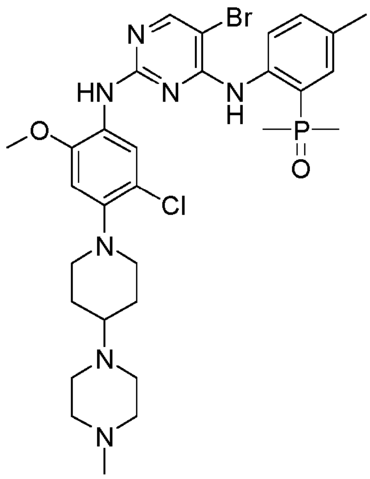

Compound 2B (253 mg, 0.67 mmol), compound 14B (243 mg, 1.01 mmol), 15% hydrogen chloride ethanol solution (493 mg, 2.02 mmol) and 4 mL of ethylene glycol monomethyl ether were added into a reaction flask, sealed and reacted at 120°C for 5-6 hours until TLC test showed that the reaction was completed. Then, the reaction solution was cooled to room temperature and added with 4 mL of saturated $NaHCO_3$ aqueous solution and 12 mL of $H_2O$ to precipitate a large amount of solids which were continuously stirred for about 0.5 hour and then subjected to suction filtration. The filter cake was washed with water, dried, and then separated and purified through column chromatography (DCM/MeOH=15/1 to 10/1), and then pulped with 6 mL of mixed solvent (PE/EA=5/1) to obtain compound 14 (120 mg, yield 26.3%). $^1H$ NMR (400 MHz, $CDCl_3$) δ=10.38 (s, 1H), 8.39 - 8.24 (m, 2H), 8.19 (s, 1H), 7.47 (d, *J*=8.8 Hz, 1H), 7.41 (s, 1H), 7.10 (d, *J=14.4* Hz, 1H), 6.61 (s, 1H), 3.88 (s, 3H), 3.40 (d, *J*=11.2 Hz, 2H), 2.83 - 2.46 (m, 9H), 2.38 (s, 3H), 2.33 (s, 3H), 2.16 (s, 2H), 1.95 (d, *J=11.4* Hz, 2H), 1.81 (m, 8H). MS-ESI (m/z): 676.1902 $(M+H)^+$.

**Example 15**

**Compound 15A:**

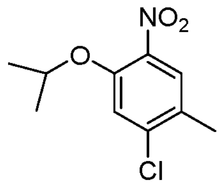

2-chloro-4-fluoro-5-nitrotoluene (10.0 g, 52.4 mmol), cesium carbonate (83.2 g, 262 mmol), and 100 mL of isopropanol were added to a 250mL single-necked flask, and reacted at 60°C for 4 hours until TLC test showed that the raw materials disappeared. Then, the reaction solution was cooled to room temperature, added with 100 mL of ethyl acetate, stirred for 10 minutes, and then filtered. The filter cake was washed with 50 mL of ethyl acetate, and concentrated to dryness under reduced pressure. The residues were added with 100 mL of water and 150 mL of ethyl acetate, and the system was allowed to settle for layering. The aqueous phases were extracted once with 100 mL of ethyl acetate, and the ethyl acetate phases were combined. Then, the ethyl acetate phases were washed with 100 mL of water twice and washed with 100 mL of saturated aqueous solution of sodium chloride twice in turn, dried with anhydrous sodium sulfate and filtered. The filtrate was concentrated to obtain compound 15A (11.45 g, yield 95.4%).

**Compound 15B:**

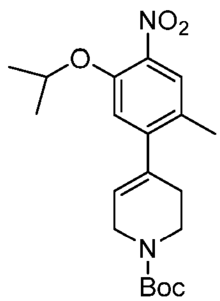

Compound 15A (9.0 g, 39.2 mmol), N-Boc-1,2,5,6-tetrahydropyridine-4-boronic acid pinacol ester (13.33 g, 43.12 mmol), $K_2CO_3$ (16.25 g, 117.6 mmol), 82 mL of DME and 8 mL of water were added into a 250mL single-necked flask. After the mixture was subjected to nitrogen replacement thrice, $Pd(dppf)Cl_2$ (2.86 g, 3.92 mmol) was added, followed by nitrogen replacement thrice again, and then reacted at 120°C for 5 hours until TLC test showed that the raw materials disappeared. The reaction solution was cooled to room temperature and filtered. The filter cake was washed with 50 mL of ethyl acetate, the filtrate was concentrated to dryness under reduced pressure, and the residues were purified through column chromatography (PE:EA=20:1-10:1) to obtain pale yellow oily compound 15B (8.8 g, yield 60.0%).1H NMR (400 MHz, $CDCl_3$) δ = 7.62 (s, 1H), 6.79 (s, 1H), 5.62 (s, 1H), 4.63 (m, 1H), 4.06 (d, J = 2.0 Hz, 2H), 3.64 (t, J = 5.6 Hz, 2H), 2.33 (s, 2H), 2.24 (s, 3H), 1.51 (s, 9H), 1.37 (d, J = 6.0 Hz, 6H). MS-ESI (m/z): 399.1882 $(M+Na)^+$.

**Compound 15C:**

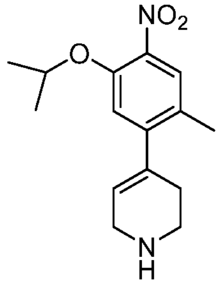

Compound 15B (8.8 g, 23.4 mmol) and 20 mL of ethyl acetate were added into a 150mL single-necked flask, stirred and dissolved, then added with 25 mL of 15% hydrogen chloride ethyl acetate solution, and stirred overnight at room temperature. TLC test showed that the raw materials disappeared. The reaction solution was added with 100 mL of water, stirred for 10 minutes, and the system was allowed to settle for layering. Then, the ethyl acetate layer was washed with 40

mL of water. The aqueous phases were combined, and then a pH was adjusted to about 8 with saturated sodium bicarbonate solution. 100 mL of ethyl acetate was added for extraction, and then the aqueous phases were extracted with 50 mL of ethyl acetate twice. The ethyl acetate phases were combined, and then the ethyl acetate phases were washed with saturated sodium chloride solution twice, dried with anhydrous sodium sulfate, and filtered. The filtrate was concentrated to obtain compound 15C (4.2 g, yield 65.01%). $^1H$ NMR (400 MHz, $CDCl_3$) $\delta$=7.63 (s, 1H), 6.84 (s, 1H), 5.68 (s, 1H), 4.64 (m, 1H), 3.89 (d, *J*=2.4 Hz, 2H), 3.48 (t, *J*=6.0 Hz, 2H), 2.71 (m, 2H), 2.29 (s, 3H), 1.38 (d, *J*=6.0 Hz, 6H). MS-ESI (m/z): 277.1547 $(M+H)^+$.

**Compound 15D:**

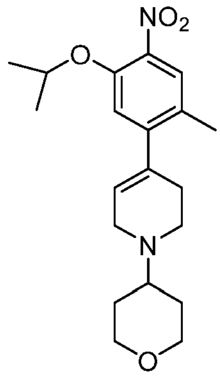

Compound 15C (1.60 g, 5.79 mmol) and 64 mL of dichloromethane were added and dissolved in a 150mL single-necked flask, then added with tetrahydro-4H-pyran-4-one (1.65 g, 17.37 mmol), acetic acid (1.32 g, 23.16 mmol) and sodium triacetoxyborohydride (4.66 g, 23.16 mmol) were added into a 150mL single-necked flask and stirred overnight at room temperature. TLC test showed that the raw materials disappeared. Then, the reaction solution was added with 120 mL of water, stirred for 10 minutes, and the system was allowed to settle for layering. Then, the aqueous phases were extracted with 20 mL of dichloromethane. The two dichloromethane phases were combined, and then the dichloromethane phases were washed with 40 mL of water and 40 mL of saturated sodium chloride solution in turn, dried with anhydrous sodium sulfate, and filtered. The filtrate was concentrated to obtain oily compound 15D (2.00 g, yield 95.80%).

**Compound 15E:**

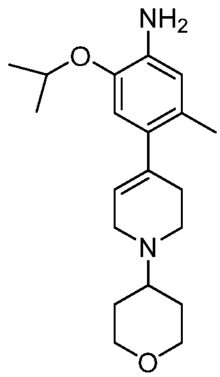

Compound 15D (0.99 g, 2.77 mmol) and 20 mL of ethanol were added and dissolved in a 50mL single-necked flask, and then added with 2.5 mL (2N) of hydrogen chloride and iron powder (0.994 g, 17.76 mmol), and reacted at 60°C for 3 hours. TLC test showed that the raw materials disappeared. After the reaction was completed, the reaction solution was cooled to room temperature and filtered. The filtrate was concentrated under reduced pressure and then purified through column chromatography to obtain oily substance 15E (779 mg, yield 85.0%). MS-ESI (m/z): 331.2381 $(M+H)^+$.

**Compound 15:**

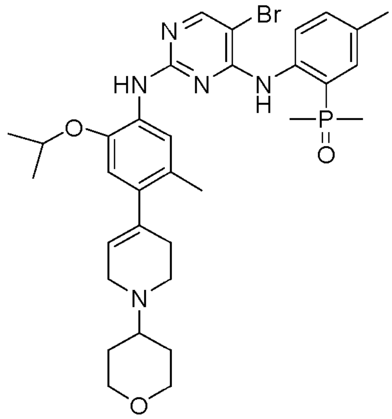

Compound 2B (315 mg, 0.84 mmol), compound 15E (417mg, 1.26 mmol), 15% hydrogen chloride ethanol solution (613 mg, 2.52 mmol) and 5.0 mL of ethylene glycol monomethyl ether were added into a reaction flask, sealed and reacted at 120°C for 5-6 hours. After TLC test showed that the reaction was completed, the reaction solution was cooled to room temperature, and then 50 mL of 3% $K_2CO_3$ aqueous solution and 50 mL of dichloromethane were added, stirred for 10 minutes, and then the system was allowed to settle for liquid separation. The upper aqueous phase was continuously washed once with 50 mL of dichloromethane, and the two dichloromethane phases were combined. Then, the dichloromethane phase was washed with 50 mL of saturated salt solution, dried with anhydrous sodium sulfate and filtered, the filtrate was concentrated and purified through column chromatography to obtain compound 15 (310 mg, yield 55.20%). MS-ESI (m/z): 668.2334 $(M+H)^+$.

**Example 16**

**Compound 16D:**

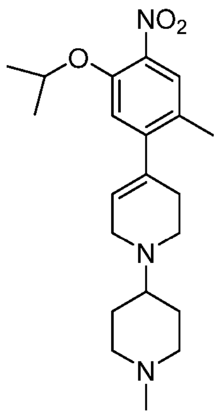

Referring to the synthesis method of compound 15D, compound 15C (4.20 g,15.20 mmol) and 160 mL of dichloromethane were added to a 250mL single-necked flask and dissolved, then added with N-methyl-4-piperidone (5.16 g, 45.60 mmol), acetic acid (3.66 g, 60.80 mmol) and sodium triacetoxyborohydride (12.88 g, 60.80 mmol), and stirred overnight at room temperature. TLC test showed that the raw materials disappeared. Then, the reaction solution was added with 60 mL of water, stirred for 10 minutes, and the system was allowed to settle for layering. Then, the aqueous phases were extracted with 60 mL of dichloromethane. The two dichloromethane phases were combined, and then the dichloromethane phases were washed with 40 mL of water and 40 mL of saturated sodium chloride solution in turn, dried with anhydrous sodium sulfate, and filtered. The filtrate was concentrated to obtain oily compound 16D (4.51 g, yield 79.4%). MS-ESI (m/z): 374.2443 $(M+H)^+$.

**Compound 16E:**

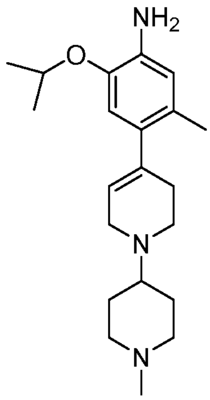

Referring to the synthesis method of compound 15E, compound 16D (1.0 g, 2.68 mmol) and 20 mL of ethanol were added and dissolved in a 50mL single-necked flask, and then added with 2.5 mL (2N) of hydrogen chloride solution and iron powder (0.960 g, 17.15 mmol), and reacted at 60°C for 3 hours. TLC test showed that the raw materials disappeared. After the reaction was completed, the reaction solution was cooled to room temperature and filtered. The filtrate was concentrated under reduced pressure and then purified through column chromatography to obtain oily substance 16E (736 mg, yield 80.0%).

**Compound 16:**

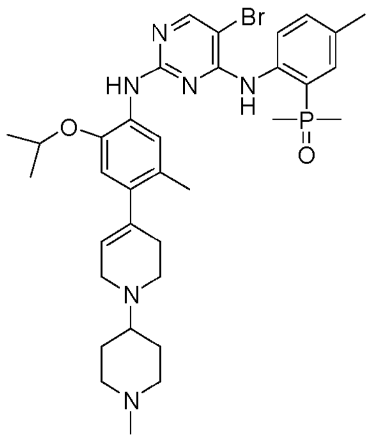

Compound 2B (400 mg, 1.07 mmol), compound 16E (333 mg, 0.97 mmol), 15% hydrogen chloride ethanol solution (708 mg, 2.91 mmol) and 6 mL of ethylene glycol monomethyl ether were added into a reaction flask, sealed and reacted at 120°C for 5-6 hours until TLC test showed that the reaction was completed. Then, the reaction solution was cooled to room temperature, added with 6 mL of saturated $NaHCO_3$ aqueous solution and 6 mL of $H_2O$, and extracted with 30 mL of dichloromethane thrice. The organic phases were combined, and then washed with 30 mL of saturated aqueous solution of sodium chloride, dried with anhydrous sodium sulfate, filtered, concentrated, and then separated and purified through column chromatography (DCM/MeOH=0/1) to obtain compound 16 (120 mg, yield 16.5%). MS-ESI (m/z): 681.2644 $(M+H)^+$.

**Example 17**

**Compound 17A:**

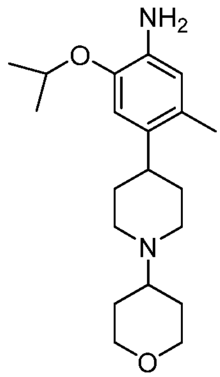

Compound 15D (1.8 g, 5.0 mmol), 0.27 g of 5% Pd/C and 30 mL of methanol were weighed and added into a 100 mL single-necked flask, replaced with hydrogen for 2-3 times, and stirred overnight at room temperature in a hydrogen atmosphere (normal pressure) until TLC test showed that the reaction was completed. Then, after diatomite filtration, the filtrate was concentrated in vacuum, separated and purified through column chromatography to obtain compound 17A (1.51 g, yield 90.8%). MS -ESI (m/z): 333.2572$(M+H)^+$.

**Compound 17:**

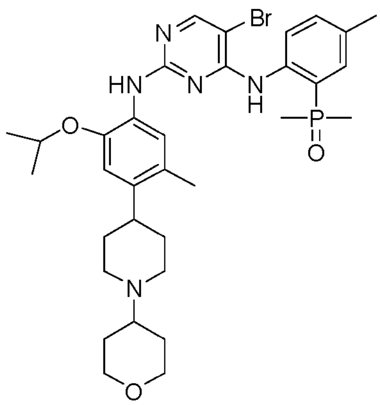

Compound 2B (566 mg, 1.51 mmol), compound 17A (706 mg, 2.12 mmol), 15% hydrogen chloride ethanol solution (960 mg, 3.64 mmol), and 4.5 mL of ethylene glycol monomethyl ether were added into a reaction flask, sealed and reacted at 120°C for 5-6 hours. After TLC test showed that the reaction was completed, the reaction solution was cooled to room temperature, and then 15 mL of saturated $NaHCO_3$ aqueous solution was added to precipitate solids which were continuously stirred for 0.5 hour and then subjected to suction filtration. The filter cake was washed with water, and the filter cake was pulped with 20 mL of mixed solvent (EA/PET=1/5) and purified to obtain compound 17 (558 mg, yield 55.2%). MS-ESI (m/z): 670.2554 $(M+H)^+$.

**Example 18**

**Compound 18A:**

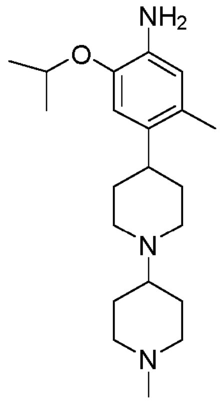

Compound 16D (1.60 g, 4.28 mmol), 1.0 g of 5% Pd/C and 10mL of methanol were weighed and added into a 100mL single-necked flask, replaced with hydrogen for 2-3 times, and stirred for 48 hours at room temperature in a hydrogen atmosphere until TLC test showed that the reaction was completed. Then, after diatomite filtration, the filtrate was concentrated in vacuum, separated and purified through column chromatography to obtain compound 18A (1.3 g, yield 87.8%).

**Compound 18:**

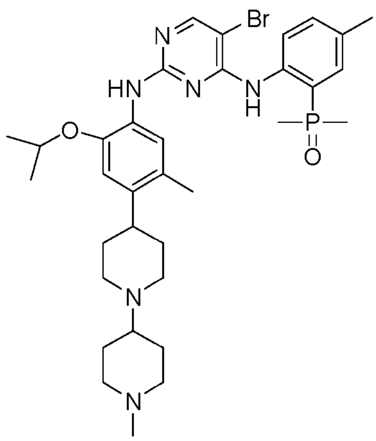

Compound 2B (570 mg, 1.52 mmol), compound 18A (500 mg, 1.45 mmol), 15% hydrogen chloride ethanol solution (1.06 g, 4.35 mmol) and 7.5 mL of ethylene glycol monomethyl ether were added into a reaction flask, and then sealed and reacted at 120°C for 4 hours until TLC test showed that the reaction was completed. The reaction solution was cooled to room temperature and then added with 15 mL of saturated $NaHCO_3$ aqueous solution to precipitate solids which were continuously stirred for about 0.5 hour and then subjected to suction filtration. The filter cake was dissolved with methanol and concentrated under reduced pressure to obtain oily substance which was added with 3 mL of ethyl acetate for dissolution, then dropwise added with 2 mL of petroleum ether, pulped and purified to obtain compound 18 (241 mg, yield 23.21%). $^1H$ NMR (400 MHz, $CDCl_3$) δ=10.30 (s, 1H), 8.35 (dd, *J*=8.4, 4.4 Hz, 1H), 8.19 (s, 1H), 8.05 (s, 1H), 7.51 (s, 1H), 7.33 (d, *J*=8.4 Hz, 1H), 7.12 (d, *J*=14.0 Hz, 1H), 6.82 (s, 1H), 4.52 (m, 1H), 3.06 (d, *J*=11.2 Hz, 2H), 2.96 (d, *J*=11.2 Hz, 2H), 2.71 - 2.60 (m, 1H), 2.38 (s, 3H), 2.34 (m, 2H), 2.29 (s, 3H), 2.16 (s, 3H), 2.01-1.91 (m, 4H), 1.84 (m, 8H), 1.72 (m, 5H), 1.37 (s, 3H),1.35 (s, 3H). MS-ESI (m/z):683.2800$(M+H)^+$.

**Example 19**

Compound 19B:

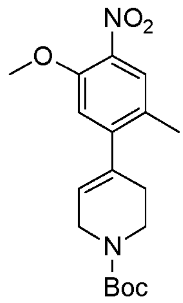

Compound 5-bromo-4-methyl-2-nitroanisole (9.65g, 39.2 mmol), N-Boc-1,2,5,6-tetrahydropyridine-4-boronic acid pinacol ester (13.33 g, 43.12 mmol), $K_2CO_3$ (16.25 g, 117.6 mmol), 82 mL of DME and 8 mL of water were added into a 250mL single-necked flask. After the mixture was subjected to nitrogen replacement thrice, Pd(dppf)$Cl_2$ (2.86 g, 3.92 mmol) was added, followed by nitrogen replacement thrice again, and then reacted at 120°C for 5 hours until TLC test showed that the raw materials disappeared. The reaction solution was cooled to room temperature and filtered. The filter cake was washed with 50 mL of ethyl acetate, the filtrate was concentrated to dryness under reduced pressure, and the residues were purified through column chromatography to obtain pale yellow oily compound 19B (7.65 g, yield 56.0%). MS-ESI (m/z): 349.1801 $(M+H)^+$.

Compound 19C:

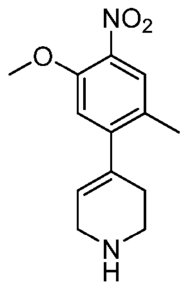

Referring to the synthesis of compound 15C, compound 15B therein was simply replaced with compound 19B to obtain compound 19C.

Compound 19D:

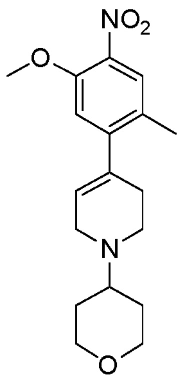

Referring to the synthesis of compound 15D, compound 15C therein was simply replaced with compound 19C to obtain compound 19D. MS-ESI (m/z): 333.1875 $(M+H)^+$.

**Compound 19E:**

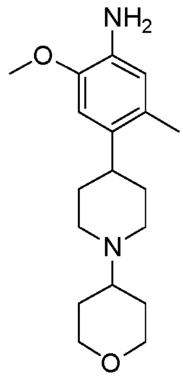

Referring to the synthesis of compound 17A, compound 15D therein was simply replaced with compound 19D to obtain compound 19E. MS-ESI (m/z): 305.2285 $(M+H)^+$.

**Compound 19:**

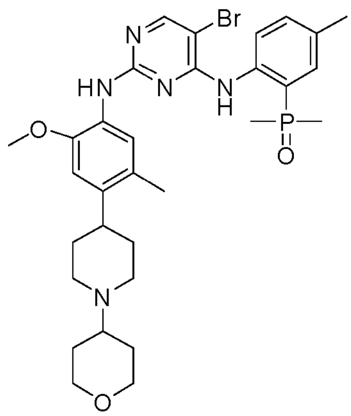

Referring to the synthesis of compound 17, compound 17A therein was simply replaced with compound 19E to obtain compound 19. MS-ESI (m/z): 642.2173 $(M+H)^+$.

**Example 20**

**Compound 20D:**

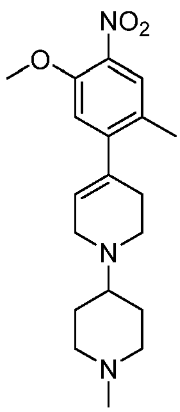

Referring to the synthesis of compound 16D, compound 15C therein was simply replaced with compound 19C to obtain compound 20D.

**Compound 20E:**

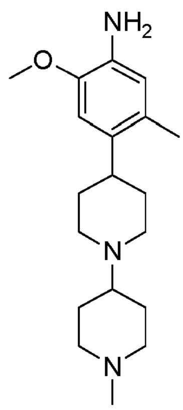

Referring to the synthesis of compound 17A, compound 15D therein was simply replaced with compound 20D to obtain compound 20E. MS-ESI (m/z): 318.2512 $(M+H)^+$.

**Compound 20:**

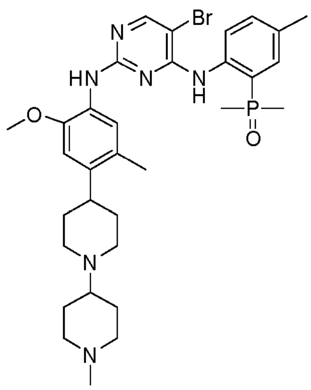

Referring to the synthesis of compound 17, compound 17A therein was simply replaced with compound 20E to obtain compound 20. MS-ESI (m/z): 655.2553 $(M+H)^+$.

**Example 21**

**Compound 21A:**

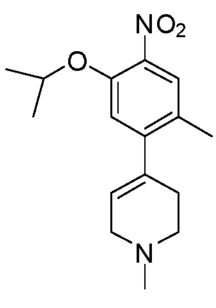

Compound 15C (4.20 g, 15.20 mmol) and 160 mL of dichloromethane were added to a 250mL single-necked

flask and dissolved, then added with paraformaldehyde (1.37 g, 45.60 mmol), acetic acid (3.66 g, 60.80 mmol) and sodium triacetoxyborohydride (12.88 g, 60.80 mmol), and stirred overnight at room temperature. TLC test showed that the raw materials disappeared. Then, the reaction solution was added with 60 mL of water, stirred for 10 minutes, and the system was allowed to settle for layering. Then, the aqueous phases were extracted with 60 mL of dichloromethane. The two dichloromethane phases were combined, and then the dichloromethane phases were washed with 40 mL of water and 40 mL of saturated sodium chloride solution in turn, dried with anhydrous sodium sulfate, and filtered. The filtrate was concentrated to obtain oily compound 21A (3.58 g, yield 81.2%).

**Compound 21B:**

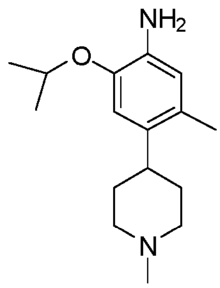

Referring to the synthesis of compound 17A, compound 15D therein was simply replaced with compound 21A to obtain compound 21B. MS-ESI (m/z): 263.2182 $(M+H)^+$.

**Compound 21:**

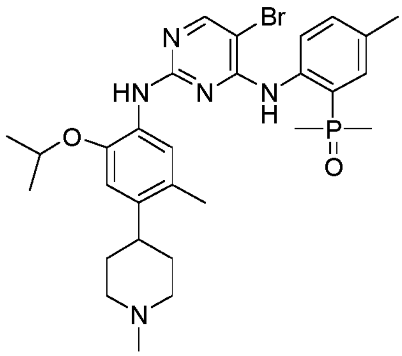

Referring to the synthesis of compound 17, compound 17A therein was simply replaced with compound 21B to obtain compound 21. MS-ESI (m/z): 600.2165 $(M+H)^+$.

**Example 22**

**Compound 22A:**

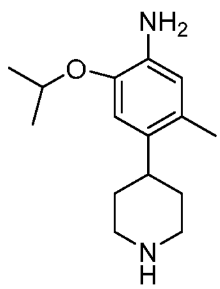

Compound 15C (1.38 g, 5.00 mmol), 0.21 g of 5% Pd/C and 30 mL of methanol were weighed into a 100mL single-necked flask, replaced with hydrogen for 2-3 times, and stirred overnight at room temperature in a hydrogen atmosphere

until TLC test showed that the reaction was completed. Then, after diatomite filtration, the filtrate was concentrated in vacuum, separated and purified through column chromatography to obtain compound 22A (1.07 g, yield 85.8%). MS -ESI (m/z): 249.1987(M+H)$^+$.

**Compound 22:**

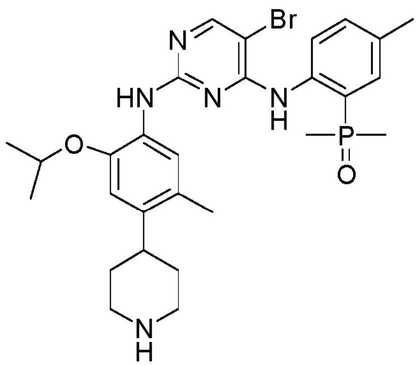

Referring to the synthesis of compound 17, compound 17A therein was simply replaced with compound 22A to obtain compound 22. MS-ESI (m/z):586.1975 (M+H)$^+$.

**Example 23**

**Compound 23A:**

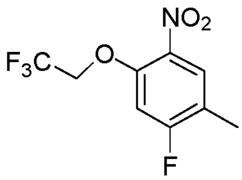

Compound 5-fluoro-4-methyl-2-nitrophenol (2.57 g, 15.00 mmol), 60% sodium hydride (2.40 g, 60.00 mmol) and 30 mL of DMF were added into a 100mL three-necked flask, stirred at 0°C for 30 minutes, and then added with 2-iodo-1,1,1-trifluoroethane (4.73 g, 22.50 mmol) in batches, Then, the mixture was restored to room temperature and stirred overnight until TLC test showed that the reaction was completed. Then, the reaction solution was added with 60 mL of water and 100 mL of ethyl acetate, stirred for 10 minutes, and the system was allowed to settle for liquid separation. The aqueous phases were continuously washed with 50 mL of ethyl acetate once. The two ethyl acetate phases were combined, and then the organic phases were washed with saturated salt solution once, dried with anhydrous sodium sulfate and filtered. The filtrate was concentrated to obtain compound 23A (2.68 g, yield 70.5%) after column chromatography.

**Compound 23B:**

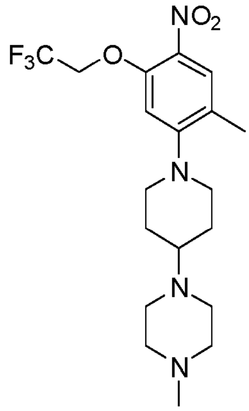

Compound 23A (2.53 g, 10.00 mmol), $K_2CO_3$ (4.14g, 30.00 mmol), 1-methyl-4-(4-piperidinyl)piperazine (2.75 g, 15.00 mmol), and 30 mL of DMF were added into a 250mL three-necked flask, heated to 120°C and stirred for about 4 hours until TLC test showed that the reaction was completed. Then, the reaction solution was cooled to room temperature, added with 60 mL of water and 100 mL of ethyl acetate, stirred for 10 minutes, and the system was allowed to settle for liquid separation. The aqueous phases were continuously washed with 50 mL of ethyl acetate once. The two ethyl acetate phases were combined, and then the organic phases were washed with saturated salt solution once, dried with anhydrous sodium sulfate and filtered. The filtrate was concentrated to obtain compound 23B (2.47 g, yield 59.2%) after column chromatography. MS -ESI (m/z): 417.2175 $(M+H)^+$.

**Compound 23C:**

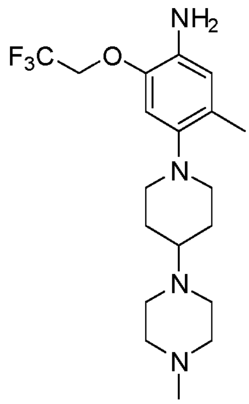

Compound 23B (2.47 g, 5.92 mmol), 0.25 g of 5% Pd/C and 30 mL of methanol were added into a 100mL single-necked flask, replaced with hydrogen for 2-3 times, and stirred overnight at room temperature in a hydrogen atmosphere until TLC test showed that the reaction was completed. Then, after diatomite filtration, the filtrate was concentrated in vacuum, separated and purified through column chromatography to obtain compound 23C (1.93 g, yield 84.5%).

Compound 23:

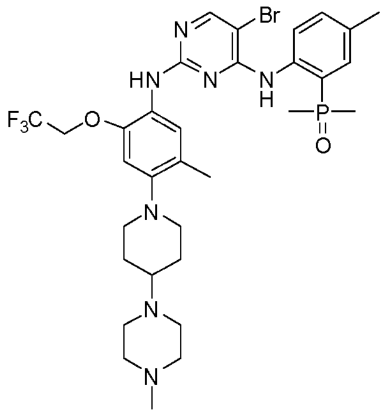

Referring to the synthesis of compound 17, compound 17A therein was simply replaced with compound 23C to obtain compound 23. MS-ESI (m/z): 724.2315 $(M+H)^+$. $^1H$ NMR (400 MHz, $CDCl_3$) δ=10.56 (s, 1H), 8.42 (dd, *J*=8.8, 4.8 Hz, 1H), 8.23 (s, 1H), 8.10 (s, 1H), 7.42 (m, 2H), 7.16 (d, *J*=14.4Hz, 1H), 6.82 (s, 1H), 4.85 (m, 2H), 3.10 (d, *J*=11.6 Hz, 2H), 2.80 - 2.48 (m, 11H), 2.36 (s, 3H), 2.32 (s, 3H), 2.15 (s, 3H), 2.05 (d, *J*=11.0 Hz, 2H), 1.88 (s, 3H), 1.84 (s, 3H), 1.78 - 1.64 (m, 2H).

**Example 24**

Compound 24D:

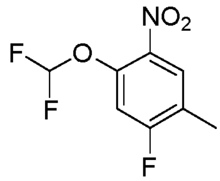

Compound 1C (2.00 g, 11.69 mmol), cesium carbonate (5.74 g, 17.52 mmol), 14 mL of DMF and 0.34 mL of $H_2O$ were added to a 50mL single-necked flask, stirred evenly at room temperature, then sodium difluorochloroacetate (3.56 g, 23.38 mmol) was added into batches, and after the addition was complete, the mixture was heated to 100°C and stirred for about 2 hours until TLC test showed that the reaction was completed. Then, the reaction solution was cooled to room temperature, added with 56 mL of $H_2O$, and extracted with 28 mL of petroleum ether twice. The organic phases were combined, then washed with 20 mL of saturated salt solution, dried with anhydrous sodium sulfate and filtered. The filtrate was concentrated to obtain compound 24D (1.49 g, yield 57.64%).

Compound 24E:

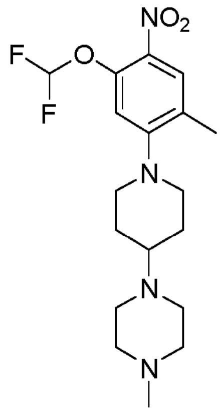

Compound 24D (1.21 g, 5.45 mmol) was added into a 50mL single-necked flask, and added with 22.35 mL of DMF and stirred for dissolution, then added with $K_2CO_3$ (1.51 g, 10.91 mmol), 1-methyl-4(4-piperidinyl)-piperazine (1.30 g, 7.09 mmol), heated to 120°C and reacted for about 2 hours until TLC test showed that the reaction was completed. Then, the reaction solution was cooled to room temperature, added with 40 mL of water, and extracted with 120 mL of EA twice. The organic phases were combined, and then the organic phases were washed with 30 mL of $H_2O$ thrice and washed with 30 mL of saturated sodium chloride aqueous solution, dried with anhydrous sodium sulfate and filtered. The filtrate was concentrated to obtain compound 24E (1.91 g, yield 91.3%).

**Compound 24F:**

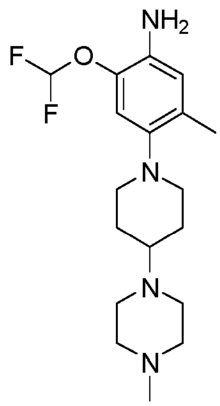

Compound 24E (1.91g, 4.97 mmol), 0.24 g of 5% Pd/C and 48 mL of methanol were added into a 100mL single-necked flask, replaced with hydrogen for 2-3 times, and stirred overnight at room temperature in a hydrogen atmosphere (normal pressure) until TLC test showed that the reaction was completed. Then, after diatomite filtration, the filtrate was concentrated in vacuum, separated and purified through column chromatography to obtain compound 24F (1.68 g, yield 95.5%). MS-ESI (m/z): 355.2302(M+H)$^+$.

**Compound 24:**

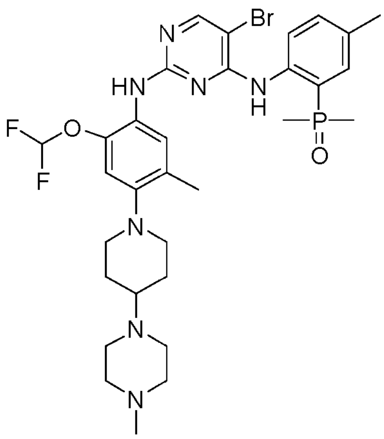

Referring to the synthesis of compound 2, compound 1F therein was simply replaced with compound 24F to obtain compound 24. MS-ESI (m/z): 692.2234 (M+H)$^+$. $^1$H NMR (400 MHz, $CDCl_3$) δ=10.50 (s, 1H), 8.42 (m, 1H), 8.18 (s, 1H), 8.06 (s, 1H), 7.50 - 7.36 (m, 2H), 7.12 (d, *J*=14.0Hz, 1H), 6.80 (s, 1H), 6.66 (s, 0.25H), 6.52 (s, 0.5H), 6.40 (s, 0.25H), 3.16 (d, *J*=11.6 Hz, 2H), 2.82 - 2.42 (m, 11H), 2.36 (s, 3H), 2.34 (s, 3H), 2.16 (s, 3H), 1.96 (d, *J*=11.0 Hz, 2H), 2.12 (s, 3H), 1.96 (s, 3H), 1.78 - 1.66 (m, 2H).

**Comparative Example 25**

**Compound 25A:**

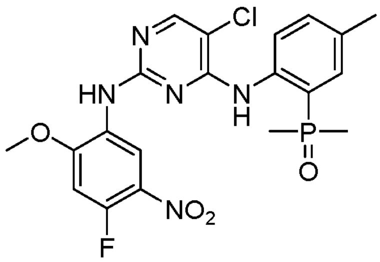

Compound 1B (2.13 g, 6.46 mmol), 4-fluoro-2-methoxy-5-nitroaniline (1.44 g, 7.75 mmol), 15% hydrogen chloride ethanol solution (4.71 g, 19.37 mmol) and 30 mL of ethylene glycol monomethyl ether were added into a reaction flask, heated to 120°C, and then sealed and reacted for about 5 hours until TLC test showed that the reaction was completed. The reaction solution was cooled to room temperature and added with 90 mL of about 5% aqueous solution of sodium bicarbonate to precipitate a large amount of claybank solids which were continuously stirred for about 0.5 hour and then subjected to suction filtration, and washed with water. The filter cake was pulped and purified to obtain compound 25A (2.09 g, yield 67.4%).

**Compound 25B:**

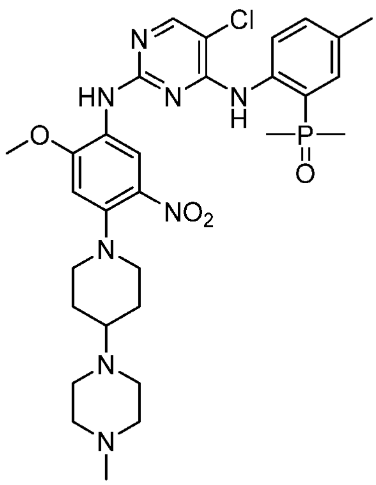

Compound 25A (1.57 g, 3.27 mmol), $K_2CO_3$ (0.91 g, 6.53 mmol), 1-methyl-4-(4-piperidinyl)piperazine (0.72 g, 3.92 mmol) and 30 mL of DMF were added into a 150mL three-necked flask, heated to 120°C and stirred for about 3 hours until TLC test showed that the reaction was completed. The reaction solution was cooled to room temperature and added with 60 mL of $H_2O$ to precipitate a large amount of solids which were continuously stirred for about 0.5 hour and then subjected to suction filtration, and washed with water. The filter cake was dried to obtain compound 25B (1.69 g, yield 80.5%). MS-ESI (m/z): 643.2758 $(M+H)^+$.

Compound 25C:

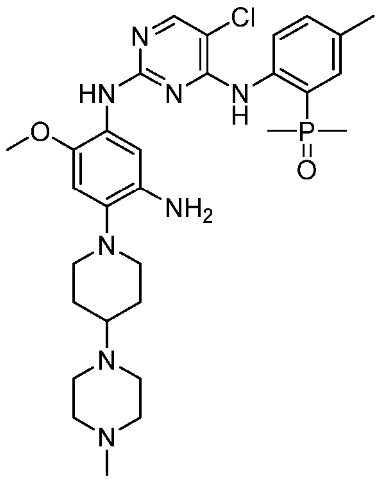

Compound 25B (1.03 g, 1.61 mmol), reduced iron powder (0.54 g, 9.64 mmol) and ammonium chloride (0.06 g, 1.12 mmol) were added into a 100mL single-necked flask, then added with 90 mL of mixed solvent (EtOH/$H_2O$=3/1) and stirred evenly, and then refluxed for about 2 hours until TLC test showed that the reaction was completed. The reaction solution was cooled to room temperature. After the reaction solution was concentrated, 330 mL of mixed solvent (DCM/MeOH=10/1) was added, stirred for about 15 minutes, and then filtered. The filtrate was concentrated to obtain compound 25C (0.88 g, yield 89.7%). MS-ESI (m/z): 613.2968 $(M+H)^+$.

Compound 25:

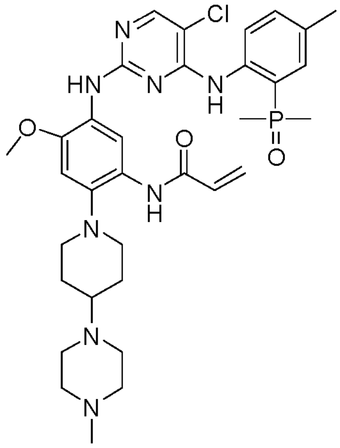

Compound 25C (514 mg, 0.84 mmol) and DIPEA (120 mg, 0.93 mmol) were added into a 50mL single-necked flask and dissolved by 7.5 mL of dichloromethane with stirring and cooled to 0-10°C; acryloyl chloride (92 mg, 1.01 mmol) was weighed and added, diluted with 2.5 mL of DCM, and added dropwise to the reaction solution slowly, after the dropwise addition was completed, the reaction was continued at 0-10°C for about 3 hours until TLC test showed that the reaction was completed. Then, 20 mL of dichloromethane and 20 mL of $H_2O$ were added to the reaction solution for liquid separation, the dichloromethane phases were washed with 20 mL of saturated aqueous solution of sodium chloride, dried with anhydrous sodium sulfate and filtered. The filtrate was concentrated, then separated and purified through column chromatography to obtain compound 25 (189 mg, yield 33.9%). MS-ESI (m/z): 667.3085 $(M+H)^+$.

**Comparative Example 26**

**Compound 26B:**

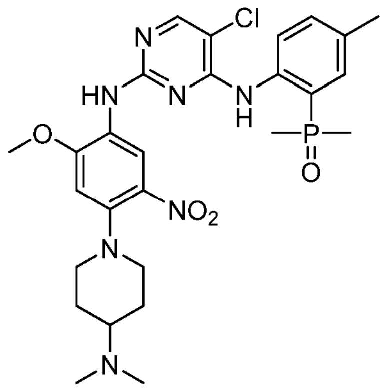

Referring to the synthesis method of compound 25B, 1-methyl-4-(4-piperidinyl)piperazine therein was simply replaced with 4-dimethylaminopiperidine to obtain compound 26B. MS-ESI (m/z): 588.2283 $(M+H)^+$.

**Compound 26C:**

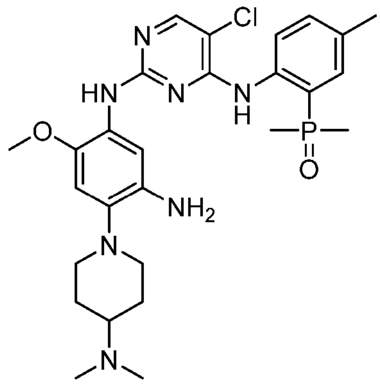

Referring to the synthesis method of compound 25C, compound 26C was obtained. MS-ESI (m/z): 558.2412 $(M+H)^+$.

**Compound 26:**

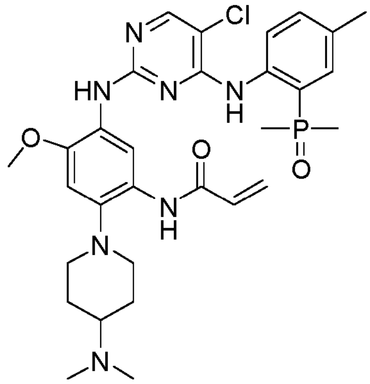

Referring to the synthesis of compound 25, the compound 25C therein was simply replaced with the compound 26C to obtain compound 26. MS-ESI (m/z): 612.2684 $(M+H)^+$.

Comparative Example 27

**Compound 27B:**

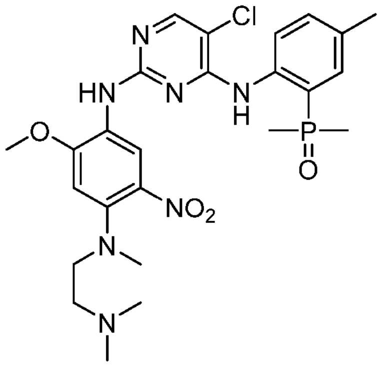

Referring to the synthesis of compound 25B, compound 1-methyl-4-(4-piperidinyl)piperazine therein was simply replaced with compound N,N,N'-trimethylethylenediamine to obtain compound 27B. MS-ESI (m/z): 562.2127 $(M+H)^+$.

**Compound 27C:**

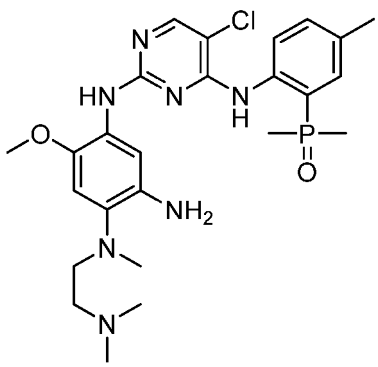

Referring to the synthesis method of compound 25C, compound 27C was obtained. MS-ESI (m/z): 532.2322 $(M+H)^+$

**Compound 27:**

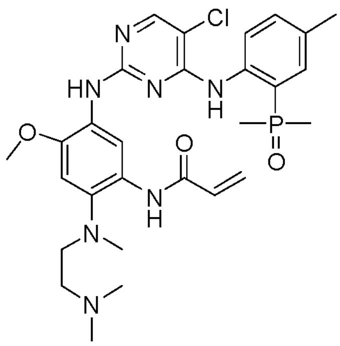

Referring to the synthesis of compound 25, compound 25C therein was simply replaced with compound 27C to obtain compound 27. MS-ESI (m/z): 586.2465 $(M+H)^+$.

Example 28

**Compound 28A:**

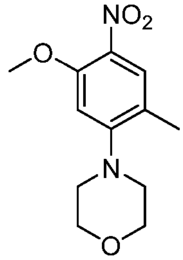

Compound 1D (4.5 g, 24.3 mmol), $K_2CO_3$ (6.71 g, 48.6 mmol), morpholine (3.17 g, 36.4 mmol) and 67.5 mL of DMF were weighed and added into a 250mL three-necked flask, heated to 120°C and reacted for about 4 hours with stirring until TLC test showed that the reaction was completed. Then, the reaction solution was cooled to room temperature and added with130 mL of $H_2O$ to precipitate a large amount of solids which were continuously stirred for about 0.5 hour and then subjected to suction filtration. The filter cake was filtered, washed with water, and dried to obtain compound 28A (3.26 g, yield 53.1%). MS -ESI (m/z): 253.1148 $(M+H)^+$.

**Compound 28B:**

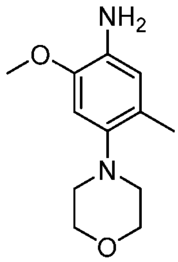

Compound 28A (3.26 g, 12.94 mmol), 0.49 g of 5% Pd/C and 60 mL of methanol were weighed into a 250mL single-necked flask, replaced with hydrogen for 2-3 times, and stirred overnight at room temperature in a hydrogen atmosphere (normal pressure) until TLC test showed that the reaction was completed. Then, after diatomite filtration, the filtrate was concentrated in vacuum and separated and purified through column chromatography to obtain compound 28B (2.67 g, yield 92.8%). MS -ESI (m/z): 223.1475 $(M+H)^+$.

**Compound 28:**

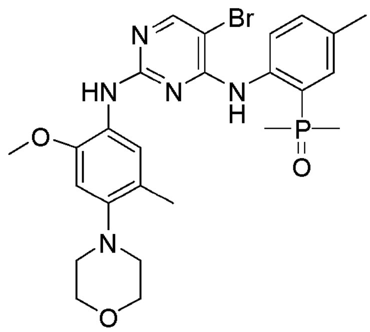

Compound 2B (566 mg, 1.51 mmol), compound 28B (471 mg, 2.12 mmol), 15% hydrogen chloride ethanol solution (960 mg, 3.64 mmol), and 4.5 mL of ethylene glycol monomethyl ether were added into a reaction flask, sealed and reacted at 120°C for 5-6 hours. After TLC test showed that the reaction was completed, the reaction solution was cooled to room temperature, and then 15 mL of saturated $NaHCO_3$ aqueous solution was added to precipitate a large amount of solids which were continuously stirred for 0.5 hour and then subjected to suction filtration. The filter cake was washed with water, and the filter cake was pulped with 20 mL of mixed solvent (EA/PET=1/5) and purified to obtain compound 28 (387 mg, yield 45.7%). MS-ESI (m/z): 560.1402 $(M+H)^+$.

**Example 29**

**Compound 29A:**

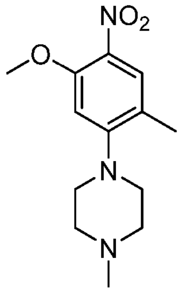

Compound 1D (4.5 g, 24.3 mmol), $K_2CO_3$ (6.71 g, 48.6 mmol), N-methylpiperazine (3.64 g, 36.4 mmol) and 67.5 mL of DMF were weighed into a 250mL three-necked flask, heated to 120°C and reacted for about 4 hours with stirring until TLC test showed that the reaction was completed. Then, the reaction solution was cooled to room temperature and added with130 mL of $H_2O$ to precipitate a large amount of solids which were continuously stirred for about 0.5 hour and then subjected to suction filtration. The filter cake was filtered, washed with water, and dried to obtain compound 29A (3.38 g, yield 52.5%). MS -ESI (m/z): 266.1547 $(M+H)^+$.

**Compound 29B:**

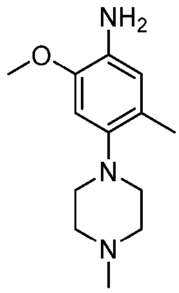

Compound 29A (3.38 g, 12.71 mmol), 0.51 g of 5% Pd/C and 60 mL of methanol were weighed and added into a 250mL single-necked flask, replaced with hydrogen for 2-3 times, and stirred overnight at room temperature in a hydrogen atmosphere (normal pressure) until TLC test showed that the reaction was completed. Then, after diatomite filtration, the filtrate was concentrated in vacuum and separated and purified through column chromatography to obtain compound 29B (2.80 g, yield 93.4%). MS -ESI (m/z): 236.1791 $(M+H)^+$.

**Compound 29:**

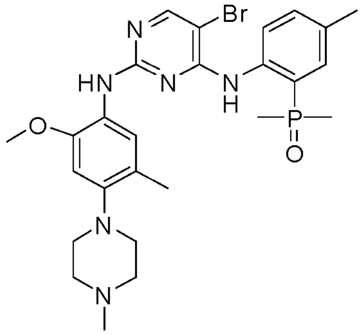

Compound 2B (566 mg, 1.51 mmol), compound 29B (500 mg, 2.12 mmol), 15% hydrogen chloride ethanol solution (960 mg, 3.64 mmol), and 4.5 mL of ethylene glycol monomethyl ether were added into a reaction flask, sealed and

reacted at 120°C for 5-6 hours. After TLC test showed that the reaction was completed, the reaction solution was cooled to room temperature, and then 15 mL of saturated $NaHCO_3$ aqueous solution was added to precipitate a large amount of solids which were continuously stirred for 0.5 hour and then subjected to suction filtration. The filter cake was washed with water, and the filter cake was pulped with 20 mL of mixed solvent (EA/PET=1/5) and purified to obtain compound 29 (350 mg, yield 40.5%). MS-ESI (m/z): 573.1728 $(M+H)^+$. $^1H$ NMR (400 MHz, $CDCl_3$) δ=10.31 (s, 1H), 8.34 (dd, *J*=8.6, 4.6 Hz, 1H), 8.19 (s, 1H), 8.05 (s, 1H), 7.39 - 7.32 (m, 2H), 7.15 - 7.08 (m, 1H), 6.67 (s, 1H), 3.87 (s, 3H), 2.95 (t, *J*=4.8 Hz, 4H), 2.63 (s, 4H), 2.40 (d, *J*=5.0 Hz, 6H), 2.17 (s, 3H), 1.84 (d, *J*=13.1 Hz, 6H). MS-ESI (m/z): 573.1728 $(M+H)^+$.

**Example 30**

**Compound 30A:**

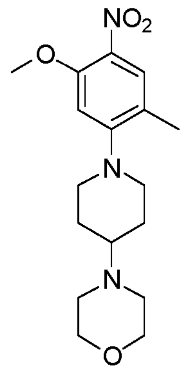

Compound 1D (4.5 g, 24.3 mmol), $K_2CO_3$ (6.71 g, 48.6 mmol), 4-(4-piperidinyl)morpholine (6.20 g, 36.4 mmol) and 67.5 mL of DMF were weighed and added into a 250mL three-necked flask, heated to 120°C and reacted for about 4 hours with stirring until TLC test showed that the reaction was completed. Then, the reaction solution was cooled to room temperature and added with130 mL of $H_2O$ to precipitate a large amount of solids which were continuously stirred for about 0.5 hour and then subjected to suction filtration. The filter cake was filtered, washed with water, and dried to obtain compound 30A (5.54 g, yield 67.8%). MS -ESI (m/z): 336.1944 $(M+H)^+$.

**Compound 30B:**

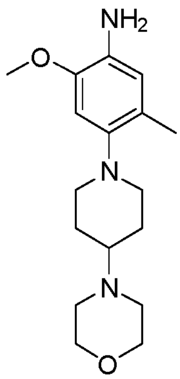

Compound 30A (5.54 g, 16.48 mmol), 0.83 g of 5% Pd/C and 60 mL of methanol were weighed and added into a 250mL single-necked flask, replaced with hydrogen for 2-3 times, and stirred overnight at room temperature in a hydrogen atmosphere (normal pressure) until TLC test showed that the reaction was completed. Then, after diatomite filtration, the filtrate was concentrated in vacuum and separated and purified through column chromatography to obtain compound 30B (4.67 g, yield 92.7%). MS -ESI (m/z): 306.2152 $(M+H)^+$.

**Compound 30:**

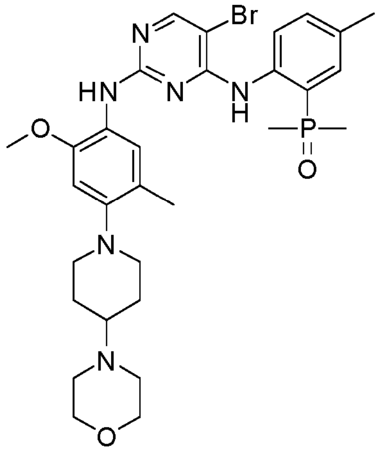

Compound 2B (566 mg, 1.51 mmol), compound 30B (648 mg, 2.12 mmol), 15% hydrogen chloride ethanol solution (960 mg, 3.64 mmol), and 4.5 mL of ethylene glycol monomethyl ether were added into a reaction flask, sealed and reacted at 120°C for 5-6 hours. After TLC test showed that the reaction was completed, the reaction solution was cooled to room temperature, and then 15 mL of saturated $NaHCO_3$ aqueous solution was added to precipitate a large amount of solids which were continuously stirred for 0.5 hour and then subjected to suction filtration. The filter cake was washed with water, and the filter cake was pulped with 20 mL of mixed solvent (EA/PET=1/5) and purified to obtain compound 30 (455 mg, yield 46.9%). MS-ESI (m/z): 643.2160 $(M+H)^+$. $^1H$ NMR (400 MHz, $CDCl_3$) δ=10.38 (s, 1H), 8.40 (dd, *J*=8.8, 4.8 Hz, 1H), 8.22 (s, 1H), 8.08 (s, 1H), 7.48 - 7.32 (m, 2H), 7.18 (d, *J*=14.0Hz, 1H), 6.66 (s, 1H), 3.95-3.78 (m, 7H), 3.20 (m, 2H), 2.88 - 2.49 (m, 7H), 2.38 (s, 3H), 2.35 (s, 3H), 2.05 (d, *J*=11.0 Hz, 2H), 1.90 (s, 3H), 1.86 (s, 3H), 1.88 - 1.76 (m, 2H).

**Example 31**

**Compound 31A:**

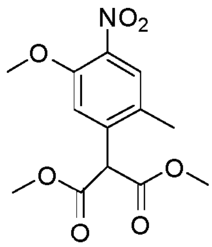

260 mL of DMSO was added into a 1.0L single-necked flask, added with NaH (9.09 g, 226 mmol, 60%) in batches under stirring, then stirred for 10 minutes, and then slowly added dropwise with dimethyl malonate (26 mL, 226 mmol), stirred at room temperature for 20 minutes after the dropwise addition was completed, and the reaction was kept at 100°C for 30 minutes to 60 minutes, then added with 5-fluoro-4-methyl-2-nitroanisole (14.0 g, 75.4 mmol) in batches, and the reaction was kept at 100°C for 60 minutes. The reaction was monitored by TLC. After the reaction was completed, the reaction solution was cooled to room temperature. Then, 550 mL of water was added dropwise, stirred at room temperature for 2 hours, and filtered. The filter cake was collected to obtain compound 31A (10.5 g, yield 46.8%) by forced air drying at 50°C. MS-ESI (m/z): 298.0956 $(M+H)^+$.

**Compound 31B:**

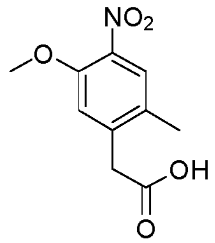

Compound 31A (10.5 g, 35.32 mmol) was dissolved in 300 mL of mixed solvent (EtOH/THF=1/1), stirred for 5 minutes, dropwise added with 150 mL of NaOH solution (2N) at room temperature, and after the dropwise addition was completed, the reaction was kept at room temperature for 1-2 hours until TLC test showed that the reaction materials disappeared. Then, when the reaction solution was concentrated under reduced pressure to a half volume remained, 6 mol/L hydrochloric acid solution was dropwise added until a pH of the reaction solution was 2 to 3. After stirring for 10 minutes, 180 mL of ethyl acetate was added for extraction, the aqueous phases were extracted with 100 mL of ethyl acetate again, the two ethyl acetate phases were combined, washed with 100 mL of water, then washed with 100 mL of saturated sodium chloride solution twice, dried with anhydrous sodium sulfate, concentrated under reduced pressure, and then pulped, purified and filtered. The filter cake was washed with a mixed solvent (petroleum ether/ethyl acetate=5:1), and dried to obtain compound 31B (6.4 g, yield 80.5%). MS-ESI (m/z): 226.0744 $(M+H)^+$.

**Compound 31C:**

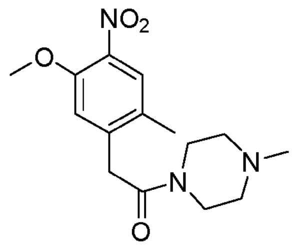

Compound 31B (5.3 g, 23.71 mmol) was suspended in 100 mL of dichloromethane and cooled in an ice bath to 0°C, added with HOBT (3.53 g, 26.1 mmol) and EDCI (5.0 g, 26.1 mmol), stirred at low temperature for 60 minutes, then added with N-methylpiperazine (2.61 g, 26.1 mmol), and further dropwise added with DIPEA (3.67 g, 28.4 mmol), the reaction was kept for 2 hours at low temperature after the dropwise addition was completed, and then reacted at room temperature for 1 hour until TLC test showed that the reaction materials disappeared. After the reaction was completed, 100 mL of water was added into the reaction solution, stirred for 10 minutes, and then the system was allowed to settle for layering. The aqueous phases were extracted with 50 mL of dichloromethane once; the two dichloromethane phases were combined and then the dichloromethane phases were washed with 100 mL of water, 100 mL of 3% $K_2CO_3$ aqueous solution and 150 mL of saturated sodium chloride solution in turn. The organic phases were dried with anhydrous sodium sulfate and filtered. The filtrate was concentrated under reduced pressure to oily substance, and then purified through column chromatography to obtain compound 31C (5.9 g, yield 80.57%). MS-ESI (m/z):308.1637 $(M+H)^+$.

**Compound 31D:**

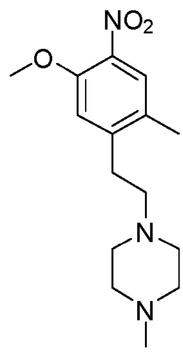

Compound 31C (4.7 g, 15.33 mmol) was dissolved in 45 mL of anhydrous THF under nitrogen protection. After cooled in an ice bath to 0°C, $BH_3$.THF solution (45 mL, 46.02 mmol) was added dropwise, the ice bath was removed after the dropwise addition was completed, then the reaction solution was stirred overnight at room temperature until the reaction materials disappeared by TLC test. After the reaction was completed, 120 mL of methanol was added dropwise to quench the mixture, and then concentrated under reduced pressure. 120 mL of methanol and 100 mL of 2N HCl solution were added into the obtained solids, refluxed at 70°C under stirring for 2 hours, then concentrated under reduced pressure to about 120 mL. 2N NaOH solution was added dropwise to adjust a pH of the mixed solution to be 8-9, then 100 mL of ethyl acetate was added for extraction, the aqueous phases were extracted once with 50 mL of ethyl acetate, the two ethyl acetate phases were combined, and then the ethyl acetate phases were washed with 80 mL of water and 80 mL of saturated sodium chloride solution in turn. The organic phases were dried with anhydrous sodium sulfate and filtered. The filtrate was concentrated under reduced pressure to oily substance, and then purified through column chromatography to obtain compound 31D (3.22 g, yield 71.50%). MS-ESI (m/z):294.1815 $(M+H)^+$.

**Compound 31E:**

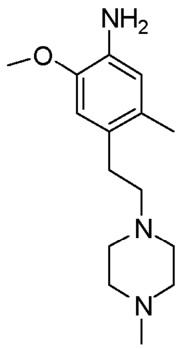

Compound 31D (3.15 g, 10.74 mol) was dissolved in 30 mL of methanol, added with 0.30 g of Pd/C (5%), replaced with hydrogen for 2-3 times, and stirred overnight at room temperature under a hydrogen atmosphere (normal pressure). TLC test showed that the reaction material disappeared. After the reaction was completed, the reaction solution was filtered with diatomite. The filtrate was concentrated and purified through column chromatography to obtain compound 31E (2.55 g, yield 90.1%). MS-ESI (m/z):264.2104 $(M+H)^+$.

**Compound 31:**

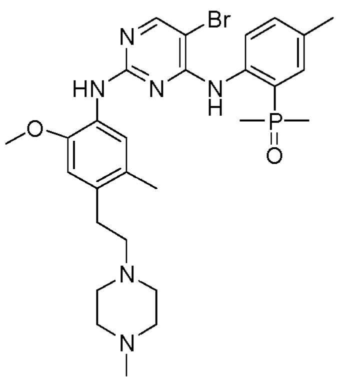

Referring to the synthesis of compound 2, compound 1F therein was simply replaced with compound 31E to obtain compound 31. MS-ESI (m/z): 601.2084 $(M+H)^+$.

**Example 32**

## Compound 32A:

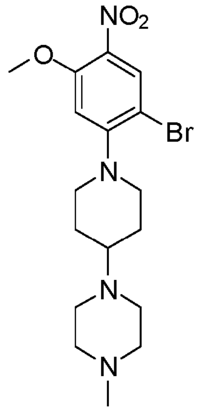

1-bromo-2-fluoro-4-methoxy-5-nitrobenzene (608 mg, 2.43 mmol), 1-methyl-4-(4-piperidinyl)piperazine (490 mg, 2.68 mmol), potassium carbonate (507 mg, 3.65 mmol) and 10 mL of DMF were added into a 100mL round-bottom flask, heated to 80°C and reacted for 2.5 hours. After TLC test showed that the reaction was completed, the reaction solution was cooled to room temperature and added with 30 mL of water to precipitate a large amount of yellow solids which were continuously stirred for 1 hour and then subjected to suction filtration. The filter cake was washed with water, and dried to obtain compound 32A (743 mg, yield 74.0%).

## Compound 32B:

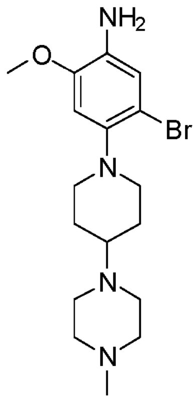

Compound 32A (740 mg, 1.79 mmol), reduced iron powder (600 mg, 10.74 mmol), ammonium chloride (75 mg, 1.40 mmol) and 67 mL of mixed solution (ethanol/water=3/1) were added into a 250mL round-bottom flask, and heated to reflux for about 6.5 hours until TLC test showed that the reaction was completed. The reaction solution was cooled to room temperature, and a pH of the reaction solution was adjusted to about 8 with 2 mol/L aqueous solution of sodium hydroxide. After the reaction solution was concentrated to dryness, 110 mL of mixed solution (dichloromethane/methanol=10/1) was added and stirred at room temperature for about 0.5 hour, and then subjected to suction filtration. The filtrate was concentrated and separated by column chromatography (dichloromethane/methanol=8/1) to obtain compound 32B (622 mg, yield 90.7%). MS-ESI (m/z): 383.1427 $(M+H)^+$.

**Compound 32:**

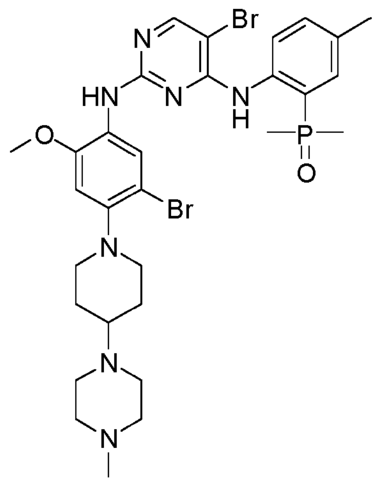

Compound 2B (253 mg, 0.67 mmol), compound 32B (387 mg, 1.01 mmol), 15% hydrogen chloride ethanol solution (439 mg, 2.02 mmol), and 4 mL of ethylene glycol monomethyl ether were added into a reaction flask, and then sealed and reacted at 100°C for 5-6 hours until TLC test showed that the reaction was completed. Then, the reaction solution was cooled to room temperature and added with 4 mL of saturated $NaHCO_3$ aqueous solution and 12 mL of $H_2O$ to precipitate a large amount of solids which were continuously stirred for about 0.5 hour and then subjected to suction filtration. The filter cake was washed with water, dried and then separated and purified by column chromatography (DCM/MeOH=15/1 to 10/1), and then pulped with 6 mL of mixed solvent (PE/EA=5/1) to obtain compound 32 (173 mg, yield 35.7%). $^1H$ NMR (400 MHz, $CDCl_3$) $\delta$=10.47 (s, 1H), 8.42 - 8.26 (m, 2H), 8.21 (s, 1H), 7.53(d, J=8.8 Hz, 1H), 7.47 (s, 1H), 7.15 (m, 1H), 6.68 (s, 1H), 3.95(s, 3H), 3.52 (d, *J*=11.2 Hz, 2H), 2.89 - 2.51 (m, 9H), 2.41 (s, 3H), 2.39 (s, 3H), 2.21 (s, 2H), 2.05 (d, *J*=11.4 Hz, 2H), 1.91 (m, 8H). MS-ESI (m/z): 722.1459 $(M+H)^+$.

**Example 33**

**Compound 33A:**

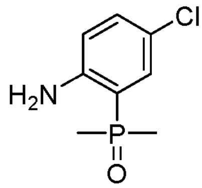

Referring to the synthesis of compound 11A, compound 2-iodo-4-fluoroaniline therein was simply replaced with compound 4-chloro-2-iodoaniline to obtain compound 33A. MS-ESI (m/z): 204.0367$(M+H)^+$.

**Compound 33B:**

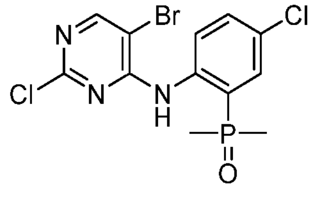

Referring to the synthesis of compound 11B, compound 11A therein was simply replaced with compound 33A to obtain compound 33B. MS-ESI (m/z): 393.9295$(M+H)^+$.

Compound 33:

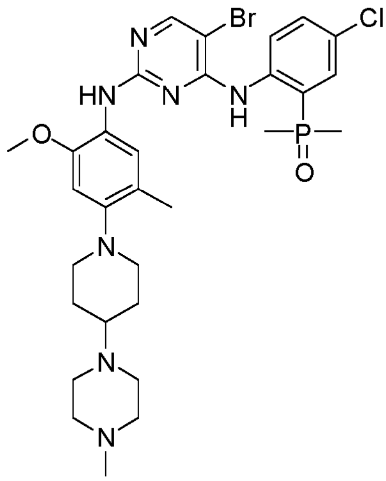

Referring to the synthesis of compound 11, compound 11B therein was simply replaced with compound 33B to obtain compound 33. $^1$H NMR (400 MHz, $CDCl_3$) δ = 10.38 (s, 1H), 8.57- 8.32(m, 2H), 8.05 (s, 1H), 7.47 (s, 1H), 7.32 (m, 1H), 7.18 - 6.95 (m, 1H), 6.69 (s, 1H), 3.93 (s, 3H), 3.25 (m, 2H), 2.89 - 2.53 (m, 9H), 2.42 (m, 5H), 2.21 (s, 3H), 2.05 (d, *J*= 11.4 Hz, 2H), 1.92 (s, 3H), 1.86 (s, 3H), 1.81 - 1.69 (m, 2H). MS-ESI (m/z): 676.1964$(M+H)^+$.

**Example 34**

Compound 34:

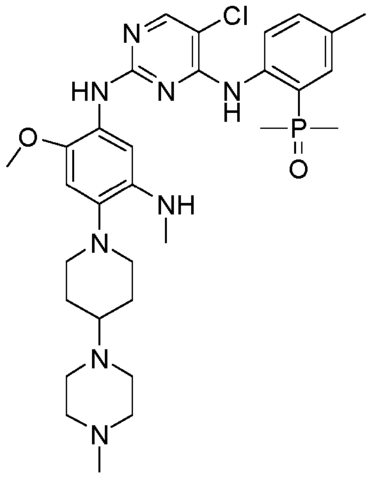

Compound 25C (305 mg, 0.50 mmol), $K_2CO_3$ (138 mg, 1.00 mmol), methyl iodide (142 mg, 1.00 mmol) and 10 mL of DMF were weighed and added into a 25mL round-bottom flask, and stirred overnight at room temperature. After the reaction was completed, the reaction solution was purified through column chromatography to obtain compound 34 (65 mg, yield 20.5%). MS-ESI (m/z): 627.3125$(M+H)^+$.

**Example 35**

**Compound 35:**

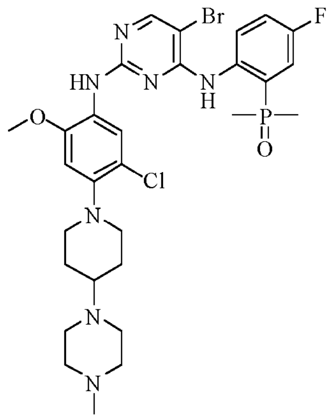

Compound 14B (403.27 mg,1.19 mmol), compound 11B (500 mg,1.32 mmol), 15% hydrogen chloride ethanol solution (963 mg, 3.96 mmol) and 7.5 mL of ethylene glycol monomethyl ether were added into a reaction flask, and sealed and reacted at 100°C for 4 hours. After TLC test showed that the reaction was completed, the reaction solution was cooled to room temperature, and then added with 15 mL of water and saturated $NaHCO_3$ aqueous solution to adjust a pH to be about 8.0, to precipitate a large amount of solids which were continuously stirred for about 0.5 hour and then subjected to suction filtration. The filter cake was washed with water, and then dried. The crude product was pulped with 6 mL of EA and purified to obtain compound 35 (380 mg, yield 38%). $^1H$ NMR (400 MHz, $CDCl_3$) δ=10.26 (s, 1H), 8.38 (m, 1H), 8.22 (d, *J*=10.8 Hz, 2H), 7.46 - 7.32 (m, 2H), 7.03 (m, 1H), 6.62 (s, 1H), 3.88 (s, 3H), 3.41 (d, *J*=11.6 Hz, 2H), 2.81 - 2.22 (m, 14H), 1.95 -1.79 (m, 10H). MS-ESI (m/z): 680.1686$(M+H)^+$.

**Example 36**

**Compound 36A:**

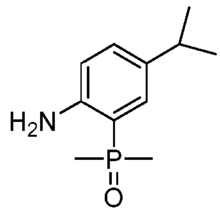

2-iodo-4-isopropylaniline (3.0 g, 11.5 mmol), $K_2CO_3$ (2.23 g, 16.1 mmol), Xantphos (0.174 g, 0.46 mmol), $Pd(OAc)_2$ (0.103 g, 0.46 mmol), dimethylphosphine oxide (1.08 g, 13.8 mmol), and 18 mL of DMF were added into a 50mL three-necked flask, heated to 100°C and reacted for about 3 hours until TLC test showed that the raw materials were completely reacted. The reaction solution was cooled to room temperature and subjected to suction filtration, then added with 54 mL of $H_2O$ to precipitate a large amount of yellow solids which were then subjected to suction filtration. The filtrate was extracted with 25 mL of dichloromethane thrice, dried with anhydrous sodium sulfate and filtered. The filtrate was concentrated to obtain oily compound 36A (2.60 g), which was used in the next reaction without purification.

**Compound 36B:**

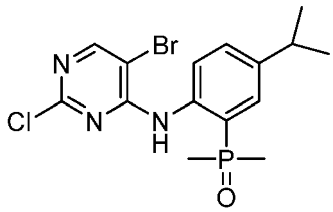

Compound 36A (2.60 g), 5-bromo-2,4-dichloropyrimidine (2.88 g, 12.6 mmol), $K_2CO_3$ (1.91 g, 13.8 mmol) and 15 mL of DMSO were added into a 50mL single-necked flask, heated to 60°C and reacted for about 3 hours until TLC text

showed that compound 36A was completely reacted. The reaction solution was cooled to room temperature, added with 45 mL of $H_2O$, and extracted with 50 mL of ethyl acetate twice. The organic phases were combined, washed with 50 mL of water twice, washed with 50 mL of saturated sodium chloride twice, dried with anhydrous sodium sulfate, and concentrated under reduced pressure to obtain pink solids, which were added with a mixed solvent (PE:EA=3:1), pulped and filtered to obtain solids which were dried to obtain compound 36B (2.5 g, yield 54%).

**Compound 36:**

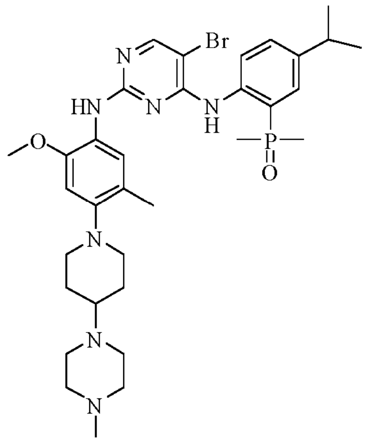

Compound 36B (500 mg, 1.24 mmol), compound 1F hydrochloride (484.11 mg, 1.36 mmol) and 4 mL of ethylene glycol monomethyl ether were added into a reaction flask, and then reacted at 100°C for 5-6 hours. After TLC test showed that compound 36B was completely reacted, the reaction solution was cooled to room temperature, then added with 4 mL of water, and a pH was adjusted to about 8.0 with saturated $NaHCO_3$ aqueous solution, to precipitate solids which were continuously stirred for 0.5 hour and then subjected to suction filtration. The filter cake was washed with water, and purified through column chromatography to obtain compound 36 (400 mg, yield 47.11%). $^1$H NMR (400 MHz, $CDCl_3$) δ=10.39 (s, 1H), 8.40 (dd, *J*=8.6, 4.6 Hz, 1H), 8.18 (s, 1H), 8.05 (s, 1H), 7.47 - 7.33 (m, 2H), 7.12 (dd, *J*=14.4, 1.6 Hz, 1H), 6.62 (s, 1H), 3.86 (s, 3H), 3.15 (d, *J*=11.6 Hz, 2H), 2.93 (m, 1H), 2.78 - 2.13 (m, 17H), 1.96 (m, 2H), 1.85 (d, *J*=13.2 Hz, 6H), 1.73 (m, 2H), 1.28 (d, *J*=6.8 Hz, 6H). MS-ESI (m/z):706.2601(M+Na)$^+$.

**Example 37**

**Compound 37:**

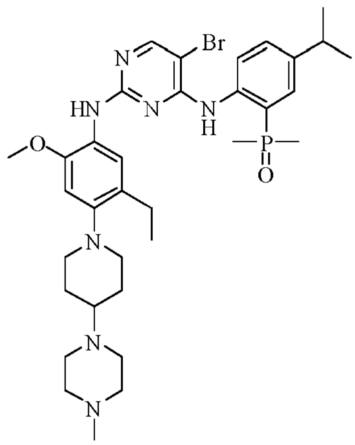

Compound 36B (440 mg, 1.09 mmol), compound 12A (400 mg, 1.20 mmol), 15% hydrogen chloride ethanol solution (798 mg, 3.28 mmol) and 6.0 mL of ethylene glycol monomethyl ether were added into a reaction flask, sealed and

reacted at 100°C for 5-6 hours. After TLC test showed that the reaction was completed, the reaction solution was cooled to room temperature, and then added with 6 mL of saturated $NaHCO_3$ aqueous solution and 12 mL of $H_2O$ to precipitate a large amount of solids, which were continuously stirred for about 0.5 hour and then subjected to suction filtration. The filter cake was washed with water, separated and purified to obtain compound 37 (228 mg, yield 29.8%). $^1$H NMR (400 MHz, $CDCl_3$) δ=10.34 (s, 1H), 8.35 (dd, *J*=8.8, 4.8 Hz, 1H), 8.19 (s, 1H), 8.08 (s, 1H), 7.37 (m, 2H), 7.12 (d, *J*=14.4 Hz, 1H), 6.67 (s, 1H), 3.86 (s, 3H), 3.08 (m, 2H), 2.92 (m, 1H), 2.80 - 2.44 (m, 11H), 2.42 - 2.26 (m, 5H), 1.95 (d, *J*=11.2 Hz, 2H), 1.84 (d, *J*=13.2 Hz, 6H), 1.72 (m, 2H), 1.28 (d, *J*=6.8 Hz, 6H), 1.03 (t, *J*=7.4 Hz, 3H). MS-ESI (m/z): 720.2757(M+Na)$^+$.

**Example 38**

**Compound 38:**

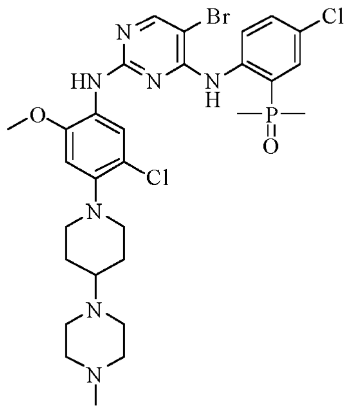

Compound 14B (309 mg, 0.91 mmol), compound 33B (300 mg, 0.76 mmol), 15% hydrogen chloride ethanol solution (554 mg, 2.28 mmol) and 4.5 mL of ethylene glycol monomethyl ether were added into a reaction flask, sealed and reacted at 100°C for 5-6 hours. After TLC test showed that the reaction was completed, the reaction solution was cooled to room temperature and added with 5 mL of saturated $NaHCO_3$ aqueous solution and 10 mL of $H_2O$, and extracted with 30 mL of dichloromethane thrice. The organic phases were combined, washed with 30 mL of saturated NaCl aqueous solution, and the organic phase was concentrated to dryness, and then separated and purified to obtain compound 38 (154 mg, yield 29.1%). MS-ESI (m/z): 696.1377(M+H)$^+$. $^1$H NMR (400 MHz, $CDCl_3$) δ = 10.30 (s, 1H), 8.35 (m, 1H), 8.26 (m, 2H), 7.48 - 7.30 (m, 2H), 7.05 (m, 1H), 6.68 (s, 1H), 3.89 (s, 3H), 3.38 (d, *J* = 11.6 Hz, 2H), 2.86 - 2.28 (m, 14H), 2.05 -1.72 (m, 10H).

**Comparative Example 1: Preparation of control compound 1**

**Control compound 1B (intermediate):**

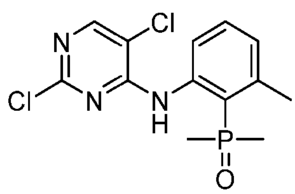

Referring to the synthesis of compound 10B, compound 5-bromo-2,4-dichloropyrimidine therein was simply replaced with compound 2,4, 5-trichloropyrimidine to obtain control compound 1B.

**Control compound 1:**

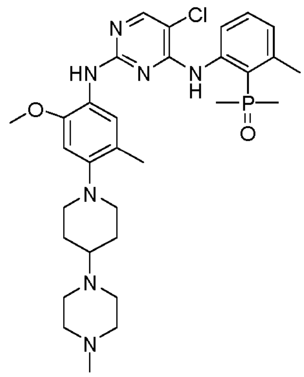

Referring to the synthesis of compound 10, compound 10B therein was simply replaced with control compound 1B to obtain control compound 1. $^1H$ NMR (400 MHz, methanol-d4) δ=8.02 (s, 1H), 7.93 (dd, J=8.0, 4.0 Hz, 1H), 7.64 (s, 1H), 7.42 (t, J=8.0 Hz, 1H), 7.15 (dd, J=7.6, 3.6 Hz, 1H), 6.69 (s, 1H), 3.84 (s, 3H), 3.11 (d, J=11.6 Hz, 2H), 3.02 - 2.41 (m, 13H), 2.32 (m, 4H), 2.04 (s, 3H), 2.00 (d, J=12.6 Hz, 2H), 1.94 (s, 3H), 1.90 (s, 3H), 1.68 (m, 2H). MS-ESI (m/z): 612.2981 (M+H)+.

**Comparative Example 2: Preparation of control compound 2**

**Control compound 2A (intermediate):**

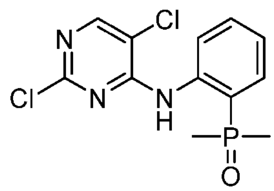

(2-aminophenyl)-dimethylphosphine oxide (2.5 g, 15.00 mmol), 2,4, 5-trichloropyrimidine (2.70 g, 15.00 mmol), $K_2CO_3$ (2.45 g, 67.75 mmol), $nBu_4NHSO_4$ (0.5 g, 1.50 mmol) and 50 mL of DMF were added into a 100mL three-necked flask, heated to 65°C and reacted for about 4.5 hours until TLC test showed that the reaction was completed. Then, the reaction solution was cooled to room temperature and added with 200 mL of $H_2O$ to precipitate a large amount of yellow solids which were continuously stirred for about 0.5 hour and then subjected to suction filtration. The filter cake was washed with100 mL of $H_2O$, and dried to obtain control compound 2A (2.84 g, yield 60.0%). MS-ESI (m/z): 316.0178(M+H)$^+$.

**Control compound 2:**

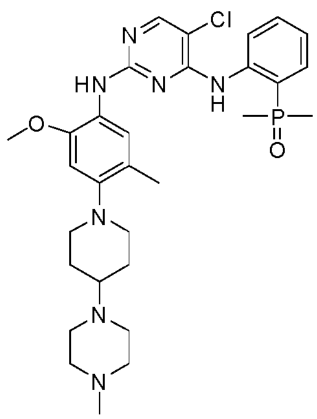

Control compound 2A (335 mg, 1.06 mmol), compound 1F (406 mg, 1.27 mmol), 15% hydrogen chloride ethanol

solution (774 mg, 3.18 mmol), and 4.5 mL of ethylene glycol monomethyl ether were added into a reaction flask, and then sealed and reacted at 120°C for 5-6 hours. After TLC test showed that the reaction was completed, the reaction solution was cooled to room temperature, and then 15 mL of saturated $NaHCO_3$ aqueous solution was added to precipitate a large amount of solids which were continuously stirred for 0.5 hour and then subjected to suction filtration. The filter cake was washed with water, and the filter cake was pulped with 9 mL of mixed solvent ($EtOH/H_2O$=1/2) and purified to obtain control compound 2 (320 mg, yield 50.5%). $^1H$ NMR (400 MHz, $CDCl_3$) δ=10.80 (s, 1H), 8.63 (dd, *J*=8.4, 4.4 Hz, 1H), 8.10 (s, 1H), 8.04 (s, 1H), 7.51 (m, 1H), 7.38 - 7.26 (m, 2H), 7.13 (m, 1H), 6.63 (s, 1H), 3.86 (s, 3H), 3.16 (d, *J*=12.0 Hz, 2H), 2.61 (m, 9H), 2.32 (s, 3H), 2.18 (s, 3H), 2.06 (m, 2H), 1.96 (m, 2H), 1.87 (s, 3H), 1.83 (s, 3H), 1.72 (m, 2H). MS-ESI (m/z): 598.2826$(M+H)^+$.

**Comparative Example 3: Preparation of control compound 3**

**Control compound 3A (intermediate):**

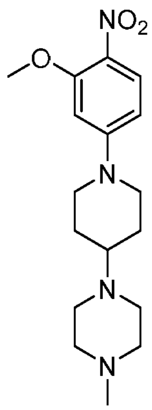

5-fluoro-2-nitroanisole (2.0 g, 11.69 mmol), 1-methyl-4-(4-piperidinyl)piperazine (2.57 g, 14.02 mmol), potassium carbonate (3.25 g, 23.37 mmol) and 30 mL of DMF were added into a 100mL round-bottom flask, heated to 120°C and reacted for about 3 hours until TLC test showed that the reaction was completed. The reaction solution was cooled to room temperature, added with 30 mL water, and extracted with 30 mL of ethyl acetate thrice. The organic phases were combined, and the organic phases were washed with 30 mL of saturated sodium chloride aqueous solution, dried with anhydrous sodium sulfate, filtered and concentrated to obtain control compound 3A (3.57 g, yield 91.3%).

**Control compound 3B (intermediate):**

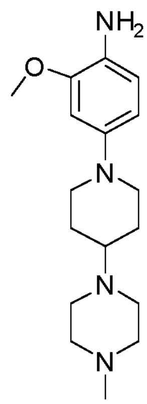

Control compound 3A (3.57 g, 10.68 mmol), 0.36 g of 5% Pd/C and 72 mL of methanol were replaced with hydrogen for 2-3 times, and stirred overnight at room temperature in a hydrogen atmosphere (normal pressure) until TLC test showed that the reaction was completed. Then, after diatomite filtration, the filtrate was concentrated in vacuum and

separated and purified through column chromatography to obtain control compound 3B (2.17 g, yield 66.8%). MS-ESI (m/z): 305.2315(M+H)$^+$.

**Control compound 3:**

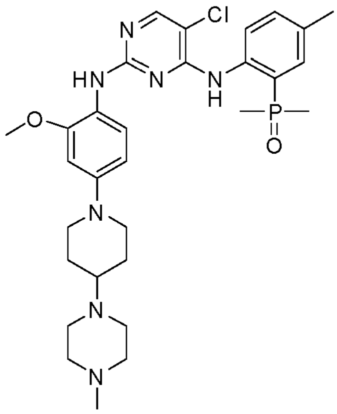

Compound 1B (400 mg, 1.21 mmol), control compound 3B (443 mg, 1.45 mmol), 15% hydrogen chloride ethanol solution (884 mg, 3.63 mmol) and 6 mL of ethylene glycol monomethyl ether were added into a reaction flask, sealed and reacted at 120°C for 5-6 hours until TLC test showed that the reaction was completed. Then, the reaction solution was cooled to room temperature, added with 6 mL of saturated $NaHCO_3$ aqueous solution and 6 mL of $H_2O$, and extracted with 30 mL of dichloromethane thrice. The organic phases were combined, and then washed with 20 mL of saturated aqueous solution of sodium chloride, dried with anhydrous sodium sulfate, filtered, concentrated, separated and purified through column chromatography (DCM/MeOH=8/1), and then pulped with 5 mL of mixed solvent (PE/EA=4/1) to obtain control compound 3 (257 mg, yield 35.4%). $^1$H NMR (400 MHz, $CDCl_3$) $\delta$=10.63 (s, 1H), 8.49 (dd, *J*=8.8, 4.8 Hz, 1H), 8.19 - 7.99 (m, 2H), 7.31 (d, *J*=8.8 Hz, 1H), 7.25 (s, 1H), 7.07 (d, *J*=14.0 Hz, 1H), 6.61 - 6.44 (m, 2H), 3.87 (s, 3H), 3.66 (d, *J*=12.0 Hz, 2H), 2.66 (m, 9H), 2.38 (s, 3H), 2.32 (s, 3H), 2.21 (s, 2H), 1.97 (m, 2H), 1.85 (s, 3H), 1.81 (s, 3H), 1.73 (m, 2H). MS-ESI (m/z): 598.2804(M+H)$^+$.

**Comparative Example 4: Preparation of control compound 4**

**Control compound 4A (intermediate):**

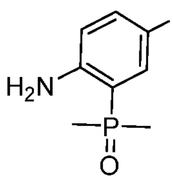

4-bromo-2-iodoaniline (30 g, 100.7 mmol), $K_2CO_3$ (22.27 g, 161.1 mmol), Xantphos (5.83 g, 10.1 mmol), $Pd(OAc)_2$ (1.12 g, 4.9 mmol), dimethylphosphine oxide (11.78 g, 150.9 mmol) and 450 mL of DMF were added into a 1L three-necked flask under the protection of $N_2$, heated to 100°C and reacted for about 3 hours until TLC test showed that the reaction was completed. Then, the reaction solution was cooled to room temperature, dropwise added with 1350 mL of $H_2O$, stirred and then subjected to suction filtration. The filter cake was washed with water and extracted with 500 mL of dichloromethane thrice. The organic phases were combined, and the organic phases were washed with 500 mL of saturated sodium chloride aqueous solution twice, dried with anhydrous sodium sulfate, filtered and concentrated to obtain control compound 4A (14 g, yield 56%).

**Control compound 4B (intermediate):**

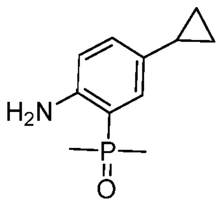

Compound 4A (12 g, 48.4 mmol), cyclopropylboronic acid (8.31 g, 96.7 mmol), $K_2CO_3$ (13.36 g, 96.7 mmol), Pd(dppf)$Cl_2$ (1.77 g, 2.42 mmol), 1,4-dioxane (120 mL), and $H_2O$ (12 mL) were added into a 500mL three-necked flask, heated to 100°C and reacted for about 8 hours under the protection of $N_2$ until TLC test showed that the reaction was completed. Then, the reaction solution was cooled to room temperature, added with 150 mL of $H_2O$, stirred and then subjected to suction filtration. The filter cake was washed with dichloromethane and then the system was allowed to settle for liquid separation. The aqueous phases were then extracted with 150 mL of dichloromethane thrice. Then, the organic phases were combined, dried with anhydrous sodium sulfate, filtered, concentrated and then separated and purified through column chromatography to obtain control compound 4B (7.5 g, yield 74%).

**Control compound 4C (intermediate):**

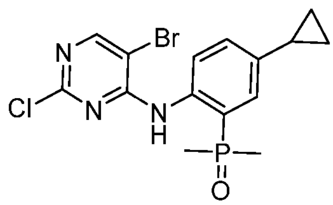

Compound 4B (7.5 g, 35.8 mmol), $K_2CO_3$ (5.95 g, 43.1mmol), 5-bromo-2,4-dichloropyrimidine (8.99 g, 39.5 mmol) and DMSO (45 mL) were added into a 100mL reaction flask, heated to 60°C and reacted for about 2.5 hours until TLC test showed that the reaction was completed. Then, the reaction solution was cooled to room temperature, and dropwise added with 135 mL of $H_2O$ to precipitate a large amount of claybank solids, which were continuously stirred for 1 hour. The filter cake was washed with 100 mL of $H_2O$, and dried to obtain control compound 4C (7.4 g, yield 51.6%).

**Control compound 4:**

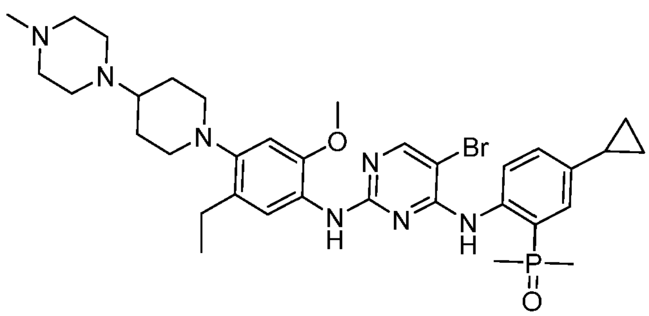

Compound 4C (1.7 g, 4.24 mmol), compound 12A (1.55 g, 4.66 mmol), trifluoroacetic acid (1.21 g, 10.6 mmol) and ethylene glycol monomethyl ether (27 mL) were added into a 100mL reaction flask, heated to 100°C and reacted for about 7 hours until TLC test showed that the reaction was completed. Then the reaction was cooled to room temperature, dropwise added with 54 mL of $H_2O$, and then dropwise added with 27 mL of saturated $NaHCO_3$ aqueous solution to precipitate a large amount of solids which were continuously stirred for 1 hour and then subjected to suction filtration. The filter cake was washed with water. The filter cake was pulped by 15 mL of ethyl acetate and purified, and then filtered and dried to obtain control compound 4 (1.68 g, yield 56.8%). $^1$H NMR (400 MHz, $CD_3OD$) $\delta$=8.13 (s, 1H), 7.88 (dd, *J*=8.8, 4.8 Hz, 1H), 7.66 (s, 1H), 7.43 (dd, *J*=14.0, 2.2 Hz, 1H), 7.19 (m, 1H), 6.76 (s, 1H), 3.85 (s, 3H), 3.04 (m, 2H), 2.32-2.75 (m, 16H), 2.01 (m, 3H), 1.83 (m, 6H), 1.74-1.65 (m, 2H), 1.06 (m, 2H), 0.96 (t, *J*=7.6 Hz, 3H), 0.76 (m, 2H). MS -ESI (m/z):

696.2711 $(M+H)^+$.

**Test Example 1** Enzymatic activity **test 1**

1. Test purpose: to detect the effect of the test compounds on the activity of EGFR kinase
2. Test information:

To assess the effect of compounds on the activity of wild-type and mutant EGFR kinases, kinase activity was tested by measuring ATP consumption in an enzymatic reaction using an ADP-Glo (Promega Corporation) kit. Samples to be tested were dissolved in DMSO and diluted in a gradient. In a microplate, an EGFR kinase, a reaction buffer (containing Tris-HCl with a pH of 7.5, $MgCl_2$, DTT and BSA), a kinase substrate Poly(Glu4,Tyr1) and a sample (a total volume of 20 μL per well) were added per well, while a blank control (no enzyme and sample) and a negative control (no sample) were set up; incubated for 15 minutes at 23°C; added with 5 μL of ATP, and reacted at 23°C for 60 minutes; added with ADP-Glo Reagent, and continuously reacted for 40 minutes at room temperature to inactivate the redundant ATP; then, added with a Kinase Detection Reagent, and reacted at room temperature for 30 minutes, then the chemiluminescence intensity L of each well was measured. The inhibition rate of the compound was calculated according to the value of the chemiluminescence intensity L, and the inhibition rate = $[1-(L_{sample} - L_{blank})/(L_{negative} - L_{blank})] \times 100\%$. $IC_{50}$ values were calculated according to the above calculation using 4Parameter Logistic Model in XLfit software.

3. Test results: $IC_{50}$ values of the enzymatic activities of the compounds according to the examples of the present invention and the compounds of the comparative examples to the EGFR-Del19, EGFR-C797S/Del19, EGFR-T790M/Del19, EGFR-T790M/L858R, EGFR-C797S/T790M/L858R, EGFR-C797S/T790M/Del19 and EGFR-WT were shown in Table 1 and Table 2.
4. Conclusion: as can be seen from Table 1 and Table 2, the compounds according to the examples of the present invention have better selectivity on the enzymatic activity of the wild-type EGFR (EGFR-WT), and better inhibitory effects on the enzymatic activity of single, double and triple mutations of EGFR (e.g., EGFR-Del19, EGFR-C797S/Del19, EGFR-T790M/Del19, EGFR-T790M/L858R, EGFR-C797S/T790M/L858R, and EGFR-C797S/T790M/Del19), which are all greatly superior to the compounds according to the comparative examples.

Table 1

| Test compounds | EGFR-Del19 $IC_{50}$ (nM) | EGFR-C797S/Del19 $IC_{50}$ (nM) | EGFR-T790M/Del19 $IC_{50}$ (nM) | EGFR-T790M/L858R $IC_{50}$ (nM) |
|---|---|---|---|---|
| Compound 1 | 20.6 | 19.8 | 4.1 | 13.9 |
| Compound 2 | 23.5 | 13.8 | 3.9 | 24.8 |
| Compound 5 | 37.1 | 73.7 | 1.9 | 40.6 |
| Compound 6 | 29.9 | 33.8 | 1.1 | 28.8 |
| Compound 9 | 46.1 | 41.9 | 11.0 | - |
| Compound 11 | 27.4 | 10.7 | 3.4 | 24.9 |
| Compound 12 | 33.7 | 9.8 | 6.9 | 20.5 |
| Compound 14 | 16.6 | 5.7 | 3.6 | 16.6 |
| Compound 24 | - | 41.6 | - | - |
| Compound 32 | 19.6 | 10.5 | 8.9 | 18.7 |
| Control compound 1 | >250 | >250 | >250 | >250 |
| Control compound 2 | >250 | >250 | >250 | >250 |
| Control compound 3 | >250 | >250 | >250 | >250 |

Table 2

| Test compounds | EGFR-C797S/T790M/L858R $IC_{50}$ (nM) | EGFR-C797S/T790M/Del19 $IC_{50}$ (nM) | EGFR-WT $IC_{50}$ (nM) |
|---|---|---|---|
| Compound 1 | 22.2 | 10.8 | 95.4 |
| Compound 2 | 21.3 | 4.2 | 168.9 |

(continued)

| Test compounds | EGFR-C797S/T790M/L858R $IC_{50}$ (nM) | EGFR-C797S/T790M/Del19 $IC_{50}$ (nM) | EGFR-WT $IC_{50}$ (nM) |
|---|---|---|---|
| Compound 5 | 30.0 | 6.9 | 112.8 |
| Compound 6 | 16.8 | 6.6 | - |
| Compound 9 | 39.9 | 26.4 | 161.8 |
| Compound 11 | 12.7 | 4.4 | 98.6 |
| Compound 12 | 16.9 | 7.5 | 160.5 |
| Compound 14 | 10.1 | 6.6 | 65.9 |
| Compound 24 | 54.0 | 24.5 | - |
| Compound 32 | 15.9 | 9.7 | 130.7 |
| Control compound 1 | >250 | 160.5 | >250 |
| Control compound 2 | >250 | >250 | >250 |
| Control compound 3 | >250 | >250 | >250 |

Test Example 2 Enzymatic activity test 2

1. Test purpose: to detect the effect of the test compounds on the activity of ALK kinase
2. Test information:
To assess the effect of compounds on the activity of EML4-ALK kinases, kinase activity was tested by measuring ATP consumption in an enzymatic reaction using an ADP-Glo (Promega Corporation) kit. Samples to be tested were dissolved in DMSO and diluted in a gradient. In a microplate, an EML4-ALK kinase, a reaction buffer (containing Tris-HCl with a pH of 7.5, $MgCl_2$, DTT and BSA), a kinase substrate IGF1 and a sample (a total volume of 20 μL per well) were added per well, while a blank control (no enzyme and sample) and a negative control (no sample) were set up; added with 5 μL of ATP, and reacted at 23°C for 60 minutes; added with ADP-Glo Reagent, and continuously reacted for 40 minutes at room temperature to inactivate the redundant ATP; then, added with a kinase detection reagent, and reacted at room temperature for 30 minutes, then the chemiluminescence intensity L of each well was measured. The inhibition rate of the compound was calculated according to the value of the chemiluminescence intensity L, and the inhibition rate = $[1-(L_{sample} - L_{blank})/(L_{negative} - L_{blank})] \times 100\%$. $IC_{50}$ values were calculated according to the above calculation using 4Parameter Logistic Model in XLfit software.
3. Test results: $IC_{50}$ values of the activities of the compounds according to the examples of the present invention and the compounds of the comparative examples to the ALK kinase were shown in Table 3.
4. Conclusion: as can be seen from Table 3, the compounds according to the examples of the present invention have better inhibitory effects on the enzymatic activity of the ALK kinase, which are all greatly superior to the compounds according to the comparative examples.

Table 3

| Test compounds | EML4-ALK $IC_{50}$ (nM) |
|---|---|
| Compound 1 | 14.9 |
| Compound 2 | 17.3 |
| Compound 5 | 16.3 |
| Compound 6 | 14.6 |
| Compound 11 | 31.2 |
| Compound 12 | 21.3 |
| Compound 14 | 12.9 |
| Compound 32 | 13.7 |
| Control compound 1 | >250 |

(continued)

| Test compounds | EML4-ALK $IC_{50}$ (nM) |
|---|---|
| Control compound 2 | >250 |
| Control compound 3 | 106.1 |

**Test Example 3 Cell anti-proliferation test 1**

1. Test purpose: to detect the effect of the test compounds on the proliferation of cell lines containing EGFR mutations in BaF3 cells
2. Test information:

2.1. Cell information: Cell lines containing EGFR mutations in BaF3 were from WuXiAppTec:

BaF3-cell lines expressing EGFR mutation

EGFR-T790M/Del19

EGFR-C797S/Del19

EGFR-T790M/L858R

EGFR-C797S/L858R

EGFR-C797S/T790M/L858R

EGFR-C797S/T790M/Del19

EGFR-WT wild-type cell lines

2.2. Test method

Day 0: Cells were seeded in a plate.

a. An ultraviolet lamp of a biological safety cabinet was turned on and countdown was started for 30 minutes.
b. A medium was preheated in a 37°C water bath.
c. After the ultraviolet irradiation was finished, the biological safety cabinet was opened. The pre-heated medium, PBS, and the like, were wiped with alcohol and placed in the biological safety cabinet.
d. The cells were removed from an incubator, blown to a uniform cell suspension in the biological safety cabinet and counted.
e. Based on the cell counting results, the cell suspension was adjusted to a density of 3,000 cells per well and 50 microliters per well, and seeded in 384-well plates.
f. The cells after seeding were incubated in a 5% $CO_2$ incubator at 37°C for 3 hours.
g. Compounds were added to the cell plates using Tecan (liquid workstation).

Day 3: the cell plate added with the compounds and CTG (CellTiter-Glo chemiluminescence cell activity assay reagent) were equilibrated at room temperature, 25 μl of CTG was added to each well, centrifuged at 1,000 rpm for 1 minute, and shaken for 1-2 minutes. And centrifuging for 1 minute at 1,000 rpm again, the cell plates were allowed to settle for 10 minutes to detect a signal value in Envision. The $IC_{50}$ of each compound was calculated by computer fitting with XL-Fit analysis software (the $IC_{50}$ of the compound on the cell activity inhibition could be obtained by reading the concentrations of the corresponding compounds at 50% inhibition rate). Inhibition rate% = (reading value of the control group without drug - reading value of the sample)/reading value of the control group without drug × 100.

3. Test results: the activity inhibition $IC_{50}$ values of the compounds according to the examples of the present invention, the compounds according to the comparative examples and the main drugs for treating non-small cell lung cancer in the

market such as Brigatinib and Osimertinib, on the Ba/F3 cells expressing EGFR-T790M/Del19, EGFR-C797S/Del19, EGFR-T790M/L858R, EGFR-C797S/L858R, EGFR-C797S/T790M/L858R, EGFR-C797S/T790M/Del19 and EGFR-WT were shown in Table 4 and Table 5.

4. Conclusion: as can be seen from Table 4 and Table 5, the compounds according to the examples of the present invention have better selectivity on the activity of the Ba/F3 cells expressing EGFR wild-type (EGFR-WT), have better inhibition effects on the proliferation of the Ba/F3 cells expressing EGFR double mutations and triple mutations (such as EGFR-T790M/Del19, EGFR-C797S/Del19, EGFR-T790M/L858R, EGFR-C797S/L858R, EGFR-C797S/T790M/L858R and EGFR-C797S/T790M/Del19), and particularly, the inhibition activity of the compounds 1, 2, 4, 5, 6, 9, 11, 12, 14, 23, 24, 25, 28, 29, 30, 32, 33, 35, 36, 37 and 38 according to the examples on the Ba/F3 cell lines expressing EGFR double mutations and triple mutations are greatly better than that of the control compounds 1, 2 and 3 and the marketed drugs such as Brigatinib and Osimertinib.

Table 4

| Test compounds | EGFR-T790M/Del19 $IC_{50}$ (nM) | EGFR-C797S-Del19 $IC_{50}$ (nM) | EGFR-T790M/L858R $IC_{50}$ (nM) | EGFR-C797S/L858R $IC_{50}$ (nM) |
|---|---|---|---|---|
| Compound 1 | 14.6 | 39.9 | 48.3 | 90.0 |
| Compound 2 | 6.5 | 48.1 | 22.5 | 32.6 |
| Compound 4 | 43.8 | 80.6 | - | - |
| Compound 5 | 38.1 | 50.0 | 55.9 | 90.6 |
| Compound 6 | 10.5 | 51.0 | 29.3 | 37.1 |
| Compound 9 | 24.1 | - | - | - |
| Compound 11 | 17.3 | 66.1 | 21.6 | 24.1 |
| Compound 12 | 8.1 | 29.3 | 24.6 | 113.4 |
| Compound 13 | 29.0 | 60.1 | - | - |
| Compound 14 | 2.3 | 43.9 | 17.5 | 31.7 |
| Compound 23 | 54.3 | 79.6 | - | - |
| Compound 24 | 23.5 | 41.6 | - | - |
| Comparative Compound 25 | 34.2 | - | - | - |
| Compound 28 | 50.6 | 85.2 | - | - |
| Compound 29 | 40.5 | 57.8 | 104.6 | - |
| Compound 30 | 29.5 | 55.6 | - | - |
| Compound 32 | 3.8 | 51.6 | 16.9 | 43.8 |
| Compound 33 | 25.8 | - | 24.7 | 84.9 |
| Compound 35 | 23.4 | - | 46.1 | 179.7 |
| Compound 36 | 48.5 | - | 32.1 | 133.0 |
| Compound 37 | 41.0 | - | 18.9 | 103.4 |
| Compound 38 | 26.3 | - | - | - |
| Control compound 1 | 165.8 | 184.0 | 156.5 | 572.6 |
| Control compound 2 | 171.8 | 176.6 | - | - |
| Control compound 3 | 216.2 | 202.6 | - | - |
| Brigatinib | 164.4 | 177.8 | 148.0 | 547.7 |
| AZD9291 Osimertinib | - | 513.4 | - | 1115.0 |

Table 5

| Test compounds | EGFR-C797S/T790 M/L858R $IC_{50}$ (nM) | EGFR-C797S/T790 M/Del19 $IC_{50}$ (nM) | EGFR-WT $IC_{50}$ (nM) |
|---|---|---|---|
| Compound 1 | 96.9 | 21.5 | 1288.9 |
| Compound 2 | 64.1 | 16.5 | 1260.1 |
| Compound 4 | 203.7 | 50.5 | 815.1 |
| Compound 5 | 119.0 | 26.8 | 1775.1 |
| Compound 6 | 74.6 | 19.6 | 1228.5 |
| Compound 9 | 185.7 | 34.2 | 1892.2 |
| Compound 11 | 82.6 | 19.0 | 1092.7 |
| Compound 12 | 37.1 | 13.5 | 785.3 |
| Compound 13 | 168.4 | 36.4 | 691.4 |
| Compound 14 | 76.4 | 18.9 | 619.7 |
| Compound 23 | 128.7 | 34.1 | 1614.9 |
| Compound 24 | 54.0 | 24.5 | 887.1 |
| Compound 28 | 134.0 | 35.5 | 1208.0 |
| Compound 29 | 125.3 | 32.2 | 1492.4 |
| Compound 30 | 62.9 | 25.8 | 1652.0 |
| Compound 32 | 85.9 | 22.7 | 805.7 |
| Compound 33 | 90.7 | 22.9 | 965.8 |
| Compound 35 | 126.9 | 19.4 | 2919.8 |
| Compound 36 | 95.9 | 45.1 | 2604.0 |
| Compound 37 | 96.8 | 87.0 | 4766.4 |
| Control compound 1 | 626.0 | 180.1 | 1603.4 |
| Control compound 2 | 582.6 | 221.4 | 1760.6 |
| Control compound 3 | 565.8 | 191.7 | 2343.3 |
| Brigatinib | 751.6 | 194.3 | 1700.0 |
| AZD9291 Osimertinib | 1171.0 | 800.2 | - |

**Test Example 4 Cell anti-proliferation test 2**

1. Test purpose: to detect the effect of the test compounds on the proliferation of cell lines containing EGFR mutations in PC9 cells
2. Test information:

2.1. Cell information: PC9 cells carried EGFR Del19 mutation, and the rest of mutant cells were constructed from PC9 cells according to the conventional stable cell strain construction method.

PC9-cell lines expressing EGFR mutation

EGFR-Del19

EGFR-T790M/Del19

EGFR-C797S/T790M/Del19

2.2. Test method:

The test compounds were diluted by 5 times each time with an appropriate concentration as an initial concentration of the test, and totally diluted into 6 concentration gradients. Brigatinib (purchased from Selleck) was diluted by three times with 5 μM as an initial concentration of the test, and totally diluted into 6 concentration gradients. The test compounds and Brigatinib were added into the above cells respectively, and incubated at 37°C and 5%$CO_2$ for 72 hours. SRB (Sulforhodamine B) detection method was used, and the optical density value of each well was read by a microplate reader at 490 nm wavelength.

The optical density of the cells at the drug action of 0 was set as a Tz value representing the value of the cells at the time that the drug was added. The optical density value of the cells after the solvent control DMSO acted for 72 hours was set as a C value. The optical density of the cells on which the test compounds acted for 72 hours was set as a Ti value. The response of the cells to the drug was calculated according to a method proposed by the U.S. NIH-NCI (National Institutes of Health-National Institute of Cancer): When Ti is more than or equal to Tz, the value was [(Ti-Tz)/(C-Tz)] × 100; and when Ti is less than Tz, the value was [(Ti-Tz)/Tz] × 100. $GI_{50}$ values (the concentration of the test compound required for 50% cell growth inhibition) were calculated according to the above calculation using a 4 Parameter Logistic Model in XLfit software.

3. Test results: the $GI_{50}$ values of the compounds according to the examples of the present invention, the compounds according to the comparative examples and Brigatinib (the main drug for treating the non-small cell lung cancer in the market) on the PC9 cells expressing EGFR-Del19, EGFR-T790M/Del19, and EGFR-C797S/T790M/Del19 were shown in Table 6.

4. Conclusion: as can be seen from Table 6, the compounds according to the examples of the present invention have better inhibitory effects on the proliferation activity of PC9 cells expressing EGFR single mutation, double mutation and triple mutation (such as EGFR-Del19, EGFR-T790M/Del19, and EGFR-C797S/T790M/Del19), and have inhibitory activity superior to that of the control compounds 1, 2 and 3 and the marketed drug Brigatinib.

Table 6

| Test compounds | EGFR-Del19 $GI_{50}$ (nM) | EGFR-T790M/Del19 $GI_{50}$ (nM) | EGFR-C797S/T790M/Del19 $GI_{50}$ (nM) |
|---|---|---|---|
| Compound 1 | 57.5 | 17.5 | 19.0 |
| Compound 2 | 48.4 | 12.8 | 15.6 |
| Compound 5 | 42.6 | 15.9 | 23.6 |
| Compound 6 | 65.9 | 10.7 | 20.8 |
| Compound 11 | 43.9 | 14.6 | 10.9 |
| Compound 12 | 35.6 | 13.4 | 9.7 |
| Compound 14 | 40.2 | 8.5 | 6.6 |
| Control compound 1 | 265.7 | 267.1 | 336.8 |
| Control compound 2 | 261.6 | 288.9 | 315.8 |
| Control compound 3 | 367.9 | 389.2 | 502.5 |
| Brigatinib | 286.9 | 255.8 | 516.1 |

**Test Example 5 Cell anti-proliferation test 3**

1. Test purpose: to detect the effect of the test compounds on the proliferation of cell lines containing ALK mutations in BaF3 cells

2. Test information:

2.1. Cell information: Cell lines containing ALK mutations in BaF3 were from WuXiAppTec:

Ba/F3-cell lines expressing ALK gene fusion and mutation

Ba/F3-EML-4-ALK-WT

Ba/F3-EML-4-ALK-L1196M

2.2. Test method

Day 0: Cells were seeded in a plate.

a. An ultraviolet lamp of a biological safety cabinet was turned on, and countdown was started for 30 minutes.
b. A medium was preheated in a 37°C water bath.
c. After the ultraviolet irradiation was finished, the biological safety cabinet was opened. The pre-heated medium, PBS, and the like, were wiped with alcohol and placed in the biological safety cabinet.
d. The cells were removed from an incubator, blown to a uniform cell suspension in the biological safety cabinet and counted.
e. Based on the cell counting results, the cell suspension was adjusted to a density of 3,000 cells per well and 50 microliters per well, and seeded in 384-well plates.
f. The cells after seeding were incubated in a 5% $CO_2$ incubator at 37°C for 3 hours.
g. Compounds were added to the cell plates using Tecan (liquid workstation).

Day 3: the cell plate added with the compounds and CTG were equilibrated at room temperature, 25 μl of CTG was added to each well, centrifuged at 1,000 rpm for 1 minute, and shaken for 1-2 minutes. And centrifuging for 1 minute at 1,000 rpm again, the cell plates were allowed to settle for 10 minutes to detect a signal value in Envision. The $IC_{50}$ of each compound was calculated by computer fitting with XL-Fit analysis software (the $IC_{50}$ of the compound on the cell activity inhibition could be obtained by reading the concentrations of the corresponding compounds at 50% inhibition rate). Inhibition rate% = (reading value of the control group without drug - reading value of the sample)/reading value of the control group without drug × 100.

3. Test results: the $IC_{50}$ values of the compounds according to the examples of the present invention and the compounds according to the comparative examples on the BaF3 cells expressing EML-4-ALK-WT and EML-4-ALK-L1196M were shown in Table 7.
4. Conclusion: as can be seen from Table 7, the compounds according to the examples of the present invention have very good inhibitory effects on the proliferation of the BaF3 cells expressing EML-4-ALK-WT and EML-4-ALK-L1196M, and have inhibitory activity superior to that of the control compounds 1, 2 and 3.

Table 7

| Test compounds | EML-4-ALK-WT $IC_{50}$ (nM) | EML-4-ALK-L1196M $IC_{50}$ (nM) |
|---|---|---|
| Compound 1 | 12.1 | 24.4 |
| Compound 2 | 8.0 | 13.1 |
| Compound 5 | 6.5 | 15.6 |
| Compound 6 | 4.8 | 7.4 |
| Compound 11 | 9.4 | 17.1 |
| Compound 12 | 10.1 | 12.5 |
| Compound 14 | 7.4 | 13.1 |
| Compound 29 | 8.7 | 15.2 |
| Compound 32 | 8.9 | 14.3 |
| Compound 33 | 10.7 | 16.2 |
| Compound 35 | 18.2 | 14.7 |
| Control compound 1 | 34.1 | 65.0 |
| Control compound 2 | 43.6 | 47.4 |
| Control compound 3 | 39.4 | 50.2 |

**Test example 6: In-vivo pharmacodynamic study on transplanted tumor model of engineered BaF3 EGFR-DTC(C797S/T790M/Del19) cells in nude mice**

The models used in this test were subcutaneous transplanted tumor models of engineered BaF3 EGFR-DTC(C797S/T790M/Del19) cells in BALB/c nude mice. The engineered BaF3 cells were cultured in vitro suspension. BaF3 EGFR-DTC(C797S/T790M/Del19) was cultured in 5% $CO_2$ incubator at 37°C in a RPMI-compound 140 Medium with 10% fetal calf serum, 100 U/mL penicillin,100 μg/mL streptomycin and 10 μg/mL Blastcidin. Passages were treated twice a week. When the cell number reached requirement, the cells were collected, counted and inoculated. 0.2 mL ($10^6$) cells (added with matrigel, 1:1 by volume) were subcutaneously inoculated in the right back of each mouse, and divided into groups for administration with 6 mice per group when the mean tumor volume reached 109 $mm^3$.

Administration mode and frequency: Oral gavage was carried out with an administration volume of 10 mL/kg, and the model group was administered with the same volume of solvent. The administration was once per day and continuously carried out for 14 days. The doses of the compound 2 and Brigatinib were 25 mg/kg and 50 mg/kg, and the dose of the compound 33 was 25 mg/kg.

General conditions such as spirit, activity, food intake and the like of the mice were observed every day, and the weight was measured thrice every week; the short diameter (a) and the long diameter (b) of each mouse tumor were measured thrice a week with a vernier caliper, and the tumor volume was calculated according to the formula $(a^2 \times b)/2$. The Relative Tumor Volume (RTV) was calculated from the measured tumor volume, wherein $RTV=V_t/V_0$, wherein $V_0$ was the tumor volume at random grouping (i.e., $d_0$) and $V_t$ was the tumor volume at each measurement (i.e., $d_n$). The relative tumor proliferation rate of the evaluation index of the anti-tumor activity was calculated according to the following formula: relative tumor proliferation rate T/C (%):

$$T/C\ \% = \frac{T_{RTV}}{C_{RTV}} \times 100\%$$

(Note: $T_{RTV}$: treatment group RTV; $C_{RTV}$: model control group RTV. According to the therapeutic effect evaluation standard in the Technical Guidelines for Non-clinical Research of Cytotoxic Anti-tumor Drugs Issued by National Medical Products Administration of China, the value (T/C%≤40%) was effective).

The effects of various tested samples on the tumor situation of transplanted tumor model of BaF3 EGFR-DTC(C797S/T790M/Del19) cells in nude mice were shown in Tables 8-9.

Table 8: Mean tumor volume ($mm^3$) of each group of transplantable tumor models of BAF3 EGFR-DTC (C797s/T790M/Del19) in nude mice at different time points

| Test compounds | Tumor volume ($mm^3$) | | | | | | |
|---|---|---|---|---|---|---|---|
| | Day 0[a] | Day 3 | Day 5 | Day 7 | Day 10 | Day 12 | Day 14 |
| Solvent control | 109 | 150 | 188 | 415 | 622 | 743 | ▲ |
| Compound 2 (25 mg/kg) | 109 | 87 | 62 | 41 | 31 | 27 | 41 |
| Compound 2 (50 mg/kg) | 109 | 67 | 44 | 32 | 22 | 17 | 12 |
| Compound 33 (25 mg/kg) | 109 | 75 | 52 | 39 | 27 | 21 | 16 |
| Brigatinib (25mg/kg) | 109 | 139 | 152 | 276 | 476 | 755 | 1046 |
| Brigatinib (50mg/kg) | 109 | 115 | 121 | 146 | 220 | 378 | 509 |

Note: a. refers to days after administration.
▲:In the model control group, 3 mice died on day 10, 2 mice died on day 12 and 1 mouse died on the day 13. Autopsy showed that the death was caused by tumor metastasis.

Table 9: Relative tumor proliferation rate T/C (%) of transplanted tumor models of BaF3 EGFR-DTC(C797S/T790M/Del19) in nude mice

| Time<br>Test compounds | Day 3<br>T/C (%) | Day 5<br>T/C (%) | Day 7<br>T/C (%) | Day 10<br>T/C (%) | Day 12▲<br>T/C (%) |
|---|---|---|---|---|---|
| Compound 2 (25mg/kg) | 59.22 | 34.07 | 10.38 | 5.06 | 3.12 |
| Compound 2 (50 mg/kg) | 44.97 | 24.07 | 7.97 | 3.62 | 1.91 |
| Compound 33 (25mg/kg) | | 29.21 | | 4.84 | 2.56 |

(continued)

| Time<br>Test compounds | Day 3<br>T/C (%) | Day 5<br>T/C (%) | Day 7<br>T/C (%) | Day 10<br>T/C (%) | Day 12▲<br>T/C (%) |
|---|---|---|---|---|---|
| Brigatinib (25 mg/kg) | 93.86☆☆ | 81.48☆☆ | 67.69★★☆☆ | 77.68★★☆☆ | 84.64★★☆☆ |
| Brigatinib (50 mg/kg) | 77.16★★☆☆ | 64.55★★☆☆ | 35.39★★☆☆ | 34.99★★☆☆ | 41.67★★☆☆ |

★★: Compared with the original RTV data of the same dose group of the compound 2 during the same period of time,$p$<0.01;
☆☆: Compared with the original RTV data of the (25 mg/kg) dose group of the compound 33 during the same period of time,$p$<0.01;
▲: Due to all the animals in the model control died at day 13 after the administration, T/C (%) was calculated up to day 12 after the administration.

The test results show that: in the test, compound 2 under the doses of 25 mg/kg and 50 mg/kg, and compound 33 under the dose of 25 mg/kg can both obviously inhibit the tumor growth of transplanted tumors of BaF3 EGFR-DTC (C797S/T790M/Del19) in nude mice. 12 days after administration, the T/C% of compound 2 (25 mg/kg) and compound 2 (50 mg/kg) were 3.12% and 1.91% respectively, and the T/C% of compound 33 (25 mg/kg) was 2.56%. Brigatinib did not significantly inhibit the tumor growth of the transplanted tumor of BaF3 EGFR-DTC (C797S/T790M/Del19) in nude mice. 12 days after administration, the T/C% of Brigatinib (25 mg/kg) and Brigatinib (50 mg/kg) were 84.64% and 41.67% respectively.

It can be seen that the effect of compound 2 in inhibiting the tumor growth is significantly stronger than that of Brigatinib, and on day 12 of administration, the T/C% (25 mg/kg: 3.12% vs. 84.64%; 50 mg/kg: 1.91% vs. 41.67%) of compound 2 and Brigatinib are significantly different (p<0.01). In addition, the effect of compound 33 (25 mg/kg) on inhibiting the tumor growth is also significantly stronger than that of Brigatinib, and the T/C% difference between compound 33 and Brigatinib is significant (p<0.01) on day 12 of administration.

The above results indicate that compound 2 and compound 33 have obviously stronger effects of inhibiting the growth of the transplanted tumor of BaF3 EGFR-DTC (C797S/T790M/Del19) in nude mice than that of Brigatinib.

**Test Example 7: In-vivo pharmacodynamic study on transplanted tumor model of engineered BaF3 EML-4-ALK-L1196M cells in nude mice**

The models used in this test were subcutaneous transplanted tumor models of engineered BaF3 EML-4-ALK-L1196M cells in BALB/c nude mice. The engineered BaF3 cells were cultured in vitro suspension. BaF3 EML-4-ALK-L1196M was cultured in 5% $CO_2$ incubator at 37°C in a RPMI-compound 140 Medium with 10% fetal calf serum, 100 U/mL penicillin, 100 μg/mL streptomycin and 10 μg/mL Blastcidin. Passages were treated twice a week. When the cell number reached requirement, the cells were collected, counted and inoculated. 0.2 mL ($10^6$) of cells (added with matrigel, 1:1 by volume) were subcutaneously inoculated in the right back of each mouse, and divided into groups for dosing with 6 mice per group when the mean tumor volume reached 128 $mm^3$.

Administration mode and frequency: oral gavage was carried out with an administration volume of 10 mL/kg, and the model group was administered with the same volume of solvent. The administration was once per day and continuously carried out for 14 days. The dose of compound 2 was 50 mg/kg, the dose of compound 6 was 60 mg/kg, and the dose of compound 14 was 80 mg/kg.

General conditions such as spirit, activity, food intake and the like of the mice were observed every day, and the weight was measured thrice every week; the short diameter (a) and the long diameter (b) of each mouse tumor were measured thrice a week with a vernier caliper, and the tumor volume was calculated according to the formula ($a^2 \times b$)/2. The Relative Tumor Volume (RTV) was calculated from the measured tumor volume, wherein RTV=$V_t/V_0$, wherein $V_0$ was the tumor volume at random grouping (i.e., $d_0$) and $V_t$ was the tumor volume at each measurement (i.e., $d_n$). The relative tumor proliferation rate of the evaluation index of the anti-tumor activity was calculated according to the following formula: relative tumor proliferation rate T/C (%):

$$T/C\% = \frac{T_{RTV}}{C_{RTV}} \times 100\%$$

(Note: $T_{RTV}$: treatment group RTV; $C_{RTV}$: model control group RTV. According to the therapeutic effect evaluation standard in the Technical Guidelines for Non-clinical Research of Cytotoxic Anti-tumor Drugs Issued by National Medical Products Administration of China, the value (T/C%≤40%) was effective).

The effects of various tested samples on the tumor situation of transplanted tumor model of BaF3 EML-4-ALK-L1196M cells in nude mice were shown in Tables 10-11.

Table 10: Mean tumor volume ($mm^3$) of each group of transplanted tumor models of BaF3 EML-4-ALK-L1196M) in nude mice at different time points

| Test compounds | Tumor volume ($mm^3$) | | | | | | |
|---|---|---|---|---|---|---|---|
| | Day 0[a] | Day 2 | Day 5 | Day 7 | Day 9 | Day 12 | Day 14 |
| Solvent control | 128 | 228 | 485 | 906 | 1385 | 1884 | 2582 |
| Compound 2 (50 mg/kg) | 129 | 55 | 13 | 7 | 10 | 17 | 20 |
| Compound 6 (60 mg/kg) | 128 | 37 | 25 | 25 | 21 | 33 | 39 |
| Compound 14 (80 mg/kg) | 128 | 84 | 55 | 37 | 58 | 101 | 132 |
| Note: a. refers to days after administration. | | | | | | | |

Table 11: Relative tumor proliferation rate T/C (%) of transplanted tumor models of BaF3 EML-4-ALK-L1196M in nude mice

| Time / Test compounds | Day 2 T/C (%) | Day 5 T/C (%) | Day 7 T/C (%) | Day 9 T/C (%) | Day 12 T/C (%) | Day 14 T/C (%) |
|---|---|---|---|---|---|---|
| Compound 2 (50 mg/kg) | 24.19 | 2.61 | 0.74 | 0.68 | 0.89 | 0.91 |
| Compound 6 (60 mg/kg) | 16.51 | 5.28 | 3.01 | 1.59 | 1.88 | 1.99 |
| Compound 14 (80 mg/kg) | 35.71 | 11.26 | 3.99 | 3.95 | 5.24 | 6.34 |

The test results show that: in the test, compound 2 (50 mg/kg), compound 6 (60 mg/kg) and compound 14 (80 mg/kg) can all obviously inhibit the tumor growth of the transplanted tumors of BaF3 EML-4-ALK-L1196M in nude mice.

**Test Example 8: Single administration toxicity test study of orally intragastric administration of Compound 2 and Brigatinib to SD rats**

8-week-old SD rats, 10 per group, male and female half, were purchased from Beijing SiPeiFu. The rats were tested after 3 days of acclimatization in this laboratory. The oral administration was performed by intragastric administration with an administration volume of 10 mL/kg. The rats were fasted and not forbidden to drink water for 17 hours before administration. The rats in the control group were given an equal volume of solvent via oral gavage. The feeding was resumed about 2 hours after the end of the administration.

Observations were made twice daily after the administration. The day of first administration was defined as day 1 of the test. The test was carried out for 14 consecutive days, and ended in day 15.

Test doses and groups and the animal mortality at day 15 after the administration were shown in Table 12:

Table 12: Death caused by single administration toxicity test of orally intragastric administration of Compound 2 and Brigatinib to SD rats

| Test compounds | Treatment factor | Administration way | Dose (mg /kg) | Administration concentration(mg/mL) | Administration Volume (mL/kg) | Number of animals | | Mortality | | Mortality/total number of animals |
|---|---|---|---|---|---|---|---|---|---|---|
| | | | | | | Female | Male | Female | Male | |
| Solvent control | Solvent | intragastric administration | 0 | 0 | 10 | 5 | 5 | 0 | 0 | 0/10 |
| Dose 1 of compound 2 | Compound 2 | intragastric administration | 25 | 2.5 | 10 | 5 | 5 | 0 | 0 | 0/10 |
| Dose 2 of compound 2 | Compound 2 | intragastric administration | 75 | 7.5 | 10 | 5 | 5 | 0 | 0 | 0/10 |
| Dose 3 of compound 2 | Compound 2 | intragastric administration | 100 | 10 | 10 | 5 | 5 | 0 | 0 | 0/10 |
| Dose 4 of compound 2 | Compound 2 | intragastric administration | 125 | 12.5 | 10 | 5 | 5 | 0 | 0 | 0/10 |
| Dose 5 of compound 2 | Compound 2 | intragastric administration | 150 | 15 | 10 | 5 | 5 | 0 | 1 | 1/10 |
| Dose 6 of compound 2 | Compound 2 | intragastric administration | 200 | 20 | 10 | 5 | 5 | 0 | 0 | 0/10 |
| Dose 1 of Brigatinib | Brigatinib | intragastric administration | 25 | 2.5 | 10 | 5 | 5 | 0 | 0 | 0/10 |
| Dose 2 of Brigatinib | Brigatinib | intragastric administration | 75 | 7.5 | 10 | 5 | 5 | 1 | 1 | 2/10 |
| Dose 3 of Brigatinib | Brigatinib | intragastric administration | 100 | 10 | 10 | 5 | 5 | 3 | 1 | 4/10 |
| Dose 4 of Brigatinib | Brigatinib | intragastric administration | 125 | 12.5 | 10 | 5 | 5 | 4 | 2 | 6/10 |
| Dose 5 of Brigatinib | Brigatinib | intragastric administration | 150 | 15 | 10 | 5 | 5 | 5 | 4 | 9/10 |
| Dose 6 of Brigatinib | Brigatinib | intragastric administration | 200 | 20 | 10 | 5 | 5 | 5 | 5 | 10/10 |

The test results show that: in the single administration toxicity test of orally intragastric administration of compound 2 and Brigatinib to SD rats, the Maximum Tolerated Dose (MTD) of compound 2 was 125 mg/kg, while the MTD of Brigatinib was less than 75 mg/kg, indicating that under the conditions of this test, the single administration toxicity of compound 2 in the SD rats is far less than that of Brigatinib, and compound 2 has better safety.

**Test Example 9: In vivo pharmacodynamic study on subcutaneously implanted tumor models subcutaneously inoculated with NCI-H3122 and NCI-H1975 EGFR DTC (C797s/T790m/Del19) cells on both sides of BALB/c nude mouse respectively**

The models used in this test were subcutaneously implanted tumor models subcutaneously inoculated with NCI-H3122 and NCI-H1975 EGFR DTC (C797s/T790m/Del19) cells on the two sides of the same BALB/c nude mouse respectively (note: NCI-H3122 was an EML4-ALK fusion gene-positive cell line).

NCI-H3122 and NCI-H1975 EGFR DTC(C797S/T790M/Del19) were cultured in 5% $CO_2$ incubator at 37°C in a RPMI-compound 140 Medium with 10% fetal calf serum, 100 U/mL penicillin and 100 μg/mL streptomycin. Passages were treated twice a week. When the cell number reached requirement, the cells were collected, counted and inoculated.

0.2 mL ($5\times10^6$) NCI-H3122 cells (added with matrigel, 1:1 by volume) were subcutaneously inoculated into the left lower flank of each mouse and 0.2 mL ($5\times10^6$) NCI-H1975 EGFR DTC (C797S/T790M/Del19) cells (added with matrigel, 1:1 by volume) were subcutaneously inoculated into the right lower flank of each mouse. When the mean tumor volume of the NCI-H3122 subcutaneously implanted tumor reached about 116 $mm^3$ and the mean tumor volume of the NCI-H1975 EGFR DTC (C797S/T790M/Del19) subcutaneously implanted tumor reached about 110 $mm^3$, the mice were divided into groups for administration with 8 mice per group.

Administration mode and frequency: oral gavage was carried out with an administration volume of 10 mL/kg, and the model group was administered with the same volume of solvent. The administration was once per day and continuously carried out for 16 days. The doses of compound 2 were 20mg/kg and 40 mg/kg, and the dose of Crizotinib was 50 mg/kg.

General conditions such as spirit, activity, food intake and the like of the mice were observed every day, and the weight was measured thrice every week; the short diameter (a) and the long diameter (b) of each mouse tumor were measured thrice a week with a vernier caliper, and the tumor volume was calculated according to the formula $(a^2\times b)/2$. The Relative Tumor Volume (RTV) was calculated from the measured tumor volume, wherein $RTV=V_t/V_0$, wherein $V_0$ was the tumor volume at random grouping (i.e., $d_0$) and $V_t$ was the tumor volume at each measurement (i.e., $d_n$). The relative tumor proliferation rate of the evaluation index of the anti-tumor activity was calculated according to the following formula: relative tumor proliferation rate T/C (%):

$$T/C\ \% = \frac{T_{RTV}}{C_{RTV}}\times 100\%$$

Note: $T_{RTV}$: treatment group RTV; $C_{RTV}$ model control group RTV. According to the therapeutic effect evaluation standard in the Technical Guidelines for Non-clinical Research of Cytotoxic Anti-tumor Drugs Issued by National Medical Products Administration of China, the value (T/C%≤40%) was effective.

The effects of various tested samples on the tumor situation of the subcutaneously implanted tumor models subcutaneously inoculated with NCI-H3122 and NCI-H1975 EGFR DTC(C797S/T790M/Del19) cells on both sides of the same BALB/c nude mouse respectively were shown in Tables 13 and 14.

Table 13: Mean tumor volumes of subcutaneously implanted tumor model groups subcutaneously inoculated with NCI-H3122 and NCI-H1975 EGFR DTC(C797S/T790M/Del19) cells on both sides of the BALB/c nude mouse respectively at different time points

| Groups | NCI-H3122 Tumor volume ($mm^3$) | | | | | | | |
|---|---|---|---|---|---|---|---|---|
| | Day 0[a] | Day 2 | Day 4 | Day 6 | Day 9 | Day 11 | Day 13 | Day 16 |
| Solvent control | 116 | 141 | 187 | 244 | 376 | 468 | 577 | 750 |
| Compound 2 (20 mg/kg) | 116 | 115 | 92 | 84 | 74 | 63 | 58 | 61 |
| Compound 2 (40 mg/kg) | 116 | 101 | 54 | 47 | 33 | 34 | 32 | 28 |
| Crizotinib (50 mg/kg) | 116 | 139 | 162 | 196 | 268 | 302 | 312 | 372 |
| | NCI-H1975 EGFR DTC(C797S/T790M/Del19) Tumor volume ($mm^3$) | | | | | | | |
| Solvent control | 112 | 200 | 361 | 574 | 1098 | 1405 | 1748 | 2712 |

(continued)

| | NCI-H1975 EGFR DTC(C797S/T790M/Del19) Tumor volume ($mm^3$) | | | | | | | |
|---|---|---|---|---|---|---|---|---|
| Compound 2 (20 mg/kg) | 110 | 147 | 235 | 321 | 480 | 583 | 720 | 1065 |
| Compound 2 (40 mg/kg) | 113 | 133 | 177 | 217 | 269 | 308 | 327 | 385 |
| Crizotinib (50 mg/kg) | 110 | 162 | 310 | 522 | 929 | 1204 | 1462 | 2434 |
| Note: a. refers to days after administration. | | | | | | | | |

Table 14: Relative tumor proliferation rates T/C (%) of subcutaneously implanted tumor models subcutaneously inoculated with NCI-H3122 and NCI-H1975 EGFR DTC(C797S/T790M/Del19 cells on both sides of the BALB/c nude mouse respectively

| Time<br>Groups | Day 2<br>T/C (%) | Day 4<br>T/C (%) | Day 6<br>T/C (%) | Day 9<br>T/C (%) | Day 11<br>T/C (%) | Day 13<br>T/C (%) | Day 16<br>T/C (%) |
|---|---|---|---|---|---|---|---|
| | NCI-H3122 | | | | | | |
| Compound 2 (20 mg/kg) | 81.79 | 49.46 | 34.18 | 19.71 | 13.58 | 10.25 | 8.16 |
| Compound 2 (40 mg/kg) | 72.16 | 29.76 | 19.38 | 8.54 | 7.2 | 5.28 | 3.6 |
| Crizotinib (50 mg/kg) | 97.19 | 84.77 | 77.51 | 69.22 | 62.65 | 53.97 | 49.35 |
| | NCI-H1975 EGFR DTC(C797S/T790M/Del19) | | | | | | |
| Compound 2 (20 mg/kg) | 75.84 | 66.68 | 56.63 | 43.36 | 40.68 | 40.09 | 37.95 |
| Compound 2 (40 mg/kg) | 67.13 | 50.41 | 39.48 | 24.87 | 22.07 | 18.98 | 14.36 |
| Crizotinib (50 mg/kg) | 87.17 | 93.69 | 94.49 | 86.84 | 87.76 | 86.15 | 95.67 |

The test results show that: in this test, compound 2 can simultaneously and obviously inhibit the tumor growth of the subcutaneously implanted tumor of the NCI-H3122 and NCI-H1975 EGFR DTC(C797S/T790M/Del19) cells in the nude mice under the doses of 20 mg/kg and 40 mg/kg, and has a dose-effect relationship. After 16 days of administration, the T/C% of compound 2 (20 mg/kg) on the subcutaneously implanted tumor of NCI-H3122 and NCI-H1975 EGFR DTC(C797S/T790M/Del19) were 8.16% and 37.95% respectively, wherein T/C% was less than 40%, meeting the therapeutic effect evaluation standard in the Technical Guidelines for Non-clinical Research of Cytotoxic Anti-tumor Drugs Issued by National Medical Products Administration of China. The T/C% of compound 2 (40 mg/kg) on the subcutaneously implanted tumor of NCI-H3122 and NCI-H1975 EGFR DTC(C797S/T790M/Del19) were 3.6% and 14.36% respectively, has a stronger inhibitory effect on tumor growth and has a definite dose-effect relationship.

In this test, Crizotinib (50 mg/kg) did not significantly inhibit the tumor growth of the subcutaneously implanted tumor of NCI-H3122 and NCI-H1975 EGFR DTC(C797S/T790M/Del19) cells in nude mice. After 16 days of administration, the T/C% on the subcutaneously implanted tumor of NCI-H3122 and NCI-H1975 EGFR DTC(C797S/T790M/Del19) were 49.35% and 95.67% respectively, which did not reach the effective standard.

The above results indicate that compound 2 can simultaneously and obviously inhibit the tumor growth of the subcutaneously implanted tumor of NCI-H3122 and NCI-H1975 EGFR DTC(C797S/T790M/Del19) cells in nude mice, and has a clear dose-effect relationship.

**Test Example 10 Cell anti-proliferation test 4**

1. Test purpose: to detect the effect of the test compounds on the proliferation of cell lines containing ALK mutations in BaF3 cells
2. Test materials

    BaF3 EML-4-ALK-L1196M cell line, brand: KYinno, article number: KC-0102
    Cell Counting Kit-8 (CCK-8), brand: Targetmol, article number: C0005
    Multifunctional microplate reader, POLARstar Omega, brand: BMG LABTECH

3. Test method

Day 0: Cells were seeded in a plate.

a. The cells were removed from an incubator, blown to a uniform cell suspension in a clean bench and counted.
b. Based on the cell counting results, the cell suspension was adjusted to a density of 8,000 cells per well and 110 μL per well, and plated in a 96-well plate.
c. 10 μL of the compound diluted in gradient was added to the cell plate to give final concentrations of the compounds as 500, 166.67, 55.56, 18.52, 6.17, 2.06, 0.69, 0.23 and 0.08 nM.
d. The cell plate was incubated in a 5% $CO_2$ incubator at 37°C for 72 hours.

Day 3: After placing CCK8 at room temperature for equilibrium, 10 μL of CCK8 was added to each well, the cell plate was incubated in a 5% $CO_2$ incubator at 37°C for 2 hours, and then a signal value $OD_{450}$ was detected in the multifunctional microplate reader POLARstar Omega. The $IC_{50}$ of each compound was calculated by computer fitting with GraphPad prism analysis software (the $IC_{50}$ of the compound on the cell activity inhibition could be obtained by reading the concentrations of the corresponding compounds at 50% inhibition rate). Inhibition rate% = (reading value of the control group without drug - reading value of the sample)/(reading value of the control group without drug - reading value of the control group with medium only) ×100.

4. Test results: the $IC_{50}$ values of the compounds according to the examples of the present invention and the compounds according to the comparative examples on the BaF3 cells expressing EML-4-ALK- L1196M were shown in Table 15.

Conclusion: as can be seen from Table 15, the compounds according to the examples of the present invention have a very good inhibitory effect on the proliferation of BaF3 cells expressing EML-4-ALK-L1196M, and have inhibitory activity superior to that of the control compound 4.

Table 15: Inhibitory effects of compound 2 and control example 4 on BaF3 EML-4-ALK-L1196M cells

| Compound | First test $IC_{50}$ (nM) | Second test $IC_{50}$ (nM) |
|---|---|---|
| Compound 2 | 15.14 | 10.38 |
| Control compound 4 | 81.45 | 74.61 |

**Test Example 11 Pharmacokinetic test**

Six SD rats were taken, which were all male, and about 8-9 weeks old, weighing about 430 g/rat, and fasting but allowed to drink water for 18 hours.

The rats were divided into two groups, wherein the rats in group 1 were dosed with compound 2 and the rats in group 2 were dosed with control compound 4, both orally and intragastrically in a dose of 20 mg/kg. A lavage fluid was prepared using 70 mM citric acid buffer (pH 3.0) at an administration volume of 5 mL/kg.

0.6 mL of blood (EDTA-2K anti-coagulated) was collected from rat orbits at 0.5, 1.5, 3, 4.5, 8, 12, 24, 36, 48 and 72 hours after the administration respectively. The plasma was separated and stored at-70°C, and the plasma samples were analyzed using liquid chromatography tandem mass spectrometry (LC-MS/MS). The plasma concentration-time data of the individual rates was analyzed using a non-compartmental model of DAS3.3.1 software and pharmacokinetic parameters of the test compounds were calculated. The pharmacokinetic properties of the compounds in the rats were shown in Table 16.

Table 16: Pharmacokinetic properties of compounds

| Compound | Number of animals | Administration mode | Dose (mg/kg) | $C_{max}$ (ng/mL) | $AUC_{0-t}$ (h*ng/mL) |
|---|---|---|---|---|---|
| Compound 2 | 3 | PO | 20 | 2313.33 | 96251.17 |
| Control compound 4 | 3 | PO | 20 | 1423.67 | 58485.75 |

As can be seen from Table 16, the systemic exposure of compound 2 was higher than that of control compound 4 at the same dose given by a single oral dose in the rats.

## Claims

1. A compound represented by formula (I) or a pharmaceutically acceptable salt thereof:

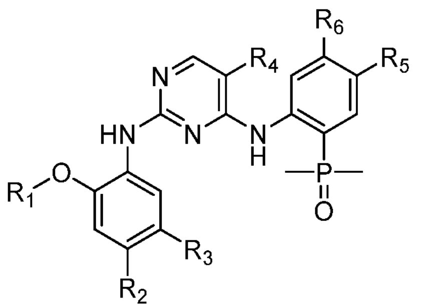

formula (I),

wherein $R_1$ is selected from $C_1$-$C_6$ alkyl and $C_3$-$C_6$ cycloalkyl, the $C_1$-$C_6$ alkyl and the $C_3$-$C_6$ cycloalkyl are optionally substituted by zero to six R';
$R_2$ is selected from hydrogen, amino, $C_1$-$C_6$ alkyl, $C_3$-$C_6$ cycloalkyl, $C_3$-$C_6$ cycloalkenyl, phenyl, 5-6 membered heteroaryl,

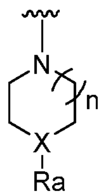

or

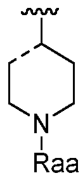

;

and the amino, the $C_1$-$C_6$ alkyl, the $C_3$-$C_6$ cycloalkyl, the phenyl and the 5-6 membered heteroaryl are optionally substituted by zero to three $R_a$ groups;
X is selected from CH, S, N or O;
n is 0, 1 or 2;
when X is O, $R_a$ is not contained;
the bond - - - - represents C-C saturated bond or C=C olefinic bond;
$R_3$ is selected from $C_1$-$C_6$ alkyl, $C_3$-$C_6$ cycloalkyl and halogen; and the $C_1$-$C_6$ alkyl and the $C_3$-$C_6$ cycloalkyl are optionally substituted by zero to three R';
$R_4$ and $R_6$ are each independently selected from hydrogen, $C_1$-$C_6$ alkyl, $C_3$-$C_6$ cycloalkyl and halogen, and the $C_1$-$C_6$ alkyl and the $C_3$-$C_6$ cycloalkyl are optionally substituted by zero to three R';
$R_5$ is selected from $C_1$-$C_6$ alkyl and halogen, and the $C_1$-$C_6$ alkyl is optionally substituted by zero to three R';
$R_a$ and $R_{aa}$ are each independently selected from hydrogen, $C_1$-$C_6$ alkyl, $C_3$-$C_6$ cycloalkyl, phenyl, 5-6 membered heteroaryl, halogen, amino, hydroxyl, cyano, nitro, $-NR_bC(O)R_c$, $-NR_bR_c$ and

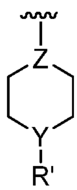

;

wherein the $C_1$-$C_6$ alkyl, the $C_3$-$C_6$ cycloalkyl, the phenyl, the 5-6 membered heteroaryl, the amino and the hydroxyl are optionally substituted by zero to three R';
Y is selected from N or O, and Z is selected from CH or N;
$R_b$ and $R_c$ are each independently selected from hydrogen, $C_1$-$C_6$ alkyl, $C_3$-$C_6$ cycloalkyl, $C_2$-$C_6$ alkenyl, $C_2$-$C_6$ alkynyl, hydroxyl and amino; and the $C_1$-$C_6$ alkyl, the $C_3$-$C_6$ cycloalkyl, the $C_2$-$C_6$ alkenyl and the $C_2$-$C_6$ alkynyl are optionally substituted by zero to three R'; and
R' is selected from hydrogen, F, Cl, Br, I, hydroxyl, acyl, carboxyl, amino, nitro, cyano, $C_1$-$C_6$ alkyl, $C_3$-$C_6$ cycloalkyl, $C_2$-$C_6$ alkenyl and $C_2$-$C_6$ alkynyl; and the $C_1$-$C_6$ alkyl, the $C_3$-$C_6$ cycloalkyl, the $C_2$-$C_6$ alkenyl, the $C_2$-$C_6$ alkynyl, the amino and the hydroxyl are optionally substituted by zero to three hydrogen, hydroxyl, carboxyl,

carbonyl, F, Cl, Br, I, amino, nitro, cyano, methyl, trifluoroethyl, difluoromethyl and monofluoromethyl.

**2.** The compound or the pharmaceutically acceptable salt thereof according to claim 1, wherein:

$R_1$ is selected from $C_1$-$C_6$ alkyl; and the $C_1$-$C_6$ alkyl is optionally substituted by zero to three R';
$R_2$ is selected from hydrogen, $C_1$-$C_6$ alkyl, amino,

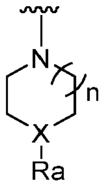 or 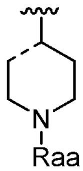;

wherein, the amino and the $C_1$-$C_6$ alkyl are substituted by one to three $R_a$;
$R_a$ and $R_{aa}$ are each independently selected from hydrogen, $NR_bR_c$, $C_1$-$C_6$ alkyl or

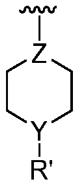;

$R_3$ is selected from $C_1$-$C_6$ alkyl, $C_3$-$C_6$ cycloalkyl and halogen; and the $C_1$-$C_6$ alkyl and the $C_3$-$C_6$ cycloalkyl are optionally substituted by zero to three R';
$R_4$ is selected from halogen, $C_1$-$C_6$ alkyl and $C_3$-$C_6$ cycloalkyl; and the $C_1$-$C_6$ alkyl and the $C_3$-$C_6$ cycloalkyl are optionally substituted by zero to three R';
$R_5$ is selected from $C_1$-$C_6$ alkyl and halogen; and the $C_1$-$C_6$ alkyl is optionally substituted by zero to three R';
$R_6$ is selected from hydrogen or methyl; and
$R_b$ and $R_c$ are each independently selected from hydrogen, $C_1$-$C_6$ alkyl and $C_2$-$C_6$ alkenyl; and the $C_1$-$C_6$ alkyl and the $C_2$-$C_6$ alkenyl are optionally substituted by zero to three R'.

**3.** The compound or the pharmaceutically acceptable salt thereof according to claim 2, wherein:

$R_3$ is selected from $C_1$-$C_6$ alkyl and $C_3$-$C_6$ cycloalkyl; and the $C_1$-$C_6$ alkyl and the $C_3$-$C_6$ cycloalkyl are optionally substituted by zero to three R';
$R_4$ is selected from halogen and $C_1$-$C_6$ alkyl; wherein, the $C_1$-$C_6$ alkyl is optionally substituted by zero to three R'
$R_5$ is $C_1$-$C_6$ alkyl; wherein, the $C_1$-$C_6$ alkyl is optionally substituted by zero to three R'; and
$R_6$ is hydrogen.

**4.** The compound or the pharmaceutically acceptable salt thereof according to claim 3, wherein:

$R_2$ is selected from amino and

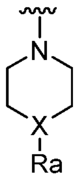;

and the amino is optionally substituted by zero to three $R_a$; wherein, X is selected from CH, N or O;
$R_a$ is selected from

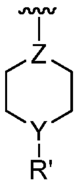,

$C_1$-$C_3$ alkyl or $NR_bR_c$;
wherein, Y is selected from N or O, and Z is selected from CH or N;
$R_b$ and $R_c$ are each independently selected from hydrogen and $C_1$-$C_3$ alkyl;
$R_3$ is selected from $C_1$-$C_6$ alkyl; and the $C_1$-$C_6$ alkyl is optionally substituted by zero to three R';
$R_4$ is selected from Cl, Br and $C_1$-$C_6$ alkyl, and the $C_1$-$C_6$ alkyl is optionally substituted by zero to three R';
$R_5$ is selected from $C_1$-$C_6$ alkyl, and the $C_1$-$C_6$ alkyl is optionally substituted by zero to three R'; and
R' is selected from hydrogen, $C_1$-$C_6$ alkyl, F, Cl, Br, I, hydroxyl and amino;
preferably,
$R_1$ is selected from methyl, ethyl, isopropyl, $CF_2H$ and $CH_2CF_3$;
$R_2$ is selected from

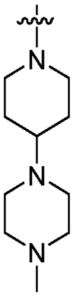, 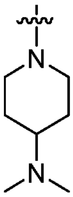, 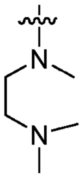, 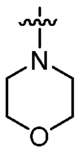, 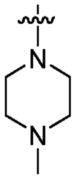 and 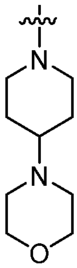;

$R_3$ is selected from methyl, ethyl and isopropyl;
$R_4$ is selected from Cl, Br, $CH_3$, $CF_3$ and $CH_2CF_3$; and
$R_5$ is selected from methyl, ethyl and isopropyl;
more preferably,
$R_1$ is selected from methyl, ethyl, isopropyl and $-CF_2H$;
$R_2$ is selected from

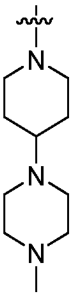, 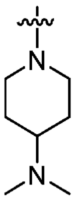, 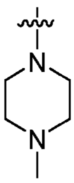 and 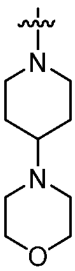;

$R_3$ is selected from methyl, ethyl and isopropyl;
$R_4$ is selected from Br; and
$R_5$ is selected from methyl, ethyl and isopropyl.

**5.** The compound or the pharmaceutically acceptable salt thereof according to claim 3, wherein:

$R_2$ is selected from amino and

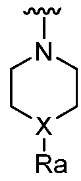

;

and the amino is optionally substituted by zero to three $R_a$; wherein, X is selected from CH, N or O;
$R_a$ is selected from

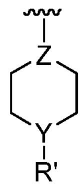

,

$C_1$-$C_3$ alkyl or $NR_bR_c$;
wherein, Y is selected from N or O, and Z is selected from CH or N;
$R_b$ and $R_c$ are each independently selected from hydrogen and $C_1$-$C_3$ alkyl;
$R_3$ is selected from $C_1$-$C_6$ alkyl; and the $C_1$-$C_6$ alkyl is optionally substituted by zero to three R';
$R_4$ is selected from Cl, Br and $C_1$-$C_6$ alkyl, and the $C_1$-$C_6$ alkyl is optionally substituted by zero to three R';
$R_5$ is selected from $C_1$-$C_6$ alkyl, and the $C_1$-$C_6$ alkyl is optionally substituted by zero to three R'; and
R' is selected from F, Cl, Br, I, hydroxyl and amino;
preferably,
$R_1$ is selected from methyl, ethyl, isopropyl, $CF_2H$ and $CH_2CF_3$;
$R_2$ is selected from

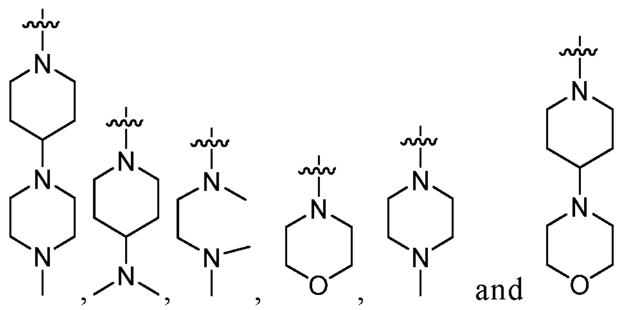

, , , , and ;

$R_3$ is selected from methyl and ethyl;
$R_4$ is selected from Cl, Br, $CF_3$ and $CH_2CF_3$; and
$R_5$ is selected from methyl and ethyl.

**6.** The compound or the pharmaceutically acceptable salt thereof according to claim 2, wherein:

$R_3$ is halogen;
$R_4$ is selected from halogen and $C_1$-$C_6$ alkyl; wherein, the $C_1$-$C_6$ alkyl is optionally substituted by zero to three R';
$R_5$ is selected from $C_1$-$C_6$ alkyl; and the $C_1$-$C_6$ alkyl is optionally substituted by zero to three R';
$R_6$ is hydrogen; and
R' is selected from hydrogen, $C_1$-$C_6$ alkyl, F, Cl, Br, I, hydroxyl and amino;
preferably,
$R_1$ is selected from methyl, ethyl and isopropyl;
$R_2$ is selected from

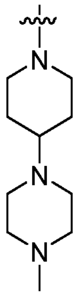, 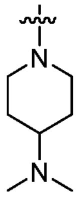, 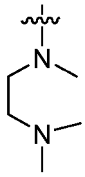, 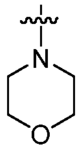, 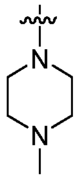 and 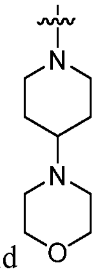;

$R_3$ is selected from Cl and Br;
$R_4$ is selected from Cl, Br, $CF_3$ and $CH_2CF_3$; and
$R_5$ is selected from methyl, ethyl and isopropyl.

**7.** The compound or the pharmaceutically acceptable salt thereof according to claim 2, wherein:

$R_3$ is halogen;
$R_4$ is selected from halogen and $C_1$-$C_6$ alkyl; wherein the $C_1$-$C_6$ alkyl is optionally substituted by zero to three R';
$R_5$ is selected from $C_1$-$C_6$ alkyl; and the $C_1$-$C_6$ alkyl is optionally substituted by zero to three R';
$R_6$ is hydrogen; and
R' is selected from F, Cl, Br, I, hydroxyl and amino;
preferably,
$R_1$ is selected from methyl, ethyl and isopropyl;
$R_2$ is selected from

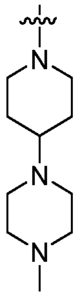, 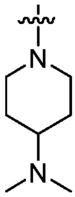, 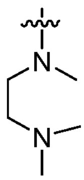, 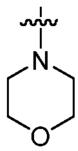, 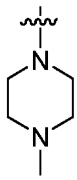 and 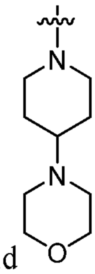;

$R_3$ is selected from Cl and Br;
$R_4$ is selected from Cl, Br, $CF_3$ and $CH_2CF_3$; and
$R_5$ is selected from methyl and ethyl.

**8.** The compound or the pharmaceutically acceptable salt thereof according to claim 2, wherein:

$R_3$ is selected from $C_1$-$C_6$ alkyl; and the $C_1$-$C_6$ alkyl is optionally substituted by zero to three R';
$R_4$ is selected from halogen and $C_1$-$C_6$ alkyl; wherein, the $C_1$-$C_6$ alkyl is optionally substituted by zero to three R';
$R_5$ is selected from halogen;
$R_6$ is hydrogen; and
R' is selected from hydrogen, $C_1$-$C_6$ alkyl, F, Cl, Br, I, hydroxyl and amino;
preferably,
$R_1$ is selected from methyl, ethyl and isopropyl;
$R_2$ is selected from

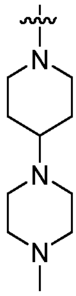, 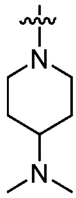, 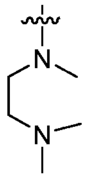, 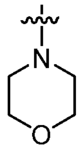, 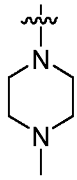 and 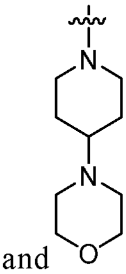;

$R_3$ is selected from methyl, ethyl and isopropyl;
$R_4$ is selected from Cl, Br, $CF_3$ and $CH_2CF_3$; and
$R_5$ is selected from F and Cl;
more preferably,
$R_1$ is selected from methyl, ethyl and isopropyl;
$R_2$ is selected from

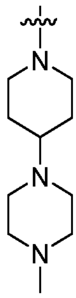, 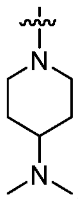, 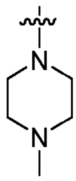 and 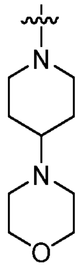;

$R_3$ is selected from methyl, ethyl and isopropyl;
$R_4$ is selected from Br; and
$R_5$ is selected from F and Cl.

**9.** The compound or the pharmaceutically acceptable salt thereof according to claim 2, wherein:

$R_3$ is selected from $C_1$-$C_6$ alkyl; and the $C_1$-$C_6$ alkyl is optionally substituted by zero to three R';
$R_4$ is selected from halogen and $C_1$-$C_6$ alkyl; wherein, the $C_1$-$C_6$ alkyl is optionally substituted by zero to three R';
$R_5$ is selected from halogen;
$R_6$ is hydrogen; and
R' is selected from F, Cl, Br, I, hydroxyl and amino;
preferably,
$R_1$ is selected from methyl, ethyl and isopropyl;
$R_2$ is selected from

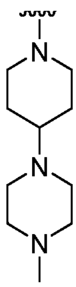, 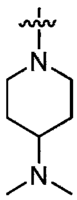, 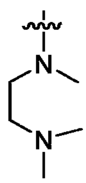, 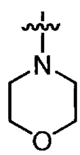, 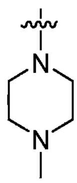 and 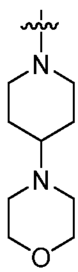;

$R_3$ is selected from methyl and ethyl;
$R_4$ is selected from Cl, Br, $CF_3$ and $CH_2CF_3$; and
$R_5$ is selected from F and Cl.

**10.** A compound or a pharmaceutically acceptable salt thereof according to claim 1, selected from:

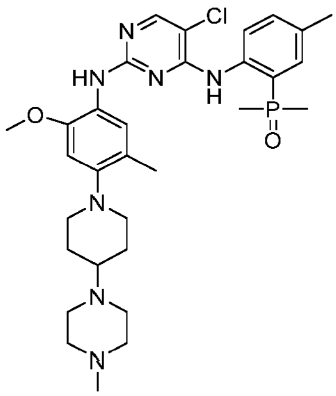

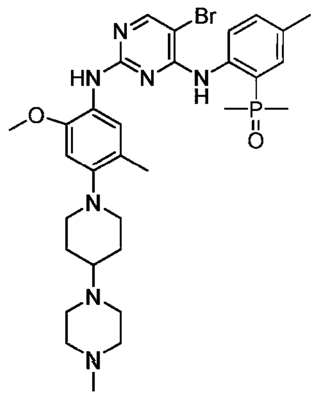

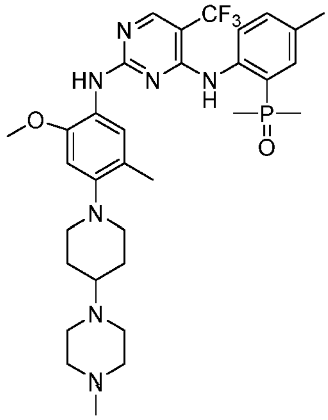

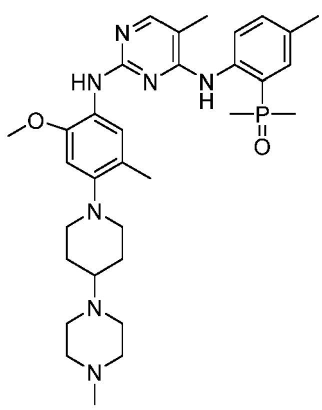

,

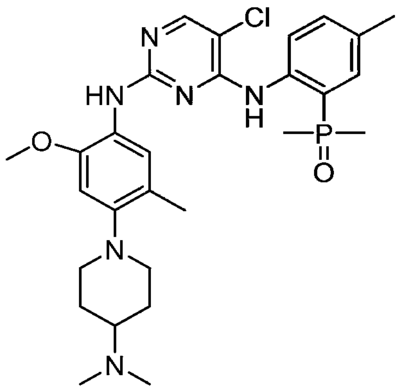

,

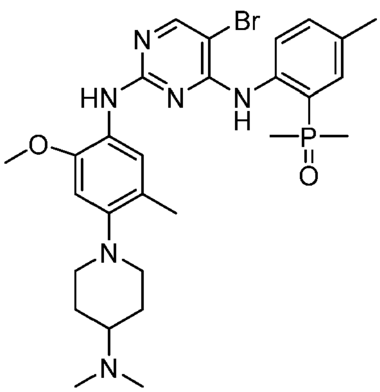

,

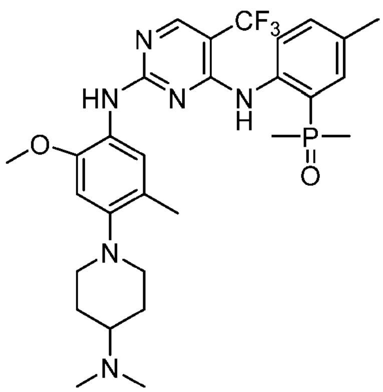

,

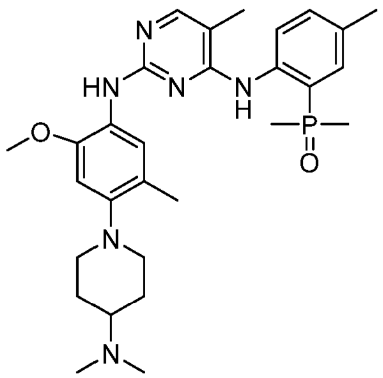,
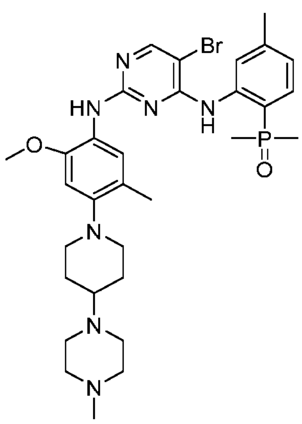,
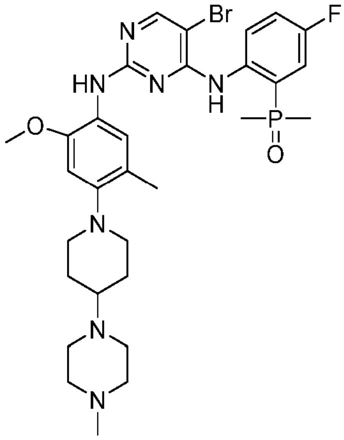,
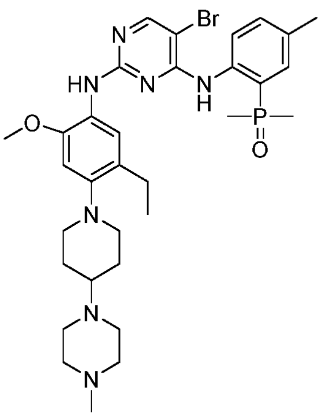,
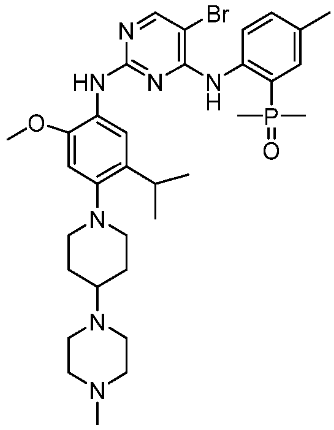,
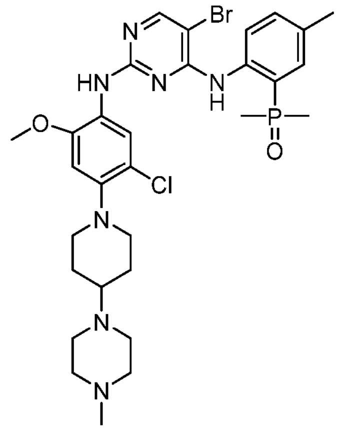,
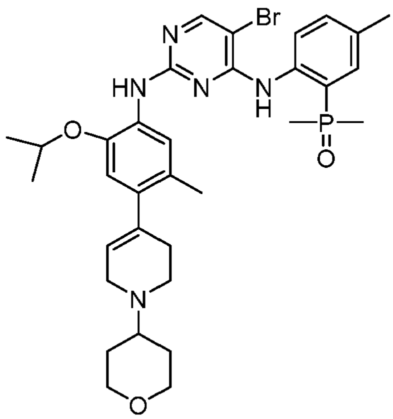,
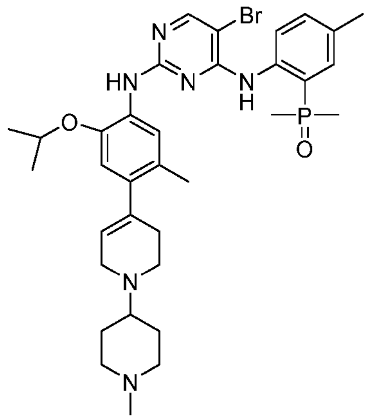,

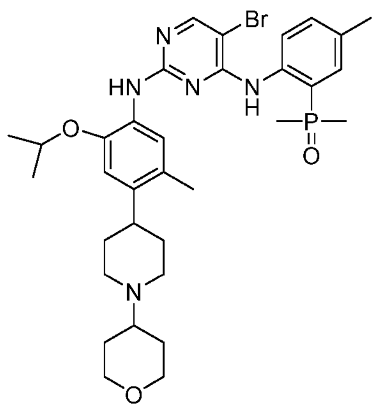
,
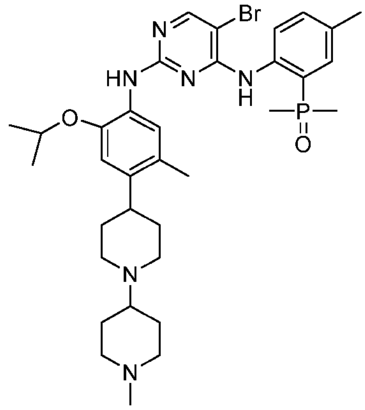
,

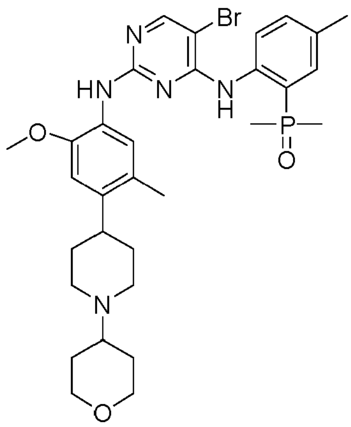
,
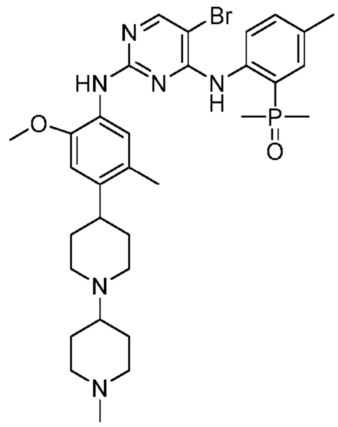
,
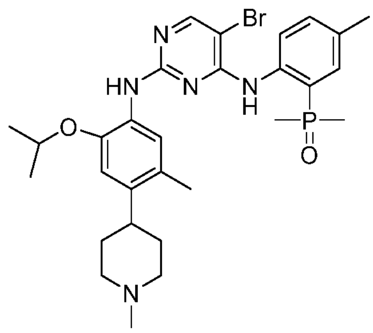
,

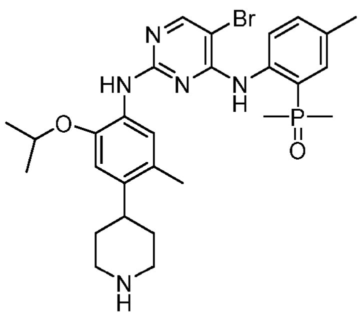
,
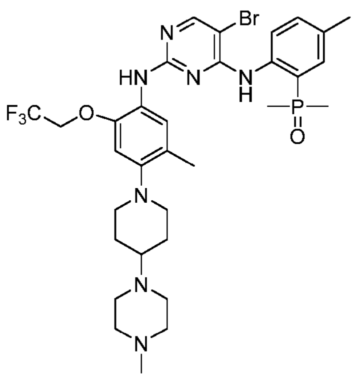
,
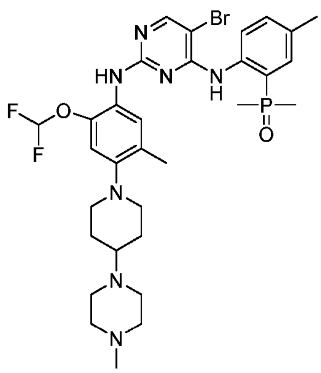
, , , ,

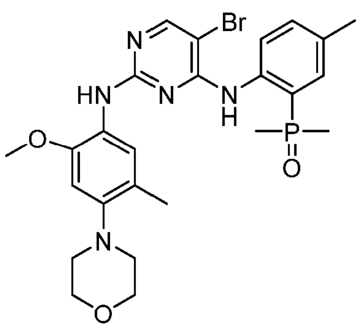
,
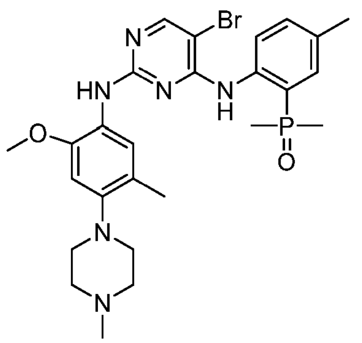
,
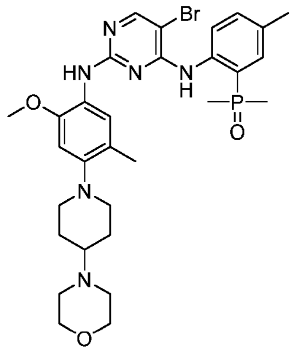
,

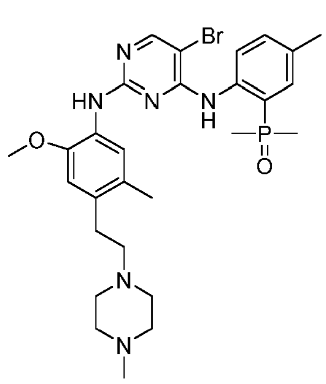 , 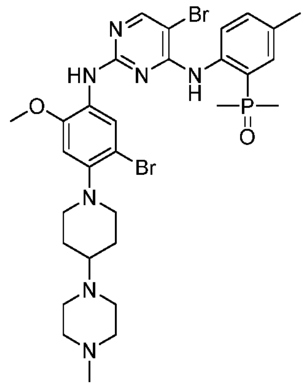 , 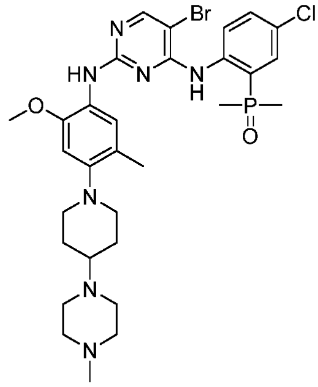 ,

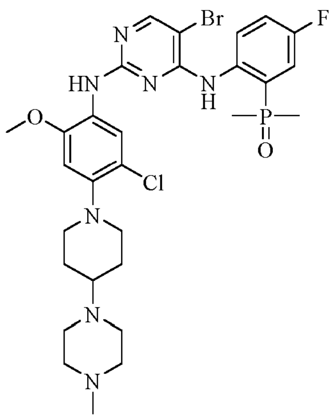 , 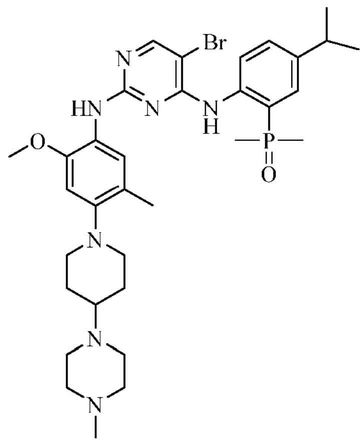 , 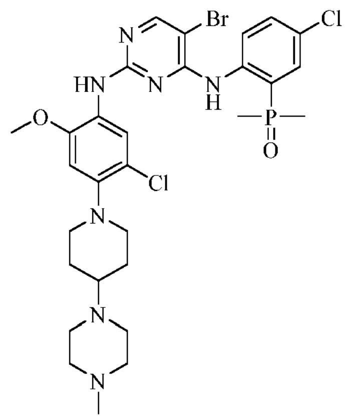

and

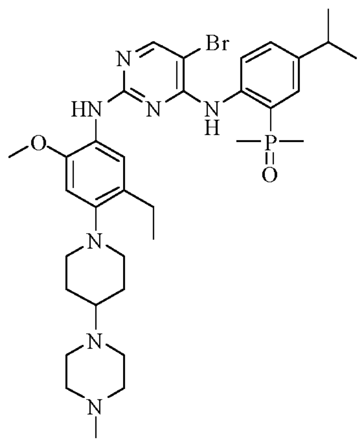 .

11. A pharmaceutical composition, comprising the compound or the pharmaceutically acceptable salt thereof according to any one of claims 1 to 10, and a pharmaceutically acceptable carrier.

12. The pharmaceutical composition of claim 11, wherein the pharmaceutical composition comprise other anti-cancer drug or anti-tumor drug; preferably, the anti-cancer drug or anti-tumor drug being one or more of cytotoxic drug, hormone drug, antimetabolite, tumor-targeted drug, PARP inhibitor, adjuvant therapy drug or anti-tumor biological drug; more preferably, the cytotoxic drug being one or more of carboplatin, cisplatin, irinotecan, paclitaxel, fluorouracil, cytarabine, lenalidomide and retinoic acid; the hormone drug being one or more of dexamethasone, fulvestrant and tamoxifen; the antimetabolite being one or more of fluorouracil, methotrexate, furan fluorouracil and cytarabine; the tumor-targeted drug being one or more of imatinib, erlotinib and lapatinib; the PARP inhibitor being one or more of Olaparib, Rubraca and Zejula; the adjuvant therapy drug being one or more of recombinant human granulocyte colony

stimulating factor, erythropoietin, pamidronate disodium and zoledronic acid; and the anti-tumor biological drug being one or more of Keytruda, Opdiv, Tecentriq, Imfinzi and Bavencio.

**13.** The compound or the pharmaceutically acceptable salt thereof according to any one of claims 1 to 10 or the pharmaceutical composition according to claim 11 or 12 for use as a medicament.

**14.** The compound or the pharmaceutically acceptable salt thereof according to any one of claims 1 to 10, or the pharmaceutical composition according to claim 11 or 12 for use in the treatment of cancer; preferably, the cancer is plasmoma, mantle cell tumor, multiple myeloma, melanoma, breast cancer, liver cancer, cervical cancer, lung cancer, lymphoma, leukemia, ovarian cancer, kidney cancer, gastric cancer, nasopharyngeal cancer, thyroid cancer, pancreatic cancer, prostate cancer, adenocarcinoma, oral cancer, esophageal cancer, squamous cell carcinoma or colon cancer.

**15.** The compound or the pharmaceutically acceptable salt thereof according to any one of claims 1 to 10, or the pharmaceutical composition according to claim 11 or 12 for use as an EGFR inhibitor, or an ALK inhibitor, or an EGFR and ALK inhibitor, or a protein kinase inhibitor,
preferably, the EGFR inhibitor, or the ALK inhibitor, or the EGFR and ALK inhibitor, or the protein kinase inhibitor is applied to plasmoma, mantle cell tumor, multiple myeloma, melanoma, breast cancer, liver cancer, cervical cancer, lung cancer, lymphoma, leukemia, ovarian cancer, kidney cancer, gastric cancer, nasopharyngeal cancer, thyroid cancer, pancreatic cancer, prostate cancer, adenocarcinoma, oral cancer, esophageal cancer, squamous cell carcinoma or colon cancer.

## Patentansprüche

**1.** Verbindung der Formel (I) oder ein pharmazeutisch akzeptables Salz davon:

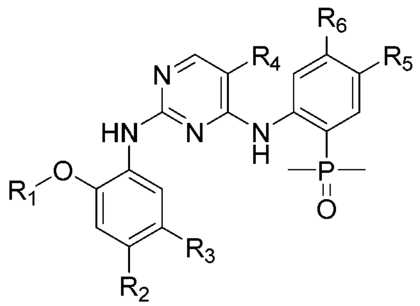

Formel (I),

wobei $R_1$ ausgewählt ist aus $C_1$-$C_6$-Alkyl und $C_3$-$C_6$-Cycloalkyl, wobei das $C_1$-$C_6$-Alkyl und das $C_3$-$C_6$-Cycloalkyl optional durch null bis sechs R' substituiert sind;
$R_2$ ausgewählt ist aus Wasserstoff, Amino, $C_1$-$C_6$-Alkyl, $C_3$-$C_6$-Cycloalkyl, $C_3$-$C_6$-Cycloalkenyl, Phenyl, 5- bis 6-gliedrigem Heteroaryl,

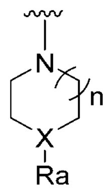

oder

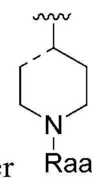

;

und das Amino, das $C_1$-$C_6$-Alkyl, das $C_3$-$C_6$-Cycloalkyl, das Phenyl und das 5- bis 6-gliedrige Heteroaryl optional durch null bis drei $R_a$-Gruppen substituiert sind;
X ausgewählt ist aus CH, S, N oder O;
n ist 0, 1 oder 2;
wenn X O ist, ist $R_a$ nicht enthalten;
die Bindung - - - - eine gesättigte C-C-Bindung oder eine olefinische C=C-Bindung darstellt;
$R_3$ ausgewählt ist aus $C_1$-$C_6$-Alkyl, $C_3$-$C_6$-Cycloalkyl und Halogen; und das $C_1$-$C_6$-Alkyl und das $C_3$-$C_6$-Cyc-

loalkyl optional durch null bis drei R' substituiert sind;
$R_4$ und $R_6$ jeweils unabhängig voneinander ausgewählt sind aus Wasserstoff, $C_1$-$C_6$-Alkyl, $C_3$-$C_6$-Cycloalkyl und Halogen, und das $C_1$-$C_6$-Alkyl und das $C_3$-$C_6$-Cycloalkyl optional durch null bis drei R' substituiert sind;
$R_5$ ausgewählt ist aus $C_1$-$C_6$-Alkyl und Halogen, wobei das $C_1$-$C_6$-Alkyl optional durch null bis drei R' substituiert ist;
$R_a$ und $R_{aa}$ jeweils unabhängig voneinander ausgewählt sind aus Wasserstoff, $C_1$-$C_6$-Alkyl, $C_3$-$C_6$-Cycloalkyl, Phenyl, 5- bis 6-gliedrigem Heteroaryl, Halogen, Amino, Hydroxyl, Cyano, Nitro, -$NR_bC(O)R_c$, -$NR_bR_c$ und

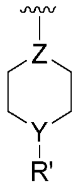 ;

wobei das $C_1$-$C_6$-Alkyl, das $C_3$-$C_6$-Cycloalkyl, das Phenyl, das 5- bis 6-gliedrige Heteroaryl, das Amino und das Hydroxyl optional durch null bis drei R' substituiert sind;
Y ausgewählt ist aus N oder O und Z ausgewählt ist aus CH oder N;
$R_b$ und $R_c$ jeweils unabhängig voneinander ausgewählt sind aus Wasserstoff, $C_1$-$C_6$-Alkyl, $C_3$-$C_6$-Cycloalkyl, $C_2$-$C_6$-Alkenyl, $C_2$-$C_6$-Alkinyl, Hydroxyl und Amino; und das $C_1$-$C_6$-Alkyl, das $C_3$-$C_6$-Cycloalkyl, das $C_2$-$C_6$-Alkenyl und das $C_2$-$C_6$-Alkinyl optional durch null bis drei R' substituiert sind; und
R' ausgewählt ist aus Wasserstoff, F, Cl, Br, I, Hydroxyl, Acyl, Carboxyl, Amino, Nitro, Cyano, $C_1$-$C_6$-Alkyl, $C_3$-$C_6$-Cycloalkyl, $C_2$-$C_6$-Alkenyl und $C_2$-$C_6$-Alkinyl; und das $C_1$-$C_6$-Alkyl, das $C_3$-$C_6$-Cycloalkyl, das $C_2$-$C_6$-Alkenyl, das $C_2$-$C_6$-Alkinyl, das Amino und das Hydroxyl optional durch null bis drei Wasserstoffatome, Hydroxyl, Carboxyl, Carbonyl, F, Cl, Br, I, Amino, Nitro, Cyano, Methyl, Trifluorethyl, Difluormethyl und Monofluormethyl substituiert sind.

**2.** Verbindung oder ein pharmazeutisch akzeptables Salz davon nach Anspruch 1, wobei:

$R_1$ ausgewählt ist aus $C_1$-$C_6$-Alkyl; und das $C_1$-$C_6$-Alkyl optional durch null bis drei R' substituiert ist;
$R_2$ ausgewählt ist aus Wasserstoff, $C_1$-$C_6$-Alkyl, Amino,

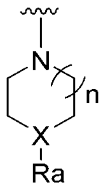 oder 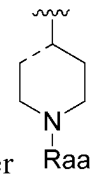 ;

wobei das Amino und das $C_1$-$C_6$-Alkyl durch ein bis drei $R_a$ substituiert sind;
$R_a$ und $R_{aa}$ jeweils unabhängig voneinander ausgewählt sind aus Wasserstoff, $NR_bR_c$, $C_1$-$C_6$-Alkyl oder

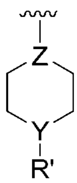 ;

$R_3$ ausgewählt ist aus $C_1$-$C_6$-Alkyl, $C_3$-$C_6$-Cycloalkyl und Halogen; und das $C_1$-$C_6$-Alkyl und das $C_3$-$C_6$-Cycloalkyl optional durch null bis drei R' substituiert sind;
$R_4$ ausgewählt ist aus Halogen, $C_1$-$C_6$-Alkyl und $C_3$-$C_6$-Cycloalkyl; und das $C_1$-$C_6$-Alkyl und das $C_3$-$C_6$-Cycloalkyl optional durch null bis drei R' substituiert sind;
$R_5$ ausgewählt ist aus $C_1$-$C_6$-Alkyl und Halogen; und das $C_1$-$C_6$-Alkyl optional durch null bis drei R' substituiert ist;
$R_6$ ausgewählt ist aus Wasserstoff oder Methyl; und
$R_b$ und $R_c$ jeweils unabhängig voneinander ausgewählt sind aus Wasserstoff, $C_1$-$C_6$-Alkyl und $C_2$-$C_6$-Alkenyl; und das $C_1$-$C_6$-Alkyl und das $C_2$-$C_6$-Alkenyl optional durch null bis drei R' substituiert sind.

**3.** Verbindung oder ein pharmazeutisch akzeptables Salz davon nach Anspruch 2, wobei:

$R_3$ ausgewählt ist aus $C_1$-$C_6$-Alkyl und $C_3$-$C_6$-Cycloalkyl; und das $C_1$-$C_6$-Alkyl und das $C_3$-$C_6$-Cycloalkyl optional durch null bis drei R' substituiert sind;
$R_4$ ausgewählt ist aus Halogen und $C_1$-$C_6$-Alkyl; wobei das $C_1$-$C_6$-Alkyl optional durch null bis drei R' substituiert ist;
$R_5$ $C_1$-$C_6$-Alkyl ist; wobei das $C_1$-$C_6$-Alkyl optional durch null bis drei R' substituiert ist; und
$R_6$ Wasserstoff ist.

**4.** Verbindung oder ein pharmazeutisch akzeptables Salz davon nach Anspruch 3, wobei:

$R_2$ ausgewählt ist aus Amino und

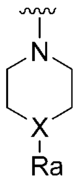 ;

und das Amino optional durch null bis drei $R_a$ substituiert ist; wobei X ausgewählt ist aus CH, N oder O;
$R_a$ ausgewählt ist aus

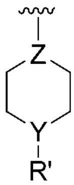 ,

$C_1$-$C_3$-Alkyl oder $NR_bR_c$;
wobei Y ausgewählt ist aus N oder O und Z ausgewählt ist aus CH oder N;
$R_b$ und $R_c$ jeweils unabhängig voneinander ausgewählt sind aus Wasserstoff und $C_1$-$C_3$-Alkyl;
$R_3$ ausgewählt ist aus $C_1$-$C_6$-Alkyl; und das $C_1$-$C_6$-Alkyl optional durch null bis drei R' substituiert ist;
$R_4$ ausgewählt ist aus Cl, Br und $C_1$-$C_6$-Alkyl, und das $C_1$-$C_6$-Alkyl optional durch null bis drei R' substituiert ist;
$R_5$ ausgewählt ist aus $C_1$-$C_6$-Alkyl, und das $C_1$-$C_6$-Alkyl optional durch null bis drei R' substituiert ist; und
R' ausgewählt ist aus Wasserstoff, $C_1$-$C_6$-Alkyl, F, Cl, Br, I, Hydroxyl und Amino;
vorzugsweise
$R_1$ ausgewählt ist aus Methyl, Ethyl, Isopropyl, $CF_2H$ und $CH_2CF_3$;
$R_2$ ausgewählt ist aus

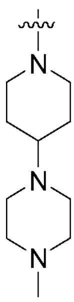 , 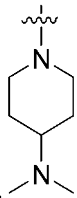 , 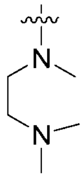 , 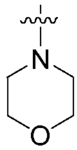 , 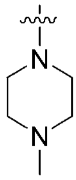 und 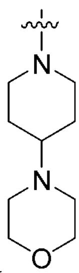 ;

$R_3$ ausgewählt ist aus Methyl, Ethyl und Isopropyl;
$R_4$ ausgewählt ist aus Cl, Br, $CH_3$, $CF_3$ und $CH_2CF_3$; und
$R_5$ ausgewählt ist aus Methyl, Ethyl und Isopropyl;
besonders vorzugsweise
$R_1$ ausgewählt ist aus Methyl, Ethyl, Isopropyl und $-CF_2H$;

$R_2$ ausgewählt ist aus

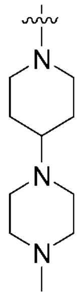, 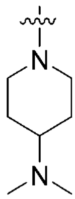, 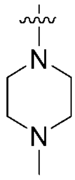 und 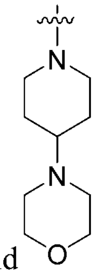;

$R_3$ ausgewählt ist aus Methyl, Ethyl und Isopropyl;
$R_4$ ausgewählt ist aus Br; und
$R_5$ ausgewählt ist aus Methyl, Ethyl und Isopropyl.

**5.** Verbindung oder ein pharmazeutisch akzeptables Salz davon nach Anspruch 3, wobei:

$R_2$ ausgewählt ist aus Amino und

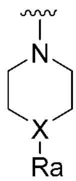;

und das Amino optional durch null bis drei $R_a$ substituiert ist; wobei X ausgewählt ist aus CH, N oder O;
$R_a$ ausgewählt ist aus

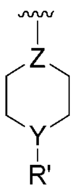,

$C_1$-$C_3$-Alkyl oder $NR_bR_c$;
wobei Y ausgewählt ist aus N oder O und Z ausgewählt ist aus CH oder N;
$R_b$ und $R_c$ jeweils unabhängig voneinander ausgewählt sind aus Wasserstoff und $C_1$-$C_3$-Alkyl;
$R_3$ ausgewählt ist aus $C_1$-$C_6$-Alkyl; und das $C_1$-$C_6$-Alkyl ist optional durch null bis drei R' substituiert;
$R_4$ ausgewählt ist aus Cl, Br und $C_1$-$C_6$-Alkyl, und das $C_1$-$C_6$-Alkyl optional durch null bis drei R' substituiert ist;
$R_5$ ausgewählt ist aus $C_1$-$C_6$-Alkyl, und das $C_1$-$C_6$-Alkyl optional durch null bis drei R' substituiert ist; und
R' ausgewählt ist aus F, Cl, Br, I, Hydroxyl und Amino;
vorzugsweise
$R_1$ ausgewählt ist aus Methyl, Ethyl, Isopropyl, $CF_2H$ und $CH_2CF_3$;
$R_2$ ausgewählt ist aus

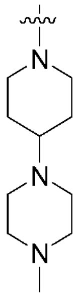, 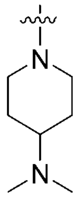, 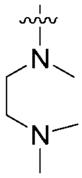, 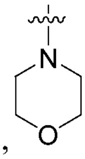, 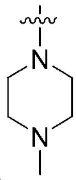 und 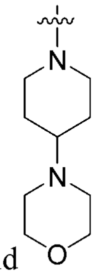;

$R_3$ ausgewählt ist aus Methyl und Ethyl;
$R_4$ ausgewählt ist aus Cl, Br, $CF_3$ und $CH_2CF_3$; und
$R_5$ ausgewählt ist aus Methyl und Ethyl.

**6.** Verbindung oder ein pharmazeutisch akzeptables Salz davon nach Anspruch 2, wobei:

$R_3$ ein Halogen ist;
$R_4$ ausgewählt ist aus Halogen und $C_1$-$C_6$-Alkyl; wobei das $C_1$-$C_6$-Alkyl optional durch null bis drei R' substituiert ist;
$R_5$ ausgewählt ist aus $C_1$-$C_6$-Alkyl; und das $C_1$-$C_6$-Alkyl optional durch null bis drei R' substituiert ist;
$R_6$ Wasserstoff ist; und
R' ausgewählt ist aus Wasserstoff, $C_1$-$C_6$-Alkyl, F, Cl, Br, I, Hydroxyl und Amino;
vorzugsweise
$R_1$ ausgewählt ist aus Methyl, Ethyl und Isopropyl;
$R_2$ ausgewählt ist aus

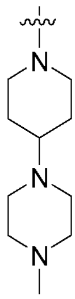, 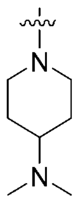, 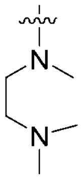, 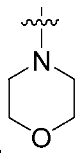, 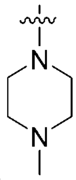 und 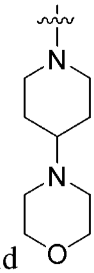;

$R_3$ ausgewählt ist aus Cl und Br;
$R_4$ ausgewählt ist aus Cl, Br, $CF_3$ und $CH_2CF_3$; und
$R_5$ ausgewählt ist aus Methyl, Ethyl und Isopropyl.

**7.** Verbindung oder ein pharmazeutisch akzeptables Salz davon nach Anspruch 2, wobei:

$R_3$ ein Halogen ist;
$R_4$ ausgewählt ist aus Halogen und $C_1$-$C_6$-Alkyl; wobei das $C_1$-$C_6$-Alkyl optional durch null bis drei R' substituiert ist;
$R_5$ ausgewählt ist aus $C_1$-$C_6$-Alkyl; und das $C_1$-$C_6$-Alkyl optional durch null bis drei R' substituiert ist;
$R_6$ Wasserstoff ist; und
R' ausgewählt ist aus F, Cl, Br, I, Hydroxyl und Amino;
vorzugsweise
$R_1$ ausgewählt ist aus Methyl, Ethyl und Isopropyl;
$R_2$ ausgewählt ist aus

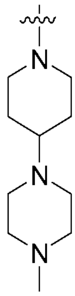 , 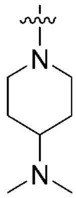 , 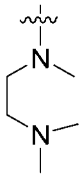 , 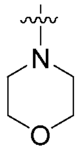 , 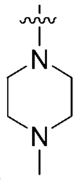 und 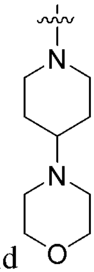 ;

$R_3$ ausgewählt ist aus Cl und Br;
$R_4$ ausgewählt ist aus Cl, Br, $CF_3$ und $CH_2CF_3$; und
$R_5$ ausgewählt ist aus Methyl und Ethyl.

**8.** Verbindung oder ein pharmazeutisch akzeptables Salz davon nach Anspruch 2, wobei:

$R_3$ ausgewählt ist aus $C_1$-$C_6$-Alkyl; und das $C_1$-$C_6$-Alkyl optional durch null bis drei R' substituiert ist;
$R_4$ ausgewählt ist aus Halogen und $C_1$-$C_6$-Alkyl; wobei das $C_1$-$C_6$-Alkyl optional durch null bis drei R' substituiert ist;
$R_5$ ausgewählt ist aus Halogen;
$R_6$ Wasserstoff ist; und
R' ausgewählt ist aus Wasserstoff, $C_1$-$C_6$-Alkyl, F, Cl, Br, I, Hydroxyl und Amino;
vorzugsweise
$R_1$ ausgewählt ist aus Methyl, Ethyl und Isopropyl;
$R_2$ ausgewählt ist aus

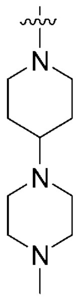 , 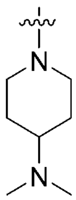 , 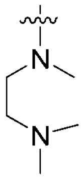 , 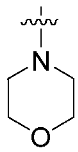 , 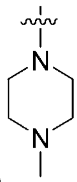 und 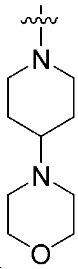 ;

$R_3$ ausgewählt ist aus Methyl, Ethyl und Isopropyl;
$R_4$ ausgewählt ist aus Cl, Br, $CF_3$ und $CH_2CF_3$; und
$R_5$ ausgewählt ist aus F und Cl;
besonders vorzugsweise
$R_1$ ausgewählt ist aus Methyl, Ethyl und Isopropyl;
$R_2$ ausgewählt ist aus

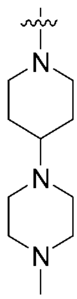 , 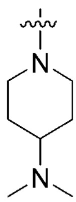 , 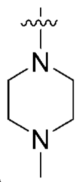 und 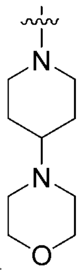 ;

$R_3$ ausgewählt ist aus Methyl, Ethyl und Isopropyl;
$R_4$ ausgewählt ist aus Br; und

$R_5$ ausgewählt ist aus F und Cl.

**9.** Verbindung oder ein pharmazeutisch akzeptables Salz davon nach Anspruch 2, wobei:

$R_3$ ausgewählt ist aus $C_1$-$C_6$-Alkyl; und das $C_1$-$C_6$-Alkyl optional durch null bis drei R' substituiert ist;
$R_4$ ausgewählt ist aus Halogen und $C_1$-$C_6$-Alkyl; wobei das $C_1$-$C_6$-Alkyl optional durch null bis drei R' substituiert ist;
$R_5$ ausgewählt ist aus Halogen;
$R_6$ Wasserstoff ist; und
R' ausgewählt ist aus F, Cl, Br, I, Hydroxyl und Amino;
vorzugsweise
$R_1$ ausgewählt ist aus Methyl, Ethyl und Isopropyl;
$R_2$ ausgewählt ist aus

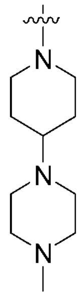 , 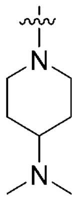 , 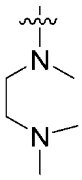 , 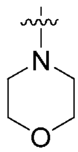 , 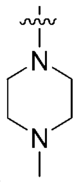 und 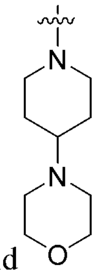 ;

$R_3$ ausgewählt ist aus Methyl und Ethyl;
$R_4$ ausgewählt ist aus Cl, Br, $CF_3$ und $CH_2CF_3$; und
$R_5$ ausgewählt ist aus F und Cl.

**10.** Verbindung oder ein pharmazeutisch akzeptables Salz davon nach Anspruch 1, ausgewählt aus:

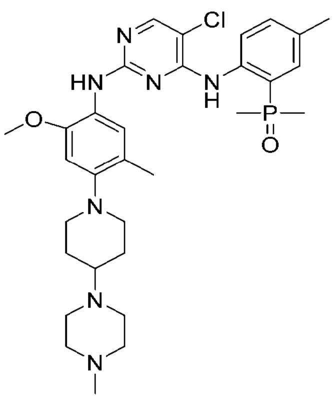 , 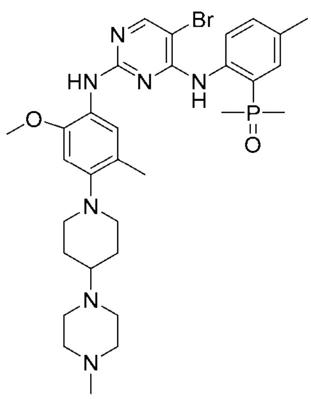 , 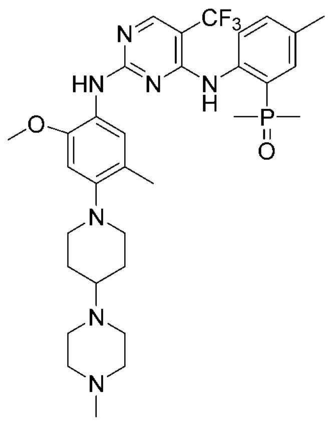 ,

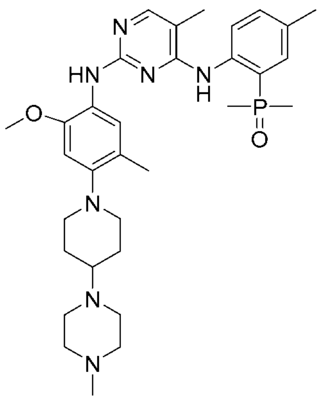,
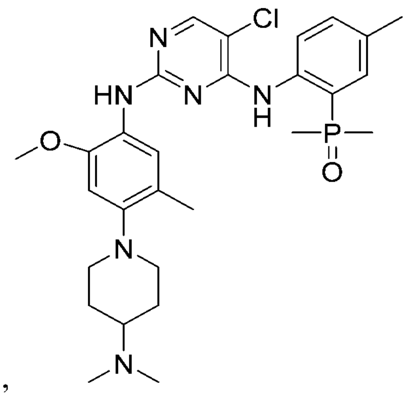,
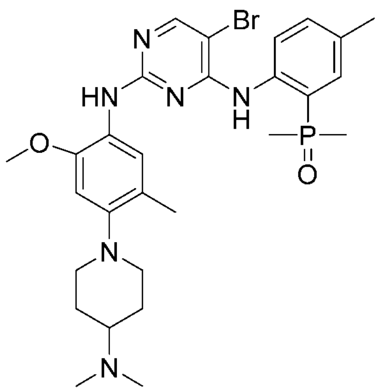,
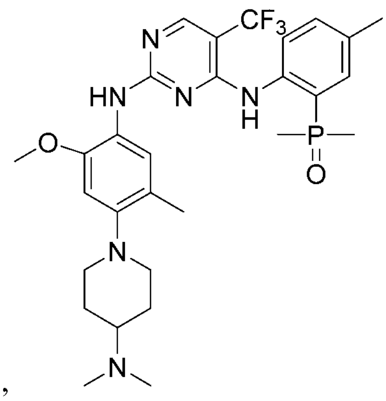,
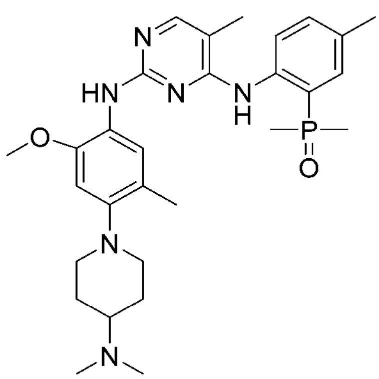,
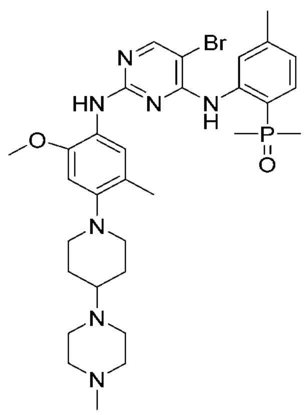,
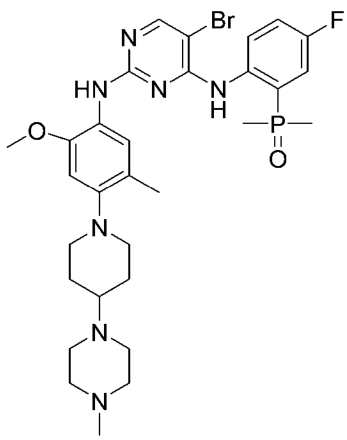,
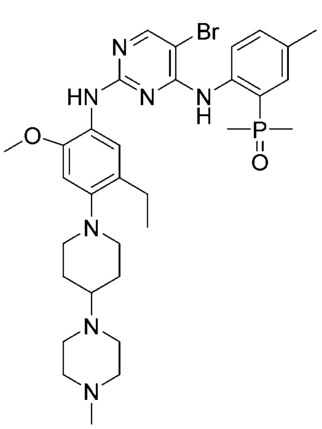,
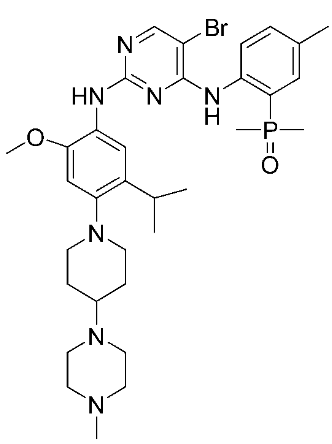,
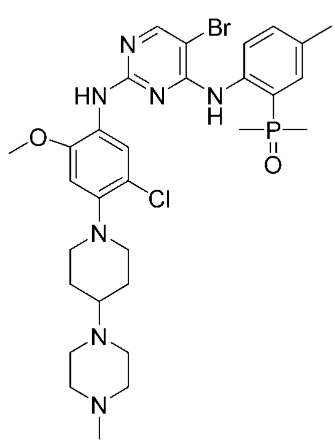,

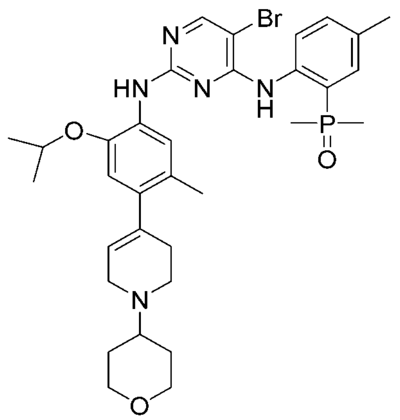
,
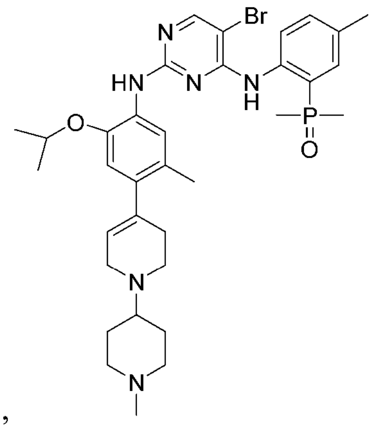
,

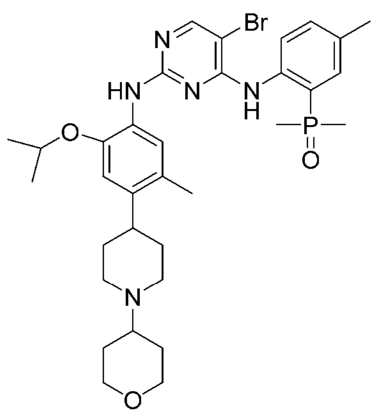
,
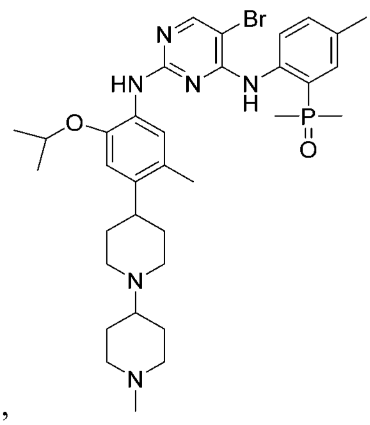
,

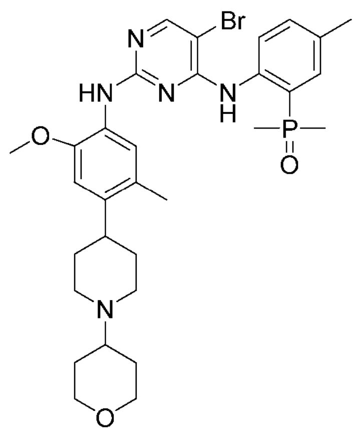
,
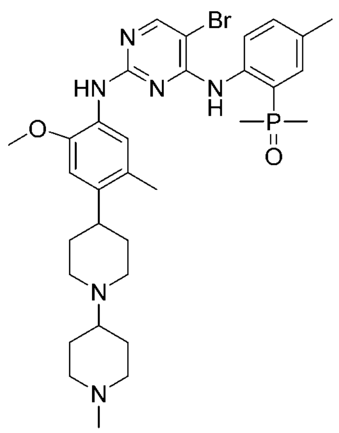
,
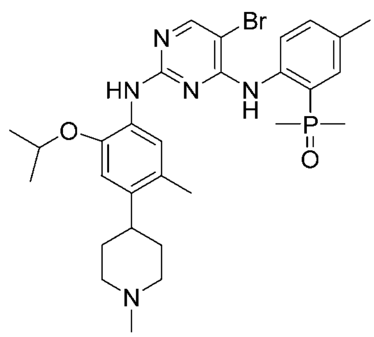
,

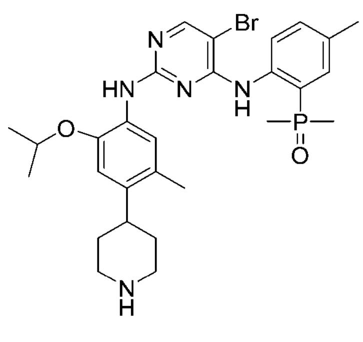
,
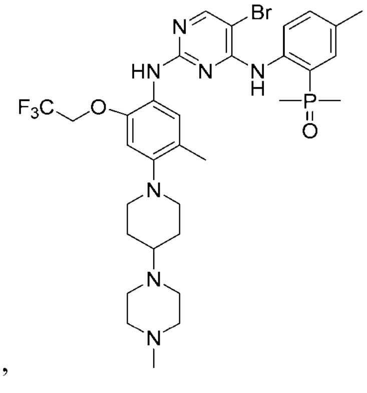
,
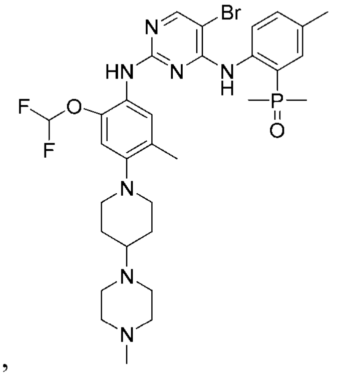
, , , ,

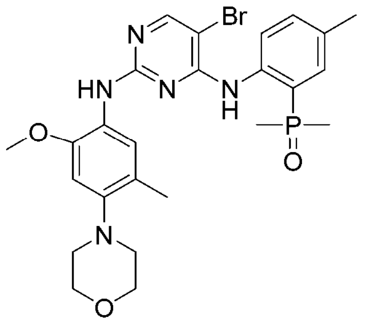
,
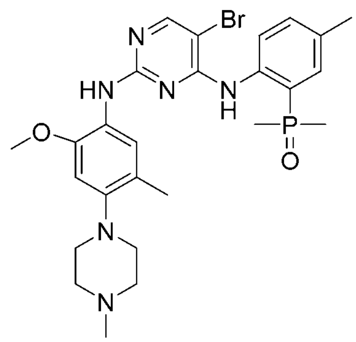
,
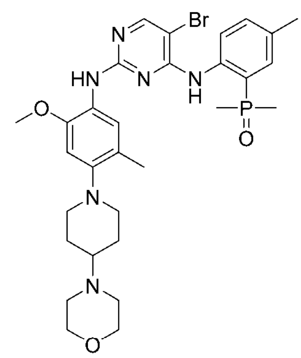
,

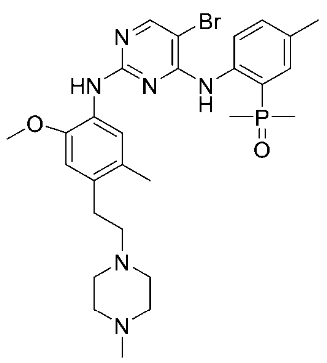
,
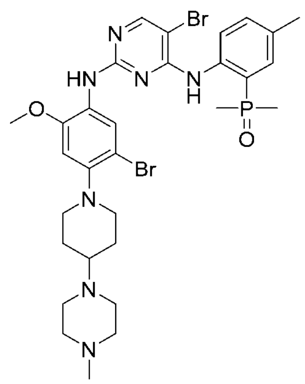
,
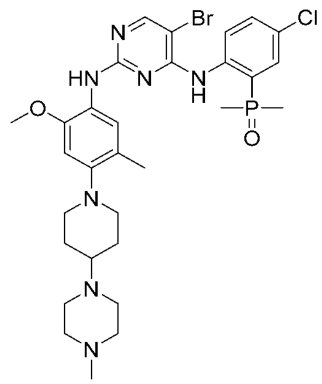
,

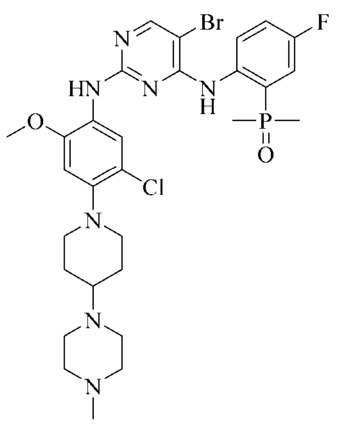
,
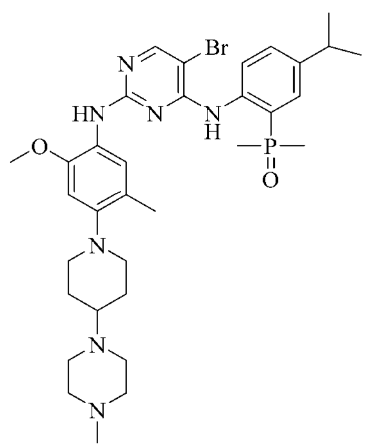
,
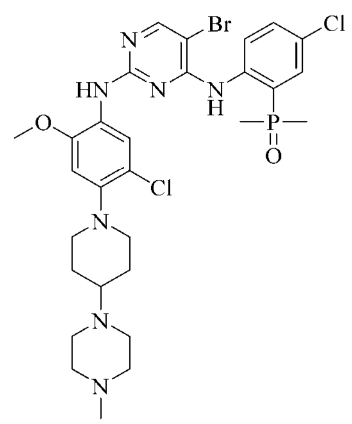

und

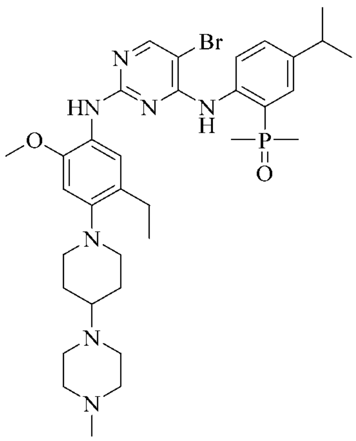

.

**11.** Pharmazeutische Zusammensetzung, umfassend die Verbindung oder ein pharmazeutisch akzeptables Salz davon nach einem der Ansprüche 1 bis 10 und einen pharmazeutisch akzeptablen Träger.

**12.** Pharmazeutische Zusammensetzung nach Anspruch 11, wobei die pharmazeutische Zusammensetzung ein weiteres Antikrebsarzneimittel oder Antitumorarzneimittel umfasst; vorzugsweise ist das Antikrebsarzneimittel oder Antitumorarzneimittel eines oder mehrere der folgenden: ein zytotoxisches Arzneimittel, ein Hormonarzneimittel, ein Antimetabolit, ein tumorgerichtetes Arzneimittel, ein PARP-Inhibitor, ein Arzneimittel zur adjuvanten Therapie oder ein biologisches Antitumor-Arzneimittel; besonders vorzugsweise ist das zytotoxische Arzneimittel eines oder mehrere der folgenden: Carboplatin, Cisplatin, Irinotecan, Paclitaxel, Fluorouracil, Cytarabin, Lenalidomid und Retinsäure; das Hormonarzneimittel eines oder mehrere der folgenden: Dexamethason, Fulvestrant und Tamoxifen; der Antimetabolit eines oder mehrere der folgenden: Fluorouracil, Methotrexat, Furanfluorouracil und Cytarabin; das tumorgerichtete Arzneimittel eines oder mehrere der folgenden: Imatinib, Erlotinib und Lapatinib; der PARP-Inhibitor eines oder mehrere der folgenden: Olaparib, Rubraca und Zejula; das Arzneimittel zur adjuvanten Therapie eines oder mehrere der folgenden: rekombinanter humaner Granulozyten-Kolonie-stimulierender Faktor, Erythropoetin, Pamidronat-Dinatrium und Zoledronsäure; und das biologische Antitumor-Arzneimittel eines oder mehrere der folgenden: Keytruda, Opdiv, Tecentriq, Imfinzi und Bavencio.

**13.** Verbindung oder ein pharmazeutisch akzeptables Salz davon nach einem der Ansprüche 1 bis 10 oder die pharmazeutische Zusammensetzung nach Anspruch 11 oder 12 zur Verwendung als Arzneimittel.

**14.** Verbindung oder ein pharmazeutisch akzeptables Salz davon nach einem der Ansprüche 1 bis 10 oder die pharmazeutische Zusammensetzung nach Anspruch 11 oder 12 zur Verwendung bei der Behandlung von Krebs; vorzugsweise ist der Krebs ein Plasmozytom, Mantelzelltumor, multiples Myelom, Melanom, Brustkrebs, Leberkrebs, Gebärmutterhalskrebs, Lungenkrebs, Lymphom, Leukämie, Eierstockkrebs, Nierenkrebs, Magenkrebs, Nasopharynxkrebs, Schilddrüsenkrebs, Bauchspeicheldrüsenkrebs, Prostatakrebs, Adenokarzinom, Mundhöhlenkrebs, Speiseröhrenkrebs, Plattenepithelkarzinom oder Dickdarmkrebs.

**15.** Verbindung oder ein pharmazeutisch akzeptables Salz davon nach einem der Ansprüche 1 bis 10 oder die pharmazeutische Zusammensetzung nach Anspruch 11 oder 12 zur Verwendung als EGFR-Inhibitor, als ALK-Inhibitor, als EGFR- und ALK-Inhibitor oder als Proteinkinase-Inhibitor,
vorzugsweise wird der EGFR-Inhibitor oder der ALK-Inhibitor oder der EGFR- und ALK-Inhibitor oder der Proteinkinase-Inhibitor angewendet bei Plasmozytom, Mantelzelltumor, multiplem Myelom, Melanom, Brustkrebs, Leberkrebs, Gebärmutterhalskrebs, Lungenkrebs, Lymphom, Leukämie, Eierstockkrebs, Nierenkrebs, Magenkrebs, Nasopharynxkrebs, Schilddrüsenkrebs, Bauchspeicheldrüsenkrebs, Prostatakrebs, Adenokarzinom, Mundhöhlenkrebs, Speiseröhrenkrebs, Plattenepithelkarzinom oder Dickdarmkrebs.

## Revendications

**1.** Composé représenté par la formule (I) ou sel pharmaceutiquement acceptable de celui-ci :

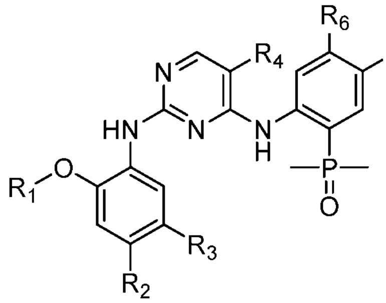

formule (I),

dans lequel $R_1$ est choisi parmi un alkyle en $C_1$-$C_6$ et un cycloalkyle en $C_3$-$C_6$, l'alkyle en $C_1$-$C_6$ et le cycloalkyle en $C_3$-$C_6$ sont éventuellement substitués par zéro à six R' ;
$R_2$ est choisi parmi un hydrogène, un amino, un alkyle en $C_1$-$C_6$, un cycloalkyle en $C_3$-$C_6$, un cycloalcényle en $C_3$-$C_6$, un phényle, un hétéroaryle à 5 à 6 chaînons,

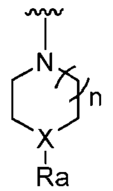

ou

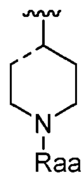

;

et l'amino, l'alkyle en $C_1$-$C_6$, le cycloalkyle en $C_3$-$C_6$, le phényle et l'hétéroaryle à 5 à 6 chaînons sont éventuellement substitués par zéro à trois groupes $R_a$ ;
X est choisi parmi CH, S, N ou O ;
n représente 0, 1 ou 2 ;
lorsque X représente O, $R_a$ n'est pas inclus ;
la liaison - - - - représente une liaison C-C saturée ou une liaison C=C oléfinique ;
$R_3$ est choisi parmi un alkyle en $C_1$-$C_6$, un cycloalkyle en $C_3$-$C_6$ et un halogène ; et l'alkyle en $C_1$-$C_6$ et le cycloalkyle en $C_3$-$C_6$ sont éventuellement substitués par zéro à trois R' ;
$R_4$ et $R_6$ sont chacun indépendamment choisis parmi un hydrogène, un alkyle en $C_1$-$C_6$, un cycloalkyle en $C_3$-$C_6$ et un halogène, et l'alkyle en $C_1$-$C_6$ et le cycloalkyle en $C_3$-$C_6$ sont éventuellement substitués par zéro à trois R' ;
$R_5$ est choisi parmi un alkyle en $C_1$-$C_6$ et un halogène, et l'alkyle en $C_1$-$C_6$ est éventuellement substitué par zéro à trois R' ;
$R_a$ et $R_{aa}$ sont chacun indépendamment choisis parmi un hydrogène, un alkyle en $C_1$-$C_6$, un cycloalkyle en $C_3$-$C_6$, un phényle, un hétéroaryle à 5 à 6 chaînons, un halogène, un amino, un hydroxyle, un cyano, un nitro, $-NR_bC(O)R_c$, $-NR_bR_c$ et

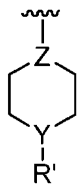

;

dans lequel l'alkyle en $C_1$-$C_6$, le cycloalkyle en $C_3$-$C_6$, le phényle, l'hétéroaryle à 5 à 6 chaînons, l'amino et l'hydroxyle sont éventuellement substitués par zéro à trois R' ;
Y est choisi parmi N ou O, et Z est choisi parmi CH ou N ;
$R_b$ et $R_c$ sont chacun indépendamment choisis parmi un hydrogène, un alkyle en $C_1$-$C_6$, un cycloalkyle en $C_3$-$C_6$, un alcényle en $C_2$-$C_6$, un alcynyle en $C_2$-$C_6$, un hydroxyle et un amino ; et l'alkyle en $C_1$-$C_6$, le cycloalkyle en $C_3$-$C_6$, l'alcényle en $C_2$-$C_6$ et l'alcynyle en $C_2$-$C_6$ sont éventuellement substitués par zéro à trois R' ; et
R' est choisi parmi un hydrogène, F, Cl, Br, I, un hydroxyle, un acyle, un carboxyle, un amino, un nitro, un cyano, un alkyle en $C_1$-$C_6$, un cycloalkyle en $C_3$-$C_6$, un alcényle en $C_2$-$C_6$ et un alcynyle en $C_2$-$C_6$ ; et l'alkyle en $C_1$-$C_6$, le cycloalkyle en $C_3$-$C_6$, l'alcényle en $C_2$-$C_6$, l'alcynyle en $C_2$-$C_6$, l'amino et l'hydroxyle sont éventuellement substitués par zéro à trois hydrogènes, hydroxyles, carboxyles, carbonyles, F, Cl, Br, I, amino, nitro, cyano,

méthyles, trifluoroéthyles, difluorométhyles et monofluorométhyles.

**2.** Composé ou sel pharmaceutiquement acceptable de celui-ci selon la revendication 1, dans lequel :

$R_1$ est choisi parmi un alkyle en $C_1$-$C_6$ ; et l'alkyle en $C_1$-$C_6$ est éventuellement substitué par zéro à trois R' ;
$R_2$ est choisi parmi un hydrogène, un alkyle en $C_1$-$C_6$, un amino,

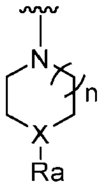 ou 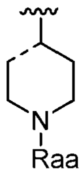 ;

dans lequel, l'amino et l'alkyle en $C_1$-$C_6$ sont substitués par zéro à trois $R_a$ ;
$R_a$ et $R_{aa}$ sont chacun indépendamment choisis parmi un hydrogène, $NR_bR_c$, un alkyle en $C_1$-$C_6$ ou

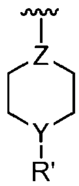 ;

$R_3$ est choisi parmi un alkyle en $C_1$-$C_6$, un cycloalkyle en $C_3$-$C_6$ et un halogène ; et l'alkyle en $C_1$-$C_6$ et le cycloalkyle en $C_3$-$C_6$ sont éventuellement substitués par zéro à trois R' ;
$R_4$ est choisi parmi un halogène, un alkyle en $C_1$-$C_6$ et un cycloalkyle en $C_3$-$C_6$ ; et l'alkyle en $C_1$-$C_6$ et le cycloalkyle en $C_3$-$C_6$ sont éventuellement substitués par zéro à trois R' ;
$R_5$ est choisi parmi un alkyle en $C_1$-$C_6$ et un halogène ; et l'alkyle en $C_1$-$C_6$ est éventuellement substitué par zéro à trois R' ;
$R_6$ est choisi parmi un hydrogène ou un méthyle ; et
$R_b$ et $R_c$ sont chacun indépendamment choisis parmi un hydrogène, un alkyle en $C_1$-$C_6$ et un alcényle en $C_2$-$C_6$ ; et l'alkyle en $C_1$-$C_6$ et l'alcényle en $C_2$-$C_6$ sont éventuellement substitués par zéro à trois R'.

**3.** Composé ou sel pharmaceutiquement acceptable de celui-ci selon la revendication 2, dans lequel :

$R_3$ est choisi parmi un alkyle en $C_1$-$C_6$ et un cycloalkyle en $C_3$-$C_6$ ; et l'alkyle en $C_1$-$C_6$ et le cycloalkyle en $C_3$-$C_6$ sont éventuellement substitués par zéro à trois R' ;
$R_4$ est choisi parmi un halogène et un alkyle en $C_1$-$C_6$ ; et l'alkyle en $C_1$-$C_6$ est éventuellement substitué par zéro à trois R'
$R_5$ représente un alkyle en $C_1$-$C_6$ ; dans lequel l'alkyle en $C_1$-$C_6$ est éventuellement substitué par zéro à trois R' ; et
$R_6$ représente un hydrogène.

**4.** Composé ou sel pharmaceutiquement acceptable de celui-ci selon la revendication 3, dans lequel :

$R_2$ est choisi parmi un amino et

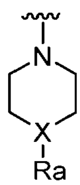 ;

et l'amino est éventuellement substitué par zéro à trois $R_a$ ; dans lequel X est choisi parmi CH, N ou O ;

$R_a$ est choisi parmi

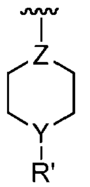

,

un alkyle en $C_1$-$C_3$ ou $NR_bR_c$ ;
dans lequel Y est choisi parmi N ou O, et Z est choisi parmi CH ou N ;
$R_b$ et $R_c$ sont chacun indépendamment choisis parmi un hydrogène et un alkyle en $C_1$-$C_3$ ;
$R_3$ est choisi parmi un alkyle en $C_1$-$C_6$ ; et l'alkyle en $C_1$-$C_6$ est éventuellement substitué par zéro à trois R' ;
$R_4$ est choisi parmi Cl, Br et un alkyle en $C_1$-$C_6$, et l'alkyle en $C_1$-$C_6$ est éventuellement substitué par zéro à trois R' ;
$R_5$ est choisi parmi un alkyle en $C_1$-$C_6$, et l'alkyle en $C_1$-$C_6$ est éventuellement substitué par zéro à trois R' ; et
R' est choisi parmi un hydrogène, un alkyle en $C_1$-$C_6$, F, Cl, Br, I, un hydroxyle et un amino ; de préférence,
$R_1$ est choisi parmi un méthyle, un éthyle, un isopropyle, $CF_2H$ et $CH_2CF_3$ ;
$R_2$ est choisi parmi

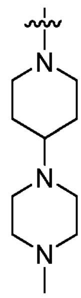 , 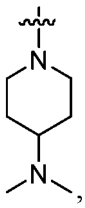 , 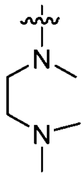 , 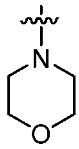 , 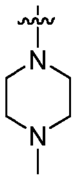 et 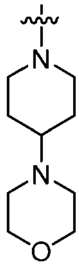 ;

$R_3$ est choisi parmi un méthyle, un éthyle et un isopropyle ;
$R_4$ est choisi parmi Cl, Br, $CH_3$, $CF_3$ et $CH_2CF_3$ ; et
$R_5$ est choisi parmi un méthyle, un éthyle et un isopropyle ;
plus préférentiellement,
$R_1$ est choisi parmi un méthyle, un éthyle, un isopropyle et $-CF_2H$ ;
$R_2$ est choisi parmi

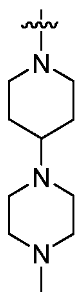 , 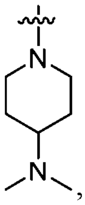 , 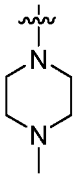 et 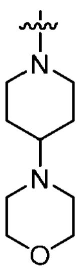 ;

$R_3$ est choisi parmi un méthyle, un éthyle et un isopropyle ;
$R_4$ est choisi parmi Br ; et
$R_5$ est choisi parmi un méthyle, un éthyle et un isopropyle.

**5.** Composé ou sel pharmaceutiquement acceptable de celui-ci selon la revendication 3, dans lequel :

$R_2$ est choisi parmi un amino et

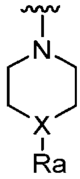

;

et l'amino est éventuellement substitué par zéro à trois $R_a$ ; dans lequel X est choisi parmi CH, N ou O ;

$R_a$ est choisi parmi

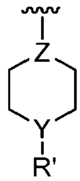

,

un alkyle en $C_1$-$C_3$ ou $NR_bR_c$ ;

dans lequel Y est choisi parmi N ou O, et Z est choisi parmi CH ou N ;

$R_b$ et $R_c$ sont chacun indépendamment choisis parmi un hydrogène et un alkyle en $C_1$-$C_3$ ;

$R_3$ est choisi parmi un alkyle en $C_1$-$C_6$ ; et l'alkyle en $C_1$-$C_6$ est éventuellement substitué par zéro à trois R' ;

$R_4$ est choisi parmi Cl, Br et un alkyle en $C_1$-$C_6$, et l'alkyle en $C_1$-$C_6$ est éventuellement substitué par zéro à trois R' ;

$R_5$ est choisi parmi un alkyle en $C_1$-$C_6$, et l'alkyle en $C_1$-$C_6$ est éventuellement substitué par zéro à trois R' ; et

R' est choisi parmi F, Cl, Br, I, un hydroxyle et un amino ;

de préférence,

$R_1$ est choisi parmi un méthyle, un éthyle, un isopropyle, $CF_2H$ et $CH_2CF_3$ ;

$R_2$ est choisi parmi

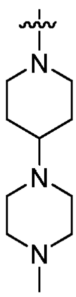
, 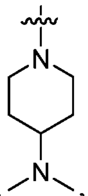
, 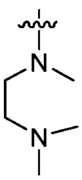
, 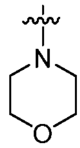
, 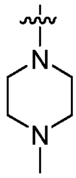
et 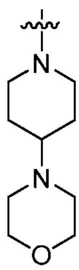
;

$R_3$ est choisi parmi un méthyle et un éthyle ;

$R_4$ est choisi parmi Cl, Br, $CF_3$ et $CH_2CF_3$ ; et

$R_5$ est choisi parmi un méthyle et un éthyle.

**6.** Composé ou sel pharmaceutiquement acceptable de celui-ci selon la revendication 2, dans lequel :

$R_3$ représente un halogène ;

$R_4$ est choisi parmi un halogène et un alkyle en $C_1$-$C_6$ ; dans lequel l'alkyle en $C_1$-$C_6$ est éventuellement substitué par zéro à trois R' ;

$R_5$ est choisi parmi un alkyle en $C_1$-$C_6$ ; et l'alkyle en $C_1$-$C_6$ est éventuellement substitué par zéro à trois R' ;

$R_6$ représente un hydrogène ; et

R' est choisi parmi un hydrogène, un alkyle en $C_1$-$C_6$, F, Cl, Br, I, un hydroxyle et un amino ;

de préférence,

$R_1$ est choisi parmi un méthyle, un éthyle et un isopropyle ;

$R_2$ est choisi parmi

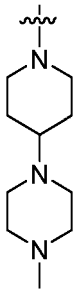, 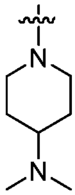, 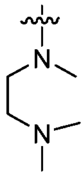, 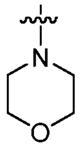, 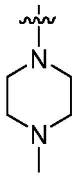 et 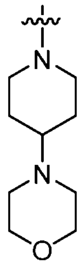 ;

$R_3$ est choisi parmi Cl et Br ;
$R_4$ est choisi parmi Cl, Br, $CF_3$ et $CH_2CF_3$ ; et
$R_5$ est choisi parmi un méthyle, un éthyle et un isopropyle.

**7.** Composé ou sel pharmaceutiquement acceptable de celui-ci selon la revendication 2, dans lequel :

$R_3$ représente un halogène ;
$R_4$ est choisi parmi un halogène et un alkyle en $C_1$-$C_6$ ; dans lequel l'alkyle en $C_1$-$C_6$ est éventuellement substitué par zéro à trois R' ;
$R_5$ est choisi parmi un alkyle en $C_1$-$C_6$ ; et l'alkyle en $C_1$-$C_6$ est éventuellement substitué par zéro à trois R' ;
$R_6$ représente un hydrogène ; et
R' est choisi parmi F, Cl, Br, I, un hydroxyle et un amino ;
de préférence,
$R_1$ est choisi parmi un méthyle, un éthyle et un isopropyle ;
$R_2$ est choisi parmi

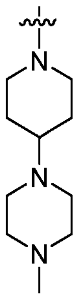, 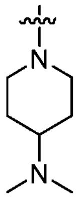, 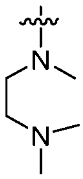, 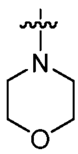, 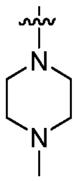 et 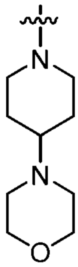 ;

$R_3$ est choisi parmi Cl et Br ;
$R_4$ est choisi parmi Cl, Br, $CF_3$ et $CH_2CF_3$ ; et
$R_5$ est choisi parmi un méthyle et un éthyle.

**8.** Composé ou sel pharmaceutiquement acceptable de celui-ci selon la revendication 2, dans lequel :

$R_3$ est choisi parmi un alkyle en $C_1$-$C_6$ ; et l'alkyle en $C_1$-$C_6$ est éventuellement substitué par zéro à trois R' ;
$R_4$ est choisi parmi un halogène et un alkyle en $C_1$-$C_6$ ; dans lequel l'alkyle en $C_1$-$C_6$ est éventuellement substitué par zéro à trois R' ;
$R_5$ est choisi parmi un halogène ;
$R_6$ représente un hydrogène ; et
R' est choisi parmi un hydrogène, un alkyle en $C_1$-$C_6$, F, Cl, Br, I, un hydroxyle et un amino ;
de préférence,
$R_1$ est choisi parmi un méthyle, un éthyle et un isopropyle ;
$R_2$ est choisi parmi

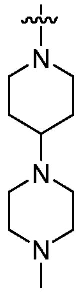 , 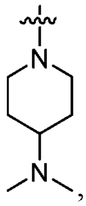 , 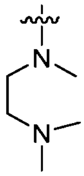 , 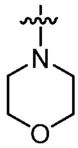 , 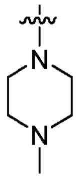 et 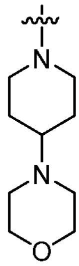 ;

$R_3$ est choisi parmi un méthyle, un éthyle et un isopropyle ;
$R_4$ est choisi parmi Cl, Br, $CF_3$ et $CH_2CF_3$ ; et
$R_5$ est choisi parmi F et Cl ;
plus préférentiellement,
$R_1$ est choisi parmi un méthyle, un éthyle et un isopropyle ;
$R_2$ est choisi parmi

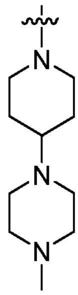 , 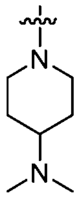 , 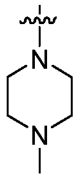 et 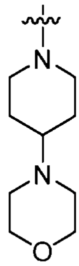 ;

$R_3$ est choisi parmi un méthyle, un éthyle et un isopropyle ;
$R_4$ est choisi parmi Br ; et
$R_5$ est choisi parmi F et Cl.

**9.** Composé ou sel pharmaceutiquement acceptable de celui-ci selon la revendication 2, dans lequel :

$R_3$ est choisi parmi un alkyle en $C_1$-$C_6$ ; et l'alkyle en $C_1$-$C_6$ est éventuellement substitué par zéro à trois R' ;
$R_4$ est choisi parmi un halogène et un alkyle en $C_1$-$C_6$ ; dans lequel l'alkyle en $C_1$-$C_6$ est éventuellement substitué par zéro à trois R' ;
$R_5$ est choisi parmi un halogène ;
$R_6$ représente un hydrogène ; et
R' est choisi parmi F, Cl, Br, I, un hydroxyle et un amino ;
de préférence,
$R_1$ est choisi parmi un méthyle, un éthyle et un isopropyle ;
$R_2$ est choisi parmi

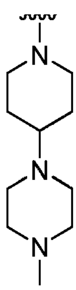 , 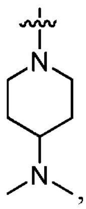 , 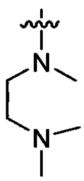 , 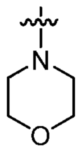 , 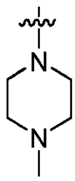 et 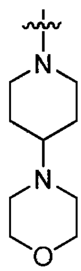 ;

$R_3$ est choisi parmi un méthyle et un éthyle ;
$R_4$ est choisi parmi Cl, Br, $CF_3$ et $CH_2CF_3$ ; et

$R_5$ est choisi parmi F et Cl.

**10.** Composé ou sel pharmaceutiquement acceptable de celui-ci selon la revendication 1, choisi parmi :

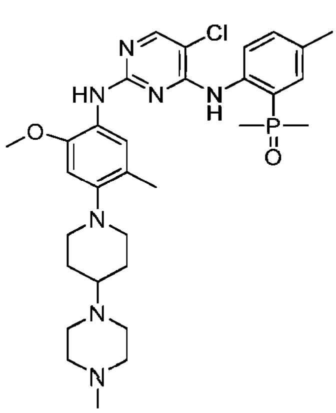

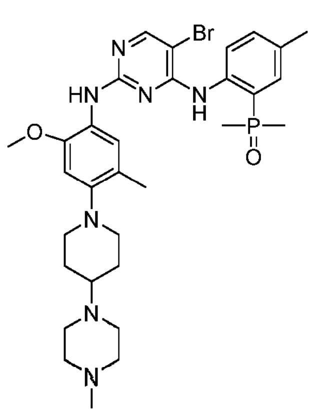

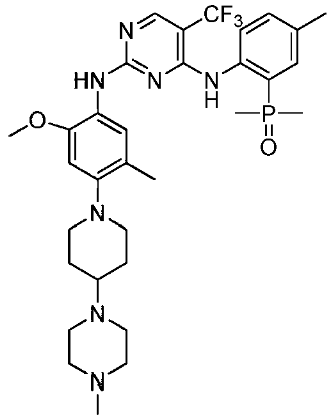

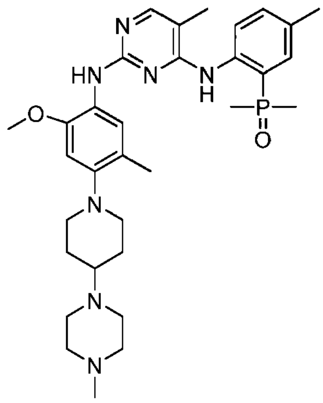

,

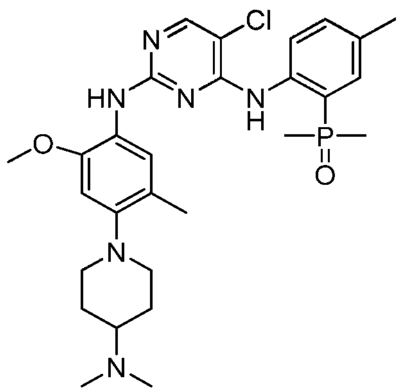

,

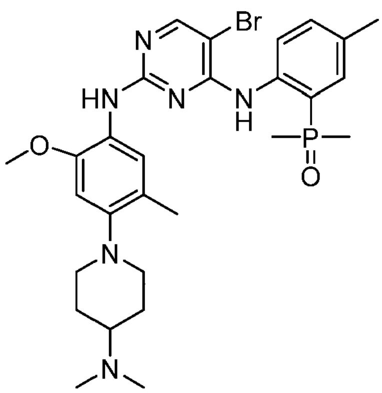

,

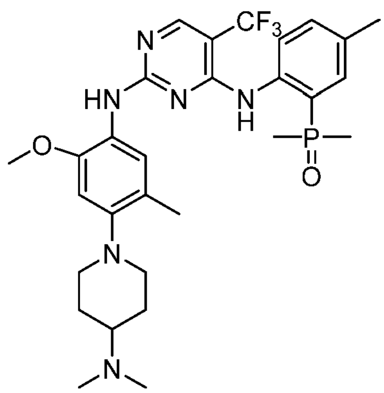

,

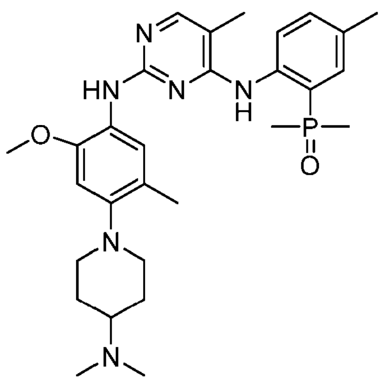

,

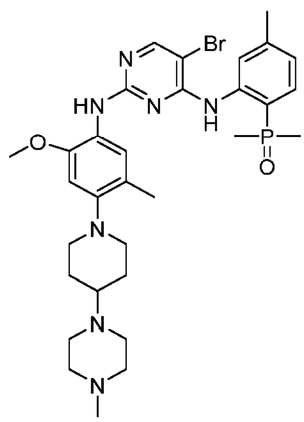

,

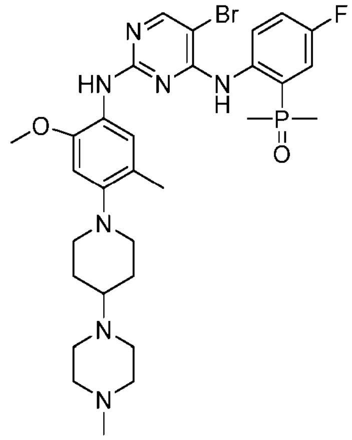

,

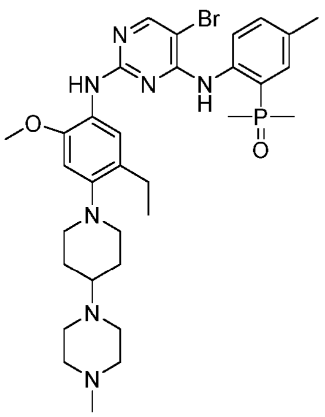

,

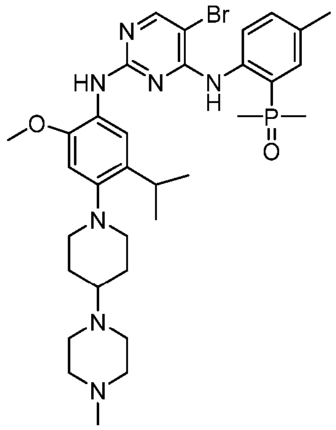

,

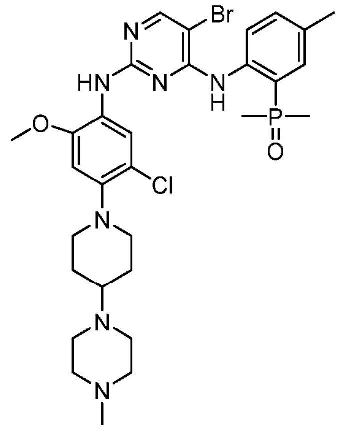

,

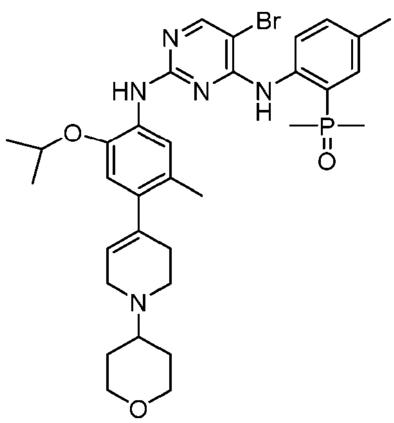

,

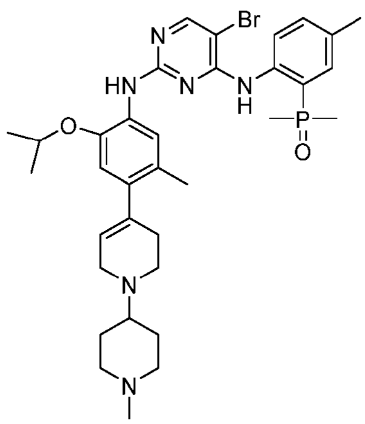

,

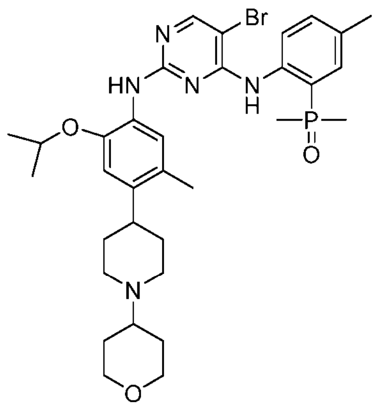 ,

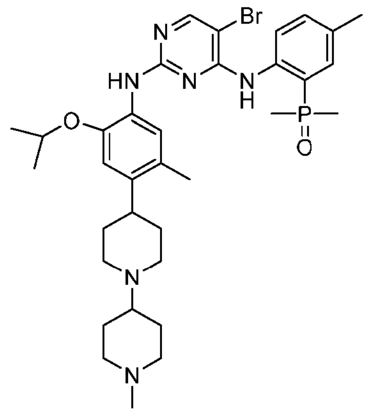 ,

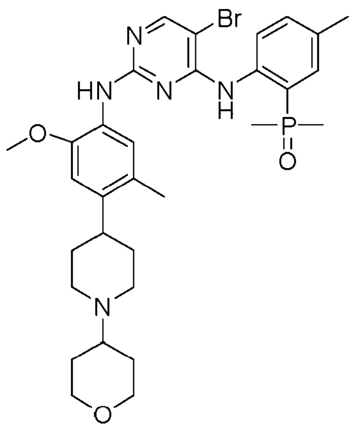 ,

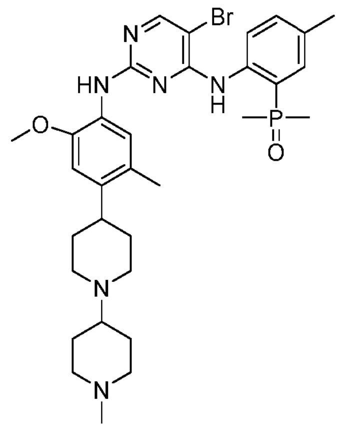 ,

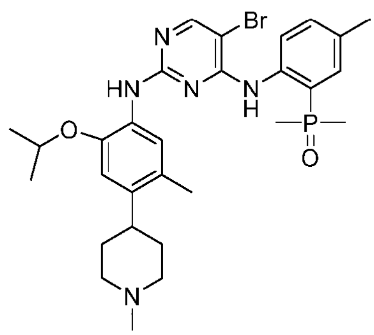 ,

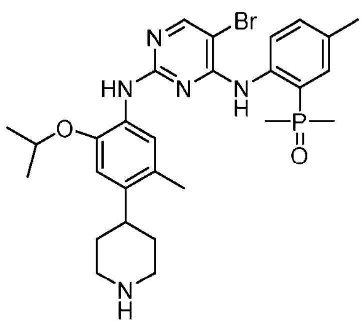 ,

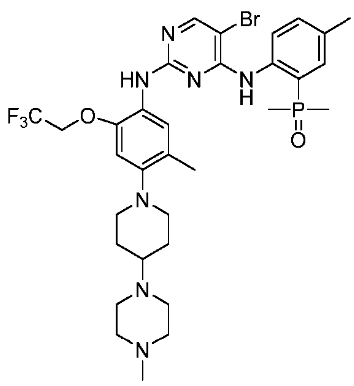 ,

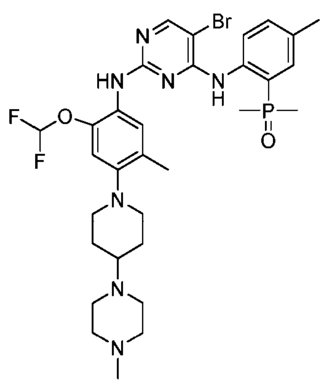 , , , ,

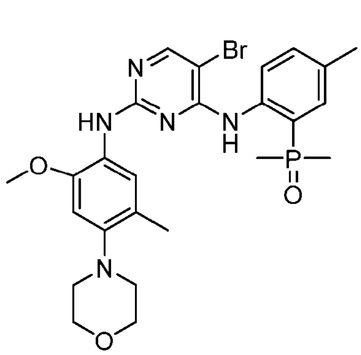 ,

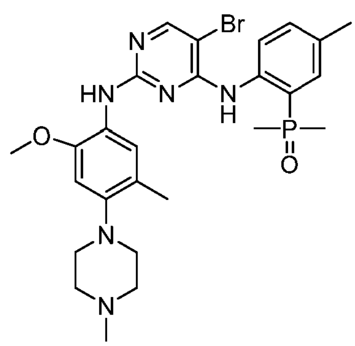 ,

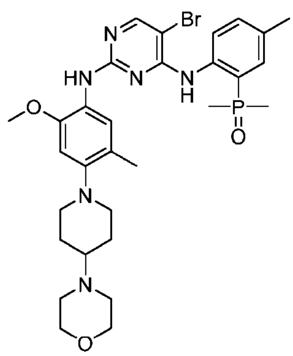 ,

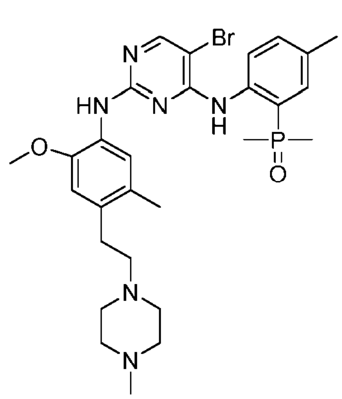

,

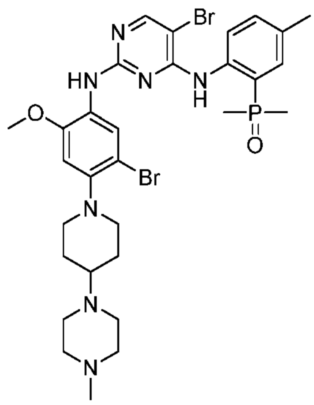

,

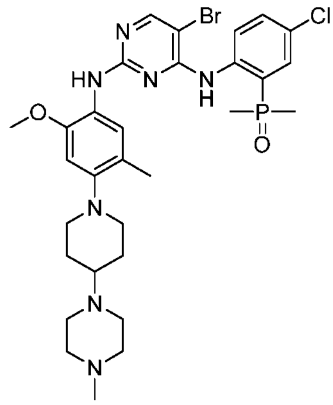

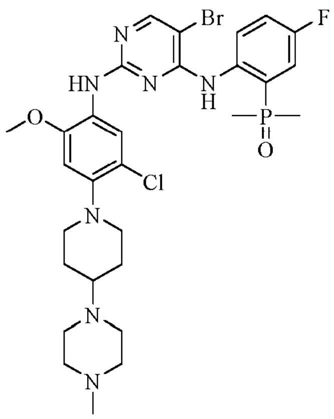

,

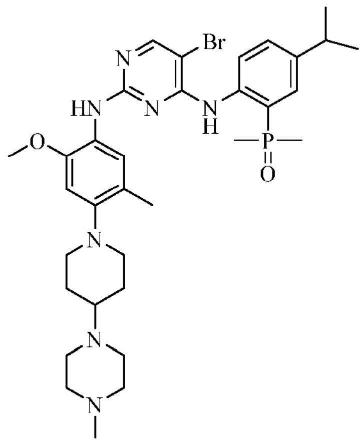

,

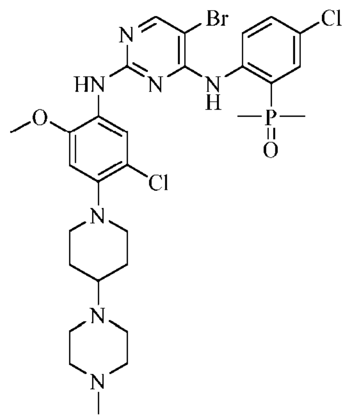

et

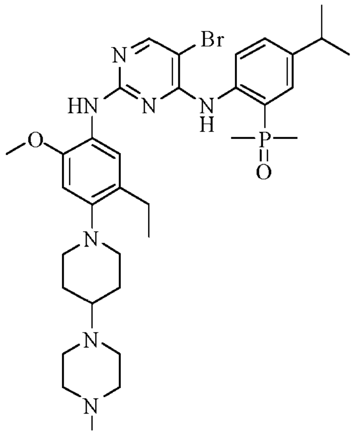

**11.** Composition pharmaceutique, comprenant le composé ou le sel pharmaceutiquement acceptable de celui-ci selon l'une quelconque des revendications 1 à 10, et un véhicule pharmaceutiquement acceptable.

**12.** Composition pharmaceutique selon la revendication 11, dans laquelle la composition pharmaceutique comprend un autre médicament anticancéreux ou médicament antitumoral ; de préférence, le médicament anticancéreux ou médicament antitumoral étant un ou plusieurs parmi un médicament cytotoxique, un médicament hormonal, un antimétabolite, un médicament ciblant une tumeur, un inhibiteur de PARP, un médicament de thérapie adjuvante ou un médicament antitumoral biologique ; plus préférentiellement, le médicament cytotoxique étant un ou plusieurs parmi le carboplatine, le cisplatine, l'irinotécan, le paclitaxel, le fluorouracile, la cytarabine, le lénalidomide et l'acide rétinoïque ; le médicament hormonal étant un ou plusieurs parmi la dexaméthasone, le fulvestrant et le tamoxifène ; l'antimétabolite étant un ou plusieurs parmi le fluorouracile, le méthotrexate, le furane fluorouracile et la cytarabine ; le

médicament ciblant une tumeur étant un ou plusieurs parmi l'imatinib, l'erlotinib et le lapatinib ; l'inhibiteur de PARP étant un ou plusieurs parmi l'Olaparib, le Rubraca et le Zejula ; le médicament de thérapie adjuvante étant un ou plusieurs parmi le facteur de stimulation des colonies de granulocytes humain recombinant, l'érythropoïétine, le pamidronate disodique et l'acide zolédronique ; et le médicament antitumoral biologique étant un ou plusieurs parmi le Keytruda, l'Opdiv, le Tecentriq, l'Imfinzi et le Bavencio.

**13.** Composé ou sel pharmaceutiquement acceptable de celui-ci selon l'une quelconque des revendications 1 à 10 ou composition pharmaceutique selon la revendication 11 ou 12 pour une utilisation comme médicament.

**14.** Composé ou sel pharmaceutiquement acceptable de celui-ci selon l'une quelconque des revendications 1 à 10, ou composition pharmaceutique selon la revendication 11 ou 12 pour une utilisation dans le traitement du cancer ; de préférence, le cancer est un plasmome, une tumeur à cellules du manteau, un myélome multiple, un mélanome, un cancer du sein, un cancer du foie, un cancer du col de l'utérus, un cancer du poumon, un lymphome, une leucémie, un cancer de l'ovaire, un cancer du rein, un cancer de l'estomac, un cancer du nasopharynx, un cancer de la thyroïde, un cancer du pancréas, un cancer de la prostate, un adénocarcinome, un cancer de la bouche, un cancer de l'œsophage, un carcinome à cellules squameuses ou un cancer du côlon.

**15.** Composé ou sel pharmaceutiquement acceptable de celui-ci selon l'une quelconque des revendications 1 à 10, ou composition pharmaceutique selon la revendication 11 ou 12 pour une utilisation comme inhibiteur d'EGFR, ou inhibiteur d'ALK, ou inhibiteur d'EGFR et d'ALK, ou inhibiteur de protéine kinase,
de préférence, l'inhibiteur d'EGFR, ou l'inhibiteur d'ALK, ou l'inhibiteur d'EGFR et d'ALK, ou l'inhibiteur de protéine kinase est appliqué à un plasmome, une tumeur à cellules du manteau, un myélome multiple, un mélanome, un cancer du sein, un cancer du foie, un cancer du col de l'utérus, un cancer du poumon, un lymphome, une leucémie, un cancer de l'ovaire, un cancer du rein, un cancer de l'estomac, un cancer du nasopharynx, un cancer de la thyroïde, un cancer du pancréas, un cancer de la prostate, un adénocarcinome, un cancer de la bouche, un cancer de l'œsophage, un carcinome à cellules squameuses ou un cancer du côlon.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012151561 A


### Non-patent literature cited in the description


- *Chinese Journal of Lung Cancer [J*, 20 February 2012, vol. 15 (2), 106-111
- *Nature Medicine*, 2015, vol. 21 (6), 560-562